# EUROPÄISCHE PATENTSCHRIFT

(11) **EP 0 574 418 B1**
(45) Veröffentlichungstag und Bekanntmachung des Hinweises auf die Patenterteilung: **11.11.1998**
(21) Anmeldenummer: 92904460.0
(22) Anmeldetag: 12.02.1992
(51) Int. Cl.: C07D 239/42, A01N 47/36, C07D 239/47, C07D 239/52, C07D 239/34, C07D 251/46, C07D 251/14, C07D 251/42

(54) **ARYLSULFONYLHARNSTOFFE, VERFAHREN ZU IHRER HERSTELLUNG UND IHRE VERWENDUNG ALS HERBIZIDE UND WACHSTUMSREGULATOREN**
ARYL SULPHONYL UREA COMPOUNDS, A METHOD OF PREPARING THEM, AND THEIR USE AS HERBICIDES AND GROWTH REGULATORS
ARYLSULFONYLUREES, LEUR PROCEDE DE PRODUCTION ET LEUR UTILISATION COMME HERBICIDES ET REGULATEURS DE LA CROISSANCE

(30) Priorität: 12.02.1991 DE 4104227
(43) Veröffentlichungstag der Anmeldung: 22.12.1993
(73) Patentinhaber: Hoechst Schering AgrEvo GmbH, 13342 Berlin (DE)
(72) Erfinder: ORT, Oswald, D-6233 Kelkheim (DE); BAUER, Klaus, D-6450 Hanau 7 (DE); BIERINGER, Hermann, D-6239 Eppstein (DE)
(86) Internationale Anmeldenummer: EP9200304
(87) Internationale Veröffentlichungsnummer: WO9213845

(56) Entgegenhaltungen:
- EP-A- 0 030 138
- EP-A- 0 084 020
- EP-A- 0 174 212
- EP-A- 0 291 851
- FR-A- 2 493 702

## Beschreibung

Arylsulfonylharnstoffe, Verfahren zu ihrer Herstellung und ihre Verwendung als Herbizide und Wachstumsregulatoren

Die Erfindung betrifft das Gebiet der Pflanzenschutzmittel, insbesondere selektive Herbizide und Wachstumsregulatoren vom Typ der heterocylisch substituierten Phenylsulfonylharnstoffe.

Aus der EP-A-007687 sind unter anderem bereits Sulfonylharnstoffe der Formel (1) bekannt, 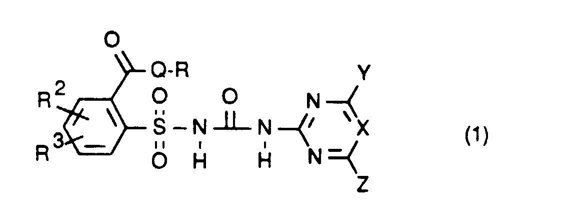 worin R² = H, Cl, Br, F, (C₁-C₃)-Alkyl, -NO₂, -SO₂CH₃, -OCH₃, -SCH₃, -CF₃, -N(CH₃)₂, -NH₂ oder -CN; R³ = H, Cl, Br, F oder CH₃; X = CH oder N, Q = O, S oder gegebenenfalls substituiertes NH; und Y, Z = H, Cl oder diverse organische Reste bedeuten. Die Verbindungen sind als Herbizide und Pflanzenwachstumsregulatoren beschrieben.

Aus EP-A-0291851 und DE-A-3900472 sind herbizide und pflanzenwachstumsregulatorische Sulfonylharnstoffe der Formel (2) bekannt, 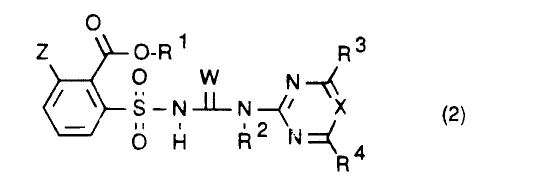 worin Z= F, Cl oder Br, R¹ = H, gegebenenfalls substituiertes Alkyl, Alkenyl, Alkinyl oder Cycloalkyl, R² = H, CH₃ oder C₂H₅, R³ = H, F, Cl, Br, CH₃ oder OCH₃, R⁴ = H, CH₃, (C₁-C₄)-Alkoxy und X = CH oder N bedeuten.

Aus EP-A-84 020 sind ebenfalls halogensubstituierte Phenylsulfonylharnstoff-Herbizide bekannt, wobei Halogen jeweils Fluor, Chlor oder Brom bedeutet und gegebenenfalls eine weiterer Substituent am Phenylring unter andererm Carbalkoxy sein kann.

Außerdem beschreibt US-A-4,566,898 den Sulfonylharnstoff der Formel (I) 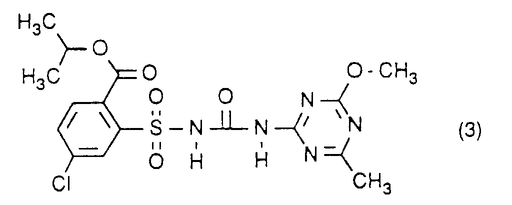 als Herbizid mit herausragenden Eigenschaften, insbesondere zur Kontrolle von Ackerfuchsschwanz in Gerste und Weizen.

Überraschend wurde nun gefunden, daß einige iodierte Arylsulfonylharnstoffe vorteilhafte Eigenschaften besitzen.

Gegenstand der vorliegenden Erfindung sind daher Verbindungen der Formel (I) und deren Salze, 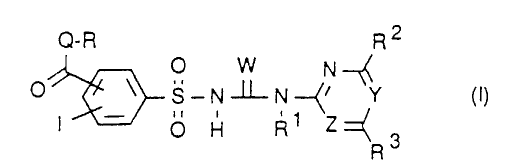 worin
- Q: Sauerstoff, Schwefel oder -N(R⁴)-, vorzugsweise O oder S, insbesondere O;
- W: Sauerstoff oder Schwefel, vorzugsweise O;
- Y, Z: unabhängig voneinander CH oder N, wobei Y und Z nicht gleichzeitig CH sind, vorzugsweise Y = CH oder N und Z = N;
- R: Wasserstoff; (C₁-C₁₂)-Alkyl; (C₂-C₁₀)-Alkenyl; (C₂-C₁₀)-Alkinyl; (C₁-C₆)-Alkyl, das ein- bis vierfach durch Reste aus der Gruppe Halogen, (C₁-C₄)-Alkoxy, (C₁-C₄)-Thioalkyl, -CN, (C₂-C₅)-Alkoxycarbonyl und (C₂-C₆)-Alkenyl substituiert ist; (C₃-C₈)-Cycloalkyl, das unsubstituiert oder durch Reste aus der Gruppe (C₁-C₄)-Alkyl, (C₁-C₄)-Alkoxy, (C₁-C₄)-Alkylthio und Halogen substituiert ist; (C₅-C₈)-Cycloalkenyl; Phenyl-(C₁-C₄)-alkyl, das im Phenylrest unsubstituiert oder substituiert ist; oder einen Rest der Formeln A-1 bis A-10 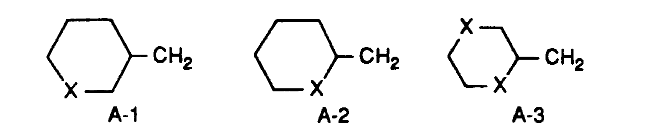 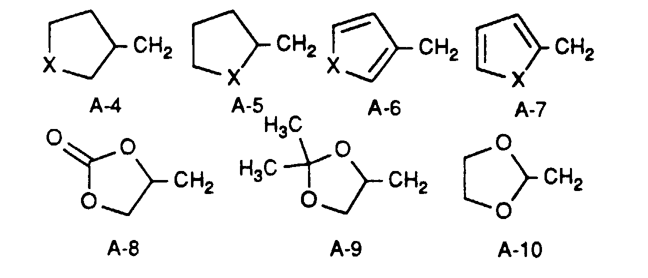
worin
- X: O, S, S(O) oder SO₂;
- R¹: Wasserstoff oder (C₁-C₃)-Alkyl;
- R²: Wasserstoff, Halogen, vorzugsweise Chlor, (C₁-C₃)-Alkyl, oder (C₁-C₃)-Alkoxy, wobei die beiden letztgenannten Reste unsubstituiert oder ein- oder mehrfach durch Halogen oder (C₁-C₃)-Alkoxy substituiert sind;
- R³: Wasserstoff, Halogen, vorzugsweise Chlor, (C₁-C₃)-Alkyl, (C₁-C₃)-Alkoxy, oder (C₁-C₃)-Alkylthio, wobei die vorgenannten alkylhaltigen Reste unsubstituiert oder ein- oder mehrfach durch Halogen oder ein- oder zweifach durch (C₁-C₃)-Alkoxy oder (C₁-C₃)-Alkylthio substituiert sind; oder einen Rest der Formel NR⁵R⁶, (C₃-C₆)-Cycloalkyl, (C₂-C₄)-Alkenyl, (C₂-C₄)-Alkinyl, (C₃-C₄)-Alkenyloxy oder (C₃-C₆)-Alkinyloxy;
- R⁴: Wasserstoff, (C₁-C₄)-Alkyl oder (C₁-C₄)-Alkoxy und
- R⁵ und R⁶: unabhängig voneinander Wasserstoff, (C₁-C₄)-Alkyl, (C₃-C₄)-Alkenyl, (C₁-C₄)-Haloalkyl oder (C₁-C₄)-Alkoxy bedeuten.

In der Formel (I) und im folgenden können Alkyl-, Alkoxy-, Haloalkyl-, Alkylamino- und Alkylthioreste, sowie die entsprechenden ungesättigten und/oder substituierten Reste jeweils geradkettig oder verzweigt sein. Alkylreste, auch in zusammengesetzten Bedeutungen wie Alkoxy, Haloalkyl usw. bedeuten beispielsweise Methyl-, Ethyl-, n- oder i-Propyl, n-, i-, t- oder 2-Butyl usw. Alkenyl- und Alkinylreste haben die Bedeutung der den Alkylresten entsprechenden möglichen ungesättigten Reste, wie z. B. 2-Propenyl, 2- oder 3-Butenyl, 2-Propinyl, 2- oder 3-Butinyl. Halogen bedeutet Fluor, Chlor, Brom oder lod. Aryl bedeutet vorzugsweise einen carbocyclischen oder heterocyclischen aromatischen Ring, der gegebenenfalls noch mit einem aliphatischen oder aromatischen Ring kondensiert sein kann; Aryl ist insbesondere Phenyl. Substituiertes Phenyl bedeutet Phenyl, das durch einen oder mehrere, vorzugsweise einen bis drei Reste aus der Gruppe Halogen, (C₁-C₄)-Alkyl, (C₁-C₄)-Alkoxy, (C₁-C₄)-Haloalkyl, (C₁-C₄)-Thioalkyl, (C₂-C₅)-Alkoxycarbonyl, (C₂-C₅)-Alkylcarbonyloxy, Carbonamid, (C₂-C₅)-Alkylcarbonylamino, (C₂-C₅)-Alkylaminocarbonyl, Di-[(C₁-C₄)-Alkyl]-aminocarbonyl und Nitro substituiert ist. Entsprechendes gilt für substituiertes Aryl.

Die Verbindungen der Formel (I) können Salze bilden, bei denen der Wasserstoff der -SO₂-NH-Gruppe durch ein für die Landwirtschaft geeignetes Kation ersetzt wird. Diese Salze sind beispielsweise Metall-, insbesondere Alkali- oder Erdalkalisalze, oder auch Ammoniumsalze oder Salze mit organischen Aminen. Ebenso kann Salzbildung durch Anlagerung einer starken Säure an den Heterocyclenteil der Verbindungen der Formel (I) erfolgen. Geeignete Säuren hierfür sind z.B. HCI, HNO₃, Trichloressigsäure, Essigsäure oder Palmitinsäure.

Manche Verbindungen der Formel (I) können ein oder mehrere asymmetrische C-Atome oder auch Doppelbindungen enthalten, die in den allgemeinen Formel (I) nicht gesondert angegeben sind. Die durch ihre spezifische Raumform definierten möglichen Stereoisomeren, wie Enantiomere, Diastereomere, Z- und E-lsomere sind jedoch alle von den Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden. Die genannten Stereoisomeren in reiner Form als auch ihre Gemische sind somit Gegenstand dieser Erfindung.

Von besonderem Interesse sind erfindungsgemäße Verbindungen der Formel (I) oder deren Salze, worin
R Wasserstoff; (C₁-C₆)-Alkyl; (C₂-C₆)-Alkenyl; (C₂-C₆)-Alkinyl; (C₁-C₄)-Alkyl, das ein- bis vierfach, vorzugsweise einfach, durch Reste aus der Gruppe Halogen, (C₁-C₂-Alkoxy-, (C₁-C₂)-Thioalkyl, (C₂-C₃)-Alkoxycarbonyl und (C₂-C₄)-Alkenyl substituiert ist; (C₅-C₆)-Cycloalkyl, das unsubstituiert oder durch Reste aus der Gruppe (C₁-C₄)-Alkyl, (C₁-C₄)-Alkoxy, (C₁-C₄)-Alkylthio und Halogen substituiert ist; (C₅-C₆)-Cycloalkenyl; Benzyl, das im Phenylrest unsubstituiert oder durch einen bis drei Reste aus der Gruppe Halogen, (C₁-C₂)-Alkyl, (C₁-C₂)-Alkoxy, (C₁-C₂)-Haloalkyl, (C₁-C₂)-Thioalkyl und (C₂-C₄)-Alkoxycarbonyl substituiert ist, oder einen Rest der genannten Formeln A-1 bis A-10, worin
X O, S, S(O) oder SO₂, vorzugsweise O,
bedeuten.

Von besonderem Interesse sind erfindungsgemäße Verbindungen der Formel (I) oder deren Salze, worin -
- R¹: Wasserstoff oder CH₃;
- R²: Wasserstoff, Halogen, vorzugsweise Chlor, (C₁-C₂)-Alkyl, oder (C₁-C₂)-Alkoxy, wobei die beiden letztgenannten Reste unsubstituiert oder ein- oder mehrfach durch Halogen oder (C₁-C₃)-Alkoxy substituiert sind;
- R³: Wasserstoff, Halogen, vorzugsweise Chlor, (C₁-C₂)-Alkyl, (C₁-C₂)-Alkoxy oder (C₁-C₂)-Alkylthio, wobei die vorgenannten alkylhaltigen Reste unsubstituiert oder ein- oder mehrfach durch Halogen oder ein- oder zweifach durch (C₁-C₂)-Alkoxy oder (C₁-C₂)-Alkylthio substituiert sind; oder einen Rest der Formel NR⁵R⁶;
- R⁴: Wasserstoff oder (C₁-C₂)-Alkyl und
- R⁵ und R⁶: unabhängig voneinander Wasserstoff oder (C₁-C₂)-Alkyl bedeuten.

Bevorzugt sind erfindungsgemäße Verbindungen der Formel (I) oder deren Salze, bei denen
- W: Sauerstoff und
- R¹: Wasserstoff oder CH₃ bedeuten.

Besonders bevorzugt sind Verbindungen der Formel (I) oder deren Salze, in denen
- Y: CH oder N,
- Z: N und
- R²: Wasserstoff, CH₃, CH₂CH₃, OCH₃, OCH₂CH₃, OCHF₂ oder Cl und
- R³: Wasserstoff, CH₃, CH₂CH₃, OCH₃, OCH₂CH₃, OCHF₂, NH(CH₃), N(CH₃)₂, CF₃, OCH₂CF₃ oder Cl sind.

Bevorzugt sind auch solche erfindungsgemäßen Verbindungen, welche eine Kombination der obengenannten bevorzugten Merkmale aufweisen.

Weiterer Gegenstand der vorliegenden Erfindung sind Verfahren zur Herstellung der Verbindungen der allgemeinen Formel (I) oder deren Salze, dadurch gekennzeichnet, daß man
a) eine Verbindung der Formel (II) 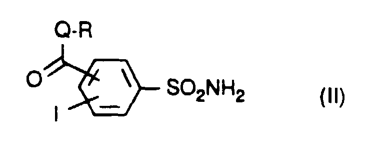 mit einem heterocyclischen Carbamat der Formel (III), 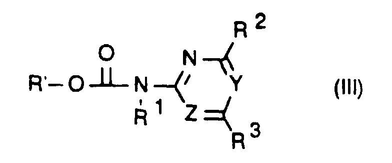 worin R' unsubstituiertes oder substituiertes Aryl oder Alkyl, vorzugsweise unsubstituiertes oder substituiertes Phenyl oder (C₁-C₄)-Alkyl, insbesondere Phenyl oder Methyl ist, umsetzt oder
b) ein Phenylsulfonylcarbamat der Formel (IV) 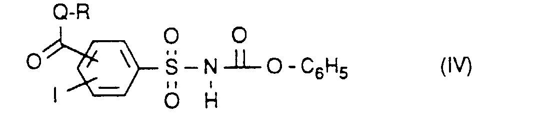 mit einem Aminoheterocyclus der Formel (V) 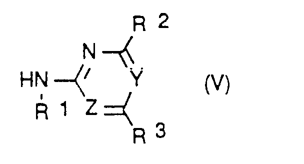 umsetzt oder
c) ein Sulfonylisocyanat der Formel (VI) 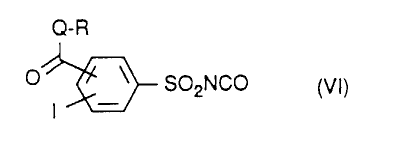 mit einem Aminoheterocyclus der unter b) genannten Formel (V) umsetzt.

Die Umsetzung der Verbindungen der Formel (II) und (III) erfolgt basenkatalysiert in einem inerten Lösungsmittel, wie z. B. Acetonitril, Dioxan oder Tetrahydrofuran bei Temperaturen zwischen 0°C und dem Siedepunkt des Lösungsmittels. Als Base wird bevorzugt 1.8-Diazabicyclo[5.4.0]undec-7-en (DBU) verwendet.

Die Sulfonamide (II) sind weitgehend neu; aus FR-A-2493702 ist die Verbindung 3-Aminosulfonyl-4-iodbenzoesäure und deren Verwendung als Antivirusmittel bekannt. Die neuen Verbindungen (II) und ihre Herstellung sind ebenfalls Gegenstand der Erfindung (siehe weiter unten Tabellen 1a und 1b).
Man erhält sie ausgehend von entsprechenden Sulfonsäurehalogeniden, bevorzugt entsprechenden Sulfochloriden, die entweder direkt mit Ammoniak oder mit tert.-Butylamin und anschließender Schutzgruppenabspaltung, z. B. durch Behandlung mit Trifluoressigsäure, zu den Sulfonamiden der Formel (II) abreagieren. Die in dem Verfahren einsetzbaren Sulfonsäurehalogenide können aus den entsprechenden Anilinen durch Diazotierung und Austausch der Diazogruppe mit Schwefeldioxid in Gegenwart eines Katalysators wie Kupfer(I)chlorid in Salzsäure oder Essigsäure erhalten werden, vgl. Meerwein, Chem. Ber. **90**, 841-52 (1957). Die Carbamate der Formel (III) können nach Methoden hergestellt werden, die in den südafrikanischen Patentanmeldungen 82/5671 und 82/5045 (oder EP-A-0072347 bzw. EP-A-0070802) beschrieben sind.

Die Umsetzungen der Verbindungen (IV) mit den Aminoheterocyclen (V) führt man vorzugsweise in inerten, aprotischen Lösungsmitteln, wie z. B. Dioxan, Acetonitril oder Tetrahydrofuran, bei Temperaturen zwischen 0°C und der Siedetemperatur des Lösungsmittels durch. Die benötigten Ausgangsverbindungen der Formel (V) sind bekannt oder lassen sich nach im Prinzip bekannten Verfahren herstellen, s. "The Chemistry of Heterocyclic Compounds", Bd. XVI, (1962), Interscience Publ., New York & London, und Supplement I dieses Handbuches. Amino-substituierte Triazinderivate werden von Smolin und Rapaport in "The Chemistry of Heterocyclic Compounds", Bd. XIII, (1959), Interscience Publ., New York & London, referiert. Die iodierten Phenylsulfonylcarbamate (IV) erhält man analog Verfahren, die in EP-A-0044808 oder EP-A-0237292 angegeben sind.

Die iodierten Arylsulfonylisocyanate der Formel (VI) sind neue Verbindungen und ebenfalls Gegenstand der Erfindung. Sie lassen sich analog Verfahren aus EP-A-0184385 herstellen und mit den obengenannten Aminoheterocyclen der Formel (V) umsetzen.

Die Salze der Verbindungen der Formel (I) werden vorzugsweise in inerten Lösungsmitteln, wie z. B. Wasser, Methanol, Dichlormethan oder Aceton bei Temperaturen von 0°-100° hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin. Als Säuren zur Salzbildung eignen sich besonders HCl, HNO₃, Trichloressigsäure, Essigsäure oder Palmitinsäure.

Die erfindungsgemäßen Verbindungen der Formel (I) weisen eine ausgezeichnete herbizide Wirksamkeit gegen ein breites Spektrum wirtschaftlich wichtiger mono- und dikotyler Schadplanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z. B. Avena, Lolium, Alopecurus, Phalaris, Echinochloa, Digitaria, Setaria etc., sowie Cyperusarten aus der annuellen Gruppe und auf Seiten der perennierenden Species Agropyron, Cynodon, Imperata, sowie Sorghum etc. und auch ausdauernde Cyperusarten gut erfaßt.

Bei dikotylen Unkrautarten erstreckt sich das Wirkungsspektrum auf Arten, wie z.B. Galium, Viola, Veronica, Lamium, Stellaria, Amaranthus, Sinapis, Ipomoea, Matricaria, Abutilon, Sida etc. auf der annuellen Seite sowie Convolvulus, Cirsium. Rumex, Artemisia etc. bei den perennierenden Unkräutern.

Unter den spezifischen Kulturbedingungen im Reis vorkommende Unkräuter, wie z.B. Sagittaria, Alisma, Eleocharis, Scirpus, Cyperus etc., werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstopp ein, und die Unkrautpflanzen bleiben in dem zum Applikationszeitpunkt vorhandenen Wuchsstadium stehen oder sterben nach einer gewissen Zeit mehr oder weniger schnell ab, sodaß auf diese Weise eine für die Kulturpflanzen schädliche Unkrautkonkurrenz sehr früh und nachhaltig durch den Einsatz der neuen erfindungsgemäßen Verbindungen beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen, wie z. B. Weizen, Gerste, Roggen, Mais, Reis, Zuckerrüben, Baumwolle und Soja, nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Emteerleichterung, wie z.B. durch Auslösen von Desikkation, Abszission und Wuchsstauchung eingesetzt werden. Des weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagem hierdurch verringert oder völlig verhindert werden kann.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW) wie Öl-in-Wasser- und Wasser-in-Öl-Emulsionen, versprühbare Lösungen oder Emulsionen, Suspensionskonzentrate (SC), Dispersionen auf Öl- oder Wasserbasis, ölmischbare Lösungen, Suspoemulsionen, Kapselsuspensionen (CS), Stäubemittel (DP), Beizmittel, Granulate zur Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7. G. Hauser Verlag München, 4. Aufl. 1986; van Valkenburg, "Pesticides Formulations", Marcel Dekker N.Y., 2nd Ed. 1972-73; K Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Galdwell NJ.; H.v. Olphen, "Introduction to Clay Colloid Ghemistry"; 2nd Ed., J. Wiley & Sons, N.Y.; Marsden, "Solvents Guide", 2nd Ed., Interscience, N.Y. 1950; McCutcheon's "Detergents and Emulsifiers Annual", MG Publ. Corp., Ridgewood N.J.; Sisley and Wood, "Encyclopedia of Surface Active Agents", Chem. Publ. Co. Inc., N.Y 1964; Schönfeldt, "Grenzflächenaktive Äthylenoxidaddukte", Wiss. Verlagsgesell., Stuttgart 1976; Winnacker-Küchler, "Chemische Technologie", Band 7, G. Hauser Verlag München, 4. Aufl. 1986.

Spritzpulver sind in Wasser gleichmäßig dispergierbare Präparate, die neben dem Wirkstoff außer einem Verdünnungs- oder Inertstoff noch Netzmittel, z.B. polyoxyethylierte Alkylphenole, polyoxethylierte Fettalkohole und Fettamine, Fettalkoholpolyglykolethersulfate, Alkansulfonate oder Alkylarylsulfonate, und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Alkylpolyglycolether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.
Teller-, Fließbett-, Extruder- und Sprühgranulate können nach üblichen Verfahren hergestellt werden; siehe z.B. Verfahren in "Spray Dyring Handbook", 3rd Ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, s. 8-57.

Für weitere Informationen zur Formulierung von Pflanzenschutzmitteln siehe z.B. G.G. Klingman, "Weed Control as a Science", John Wiley and Sons, Inc., New York,1961, Seiten 81-96 und J.D. Freyer' s. A. Evans, "Weed Control Handbook", 5th Ed., Blackwell Scientific Publications, Oxford, 1968, Seiten 101-103.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 80 Gew.-% betragen. Staubförmige Formulierungen enthalten meistens 1 bis 20 Gew.-% an Wirkstoff, versprühbare Lösungen etwa 0,2 bis 20 Gew.-%. Bei Granulaten hängt der Wirkstoffgehalt zum Teil davon ab, ob die wirksame Verbindung flüssig oder fest vorliegt. Meist liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Auf der Basis dieser Formulierungen lassen sich auch Kombinationen mit anderen im Pflanzenbau wirksamen Stoffen, z.B. Pestiziden, wie Insektiziden, Akariziden, Fungiziden und Herbiziden, und/oder Düngemitteln und/oder Wachstumsregulatoren herstellen, z.B. in Form einer Fertigformulierung oder als Tankmix.

Insbesondere können die erfindungsgemäßen Verbindungen der Formel (I) mit weiteren Herbiziden angewendet werden, wie sie z.B. aus Weed Research 26, 441-5 (1986) oder "The Pesticide Manual", 9th Edition The British Crop Protection Council, 1990, England, bekannt sind. Als Beispiele für literaturbekannte Herbizide, die erfindungsgemäß mit den Verbindungen der Formel (I) kombiniert werden können, sind folgende Wirkstoffe zu nennen (für die Wirkstoffe ist jeweils der Common Name oder Firmencode in Fettdruck und anschließend die chemische Bezeichnung in Normalschrift angegeben, siehe Schema):

### Common Name (bzw. Firmencode) Chemischer Name [Schema]

**AC 263222** 2-[4,5-dihydro-4-methyl-4-(1-methylethyl)-5-oxo-1H-imidazol-2-yl]-5-methyl-3-pyridine carboxylic acid;
**acetochlor** 2-chloro-N-(ethoxymethyl)-N-(2-ethyl-6-methylphenyl)-acetamide;
**acifluorfen** 5-[2-chloro-4-(trifluoromethyl)-phenoxy]-2-nitrobenzoic acid;
**aclonifen** 2-chloro-6-nitro-3-phenoxyaniline;
**AKH 7088** methyl [[[1-[5-[2-chloro-4-(trifluoromethyl)phenoxy]-2-nitrophenyl]-2 -methoxyethylidene]-amino]-oxy]-acetate;
**alachlor** 2-chloro-N-(2,6-diethylphenyl)-N-(methoxymethyl)-acetamide;
**alloxydim** methyl 3-[1-(allyloxyimino)-butyl]-4-hydroxy-6,6-dimethyl-2-cyclohex-3-ene-carboxylate;
**ametryn** N-ethyl-N'-(1 -methylethyl)-6-(methylthio)- 1,3,5-triazine-2,4-diamine;
**amidosulfuron** 1-[N-Methyl-N-(methylsulfonyl)-aminosulfonyl]-3-(4,6-dimethoxy-pyrimidin-2-yl)urea;
**amitrole** 1H-1,2,4-triazol-3-amine;
**AMS** Ammonium sulfamate;
**anilofos** 5-[2-[(4-chlorophenyl)(1-methylethyl)amino]-2-oxoethyl]O,O-dimethyl phosphorodithioate;
**asulam** methyl [(4-aminophenyl)sulfonyl]carbamate;
**atrazine** 6-chloro-N-ethyl-N'-(1-methylethyl)-1,3,5-triazine-2,4-diamine;
**aziprotryne** 2-azido-N-(1-methylethyl)-6-methylthio-1,3,5-triazin-2-amine;
**barban** 4-chloro-2-butynyl 3-chlorophenylcarbamate;
**BAS 516 H** 5-fluoro-2-phenyl-4H-3,1-benzoxazin-4-one;
**benazolin** 4-chloro-2-oxo-3(2H)-benzothiazoleacetic acid;
**benfluralin** N-butyl-N-ethyl-2,6-dinitro-4-(trifluoromethyl)benzenamine;
**benfuresate** 2,3-dihydro-3,3-dimethylbenzofuran-5-yl ethanesulfonate;
**bensulfuron-methyl** 2-[[[[(4,6-dimethoxy-2-pyrimidinyl)-amino]-carbonyl]-amino]-sulfonyl]-methyl]-benzoic acid, methyl ester;
**bensulide** O,O-bis-(1-methylethyl) S-[2-[(phenylsulfonyl)-amino]-ethyl] phosphorodithioate;
**bentazone** 3-(1-methylethyl)-1H-2,1,3-benzothiadiazin-4(3H)-one, 2,2-dioxide;
**benzofenap** 2-[[4(2,4-dichloro-3-methylbenzoyl)-1,3-dimethyl-1H-pyrazol-5-yl]oxy]-1-(4-methylphenyl)ethanone;
**benzofluor** N-[4-(ethylthio)-2-(trifluoromethyl)phenyl]methanesulfoneamide;
**benzoylprop-ethyl** N-benzoyl-N-(3,4-dichlorophenyl)-alanine, ethyl ester;
**benzthiazuron** N-2-benzothiazolyl-N'-methylurea;
**bialaphos** 4-(hydroxymethylphosphinyl)-L-2-aminobutanoyl-L-alanyl-L-alanine;
**bifenox** methyl 5-(2,4-dichlorophenoxy)-2-nitrobenzoate;
**bromacil** bromo-6-methyl-3-(1-methylpropyl)-2,4(1H,3H)pyrimidinedione;
**bromobutide** N-[(1,1-dimethyl)methylphenyl]-2-bromo-3,3-dimethylbutyramide;
**bromofenoxim** 3,5-dibromo-4-hydroxybenzaldehyde O-(2,4-dinitrophenyl)oxime;
**bromoxynil** 3,5-dibromo-4-hydroxybenzonitrile;
**bromuron** N'-(4-bromophenyl)-N,N-dimethylurea;
**buminafos** dibutyl [1-(butylamino)cyclohexyl]phosphonate;
**butachlor** N-(butoxymethyl)-2-chloro-N-(2,6-diethylphenyl)acetamide;
**butamifos** O-ethyl O-(5-methyl-2-nitrophenyl) (1-methylpropyl)-phosphoramidothioate;
**butenachlor** (Z)-N-but-2-enyloxymethyl-2-chloro-2',6'-diethylacetanilide;
**busoxinone** 3-[5-(1,1-dimethylethyl)-isoxazo1-3-yl)-4-hydroxy 1-methyl-2-imidazolidinone;
**buthidazole** 3-[5-(1,1-dimethylethyl)-1,3,4-thiadiazol-2-yl]-4-hydroxy-1-methyl-2-imidazolidinone;
**butralin** 4-(1,1-dimethylethyl)-N-(1-methylpropyl)-2,6-dinitrobenzenamine;
**butylate** S-ethyl bis(2-methylpropyl)carbamothioate;
**C** 4874 2-[4-[(6-chloro-2-quinoxalinyl)oxy]phenoxy]propanoic acid, (tetrahydro-2-furanyl)methyl ester;
**carbetamide** (R)-N-ethyl-2-[[(phenylamino)carbonyl]oxy]propanamide;
**CDAA** 2-chloro-N,N-di-2-propenylacetamide;
**CDEC** 2-chloroallyl diethyldithiocarbamate;
**CGA** 184927 2-[4-[(5-chloro-3-fluoro-2-pyridinyl)oxy]phenoxy]propanoic acid, 2-propynyl ester;
**chlomethoxyfen** 4-(2,4-dichlorophenoxy)-2-methoxy- -nitrobenzene;
**chloramben** 3-amino-2,5-dichlorobenzoic acid;
**chlorbromuron** 3-(4-bromo-3-chlorophenyl)-1-methoxy-1-methylurea;
**chlorbufam** 1-methyl-2-propynyl (3 chlorophenyl)carbamate;
**chlorfenac** 2,3,6-trichlorobenzeneacetic acid;
**chlorflurecol-methyl** 2-chloro-9-hydroxy-9H-fluorene-9-carboxylic acid, methyl ester;
**chloridazon** 5-amino-4-chloro-2-phenyl-3(2H)-pyridazinone;
**chlorimuron ethyl** 2-[[[[(4-chloro-6-methoxy-2-pyrimidinyl)-amino]-carbonyl]-ami no]-sulfonyl]-benzoic acid, ethyl ester;
**chlornitrofen** 1,3,5-trichloro-2-(4-nitrophenoxy)benzene;
**chlorotoluron** N'-(3-chloro-4-methylphenyl)-N,N-dimethylurea;
**chloroxuron** N'-[4-(4-chlorophenoxy)phenyl]-N,N-dimethylurea;
**chlorpropham** 1-methylethyl 3-chlorophenylcarbamate;
**chlorsulfuron** 2-chloro-N-[[(4-methoxy-6-methyl-1,3,5-triazin-2-yl)amino]-carbonyl]-benzenesulfonamide;
**chlorthal-dimethyl** 2,3,5,6-tetrachloro-1,4-benzenedicarboxylic acid, dimethyl ester;
**chlorthiamid** 2,6-dichlorobenzenecarbothioamide;
**cinmethylin** exo-1-methyl-4-(1-methylethyl)-2-[(2-methylphenyl)methoxy]-7-oxabicyclo[2.2.1]heptane;
**cinosulfuron** 1-(4,6-dimethoxy-1,3,5-triazin-2-yl)3-[2-(2-methoxyethoxy)-phenylsulfonyl]-urea;
**clethodim** (E,E)-2-[1-[[(3-chloro-2-propenyl)-oxy]-imino]-propyl]-5-[2-(ethylthio)propyl]-3-hydroxy-2-cyclohexen-1-one;
**clomazone** 2-[(2-chlorophenyl)methyl]4,4-dimethyl-3-isoxazolidinone;
**clomeprop** [(2,4-dichloro-3-methylphenyl)oxy]-2-propionic acid anilide;
**cloproxydim** (E,E)-2-[1-[[(3-chloro-2-propenyl)-oxy]-imino]-butyl]-5-[2-(ethylthio)-propyl]-3-hydroxy-2-cyclohexen-1-one;
**clopyralid** 3,6-dichloro-2-pyridinecarboxylic acid;
**cyanazine** 2-[[4-chloro-6-(ethylamino)-1,3,5-triazin-2-yl]amino]-2-methylpropanenitrile;
**cycloate** S-ethyl cyclohexylethylcarbamothioate;
**cycloxydim** 2-[1-(ethoxyimino)butyl]-5-(tetrahydrothiopyran-3-yl)-3-hydroxy-2-cyclohexen-1-one;
**cycluron** 3-cyclooctyl-1-dimethylurea;
**cyperquat** 1-methyl-4-phenylpyridimum;
**cyprazine** 2-chloro-4-(cyclopropylamino)-6-(isopropylamino)-s-triazine;
**cyprazole** N-[5-(2-chloro-1,1-dimethylethyl)-1,3,4-thiadiazol-2-yl]-cyclopropane-carboxamide;
**2,4-DB** 4-(2,4-dichlorophenoxy)butanoic acid;
**dalapon** 2,2-dichloropropanoic acid;
**desmedipha** methyl [3-[[(phenylamino)carbonyl]oxy]phenyl]carbamate;
**desmetryn** 2-(isopropylamino)-4-(methylamino)-6-(methylthio)-s-triazine;
**di-allate** S-(2,3-dichloro-2-propenyl)bis(1-methylethyl)carbamothioate;
**dicamba** 3,6-dichloro-2-methoxybenzoic acid;
**dichlobenil** 2,6-dichlorobenzonitrile;
**dichlorprop** 2-(2,4-dichlorophenoxy)propanoic acid;
**diclofop-methyl** 2-[4-(2,4-dichlorophenoxy)phenoxy]propanoic acid, methyl ester;
**diethatyl** N-(chloroacetyl)-N-(2,6-diethylphenyl)glycine;
**difenoxuron** N'-[4-(4-methoxyphenoxy)phenyl]-N,N-dimethylurea;
**difenzoquat** 1,2-dimethyl-3,5-diphenyl-1H-pyrazolium;
**diflufenican** N-(2,4-difluorophenyl)-2-[3-(trifluoromethyl)-phenoxy]-3-pyridine-carboxamide;
**dimefuron** N-[3-chloro-4-[5-(1,1-dimethylethyl)-2-oxo-1,3,4-oxadiazol-3(2H)-yl]phenyl]-N,N-dimethylurea;
**dimethachlor** 2-chloro-N-(2,6-dimethylphenyl)-N-(2-methoxyethyl)-acetamide;
**dimethametryn** N-(1,2-dimethylpropyl)-N' ethyl-6-(methylthio)- 1,3,5-triazine-2,4-diamine;
**dimethipin** 2,3-dihydro-5,6-dimethyl-1,4-dithiin, 1,1,4,4-tetraoxide;
**dinitramine** N³,N³-diethyl-2,4-dinitro-6-(trifluoromethyl)-1,3-benzenediamine;
**dinoseb** 2-(1-methylpropyl)-4,6-dinitrophenol;
**dinoterb** 2-(1,1-dimethylethyl)4,6-dinitrophenol;
**diphenamid** N,N-dimethyl-2,2-diphenylacetamide;
**dipropetryn** 6-ethylthio-N,N'-bis(-methylethyl)-1,3,5-triazine-2,4-diamine;
**diquat** 6,7-dihydrodipyrido[1,2-a:2',1'-c]pyrazinediium;
**dithiopyr** 2-(difluoromethyl)4-(2-methylpropyl)-6-(trifluoromethyl)-3,5-pyridine-dicarbothioic acid;
**diuron** N'-(3,4-dichlorophenyl)-N,N-dimethylurea;
**DNOC** 2-methyl-4,6-dinitrophenol;
**DPX-A7881** 2-[[[[(4-ethoxy-6-N-(methyl)amino-1,3,5-triazine-2-yl]-amino]-carbonyl]-amino]-sulfonyl]-benzoic acid, methyl ester;
**DPX-E9636** N-[[(4,6-dimethoxy-2-pyrimidinyl)-amino]-carbonyl]-3-(ethylsulfonyl)-2-pyridinesulfonamide;
**dymron** N-(4-methylphenyl)-N'-(-methyl-1-phenylethyl)urea;
**eglinazine-ethyl** N-[4-chloro-6-(ethylamino)-1,3,5-triazin-2-yl]-glycine ethyl ester;
**EL 177** 5-cyano-1-(1,1-dimethylethyl)-N-methyl-3H-pyrazole-4-carbo xamide;
**endothal** 7-oxabicyclo[2.2.1]heptane-2,3-dicarboxylic acid;
**EPTC** S-ethyl dipropylcarbamothioate;
**esprocarb** S-(methylphenyl) N-ethyl-N-(1,2-dimethyl)propylcarbamothioate;
**ethalfluralin** N-ethyl-N-(2-methyl-2-propenyl)-2,6-dinitro-4-(trifluoromethyl)-benzenamine:
**ethidimuron** N-[5-(ethylsulfonyl)-1,3,4-thiadiazol-2-yl]-N,N'-dimethylurea;
**ethiozin** 4-amino-6-(1,1-dimethylethyl)-3-(ethylthio)-1,2,4-triazin-5(4H)-one;
**ethofumesate** 2-ethoxy-2,3-dihydro-3,3-dimethyl-5-benzofuranyl methane-sulfonate;
**F 5231** N-[2-chloro-4-fluoro-5-[4-(3-fluoropropyl)-4,5-dihydro-5-oxo-1H-tetrazol-1-yl]-phenyl]-ethane-sulfon-amide;
**fenoprop** 2-(2,4,5-trichlorophenoxy)propanoic acid;
**fenoxaprop-ethyl** 2-[4-[(6-chloro-2-benzoxazolyl)-oxy]-phenoxy]-propanoic acid, ethyl ester;
**fenuron** N,N-dimethyl-N'-phenylurea;
**flamprop-methyl** N-benzoyl-N-(3-chloro-4-fluorophenyl)alanin, methyl ester;
**flazasulfuron** 1-(4,6-dimethoxypyrimidin-2-yl)-3-[3-(trifluoromethyl)-2-pyridylsulfonyl]-urea;
**fluazifop-butyl** 2-[4-[[5-(trifluoromethyl)-2-pyridinyl]oxy]phenoxy]propanoic acid, butyl ester;
**fluchloralin** N-(2-chloroethyl)-2,6-dinitro-N-propyl-4-(trifluoromethyl)-benzenamine;
**flumeturon** N,N-dimethyl-N'-[3-(trifluoromethyl)phenyl]urea;
**flumipropyn** 2-[4-chloro-2-fluoro-5-[(1-methyl-2-propynyl)oxy]phenyl-4,5,6,7-tetrahydro-1H-isoindole-1,3(2H)-dione;
**fluorodifen** 2-nitro-1-(4-nitrophenoxy)4-(trifluoromethyl)benzene;
**fluoroglycofen-ethyl** carboxymethyl 5-[2-chloro-4-(trifluoromethyl)phenoxy]-2-nitrobenzoate, ethyl ester;
**fluridone** 1-methyl-3-phenyl-5-[3-(trifluoromethyl)phenyl]-4(1H)-pyridinone;
**flurochloridone** 3-chloro-4-(chloromethyl)-1-[3-(trifluoromethyl)phenyl]-2-pyrrolidinone;
**fluroxypyr** 4-amino-3,5-dichloro-6-fluoro-2-pyridyloxyacetic acid;
**flurtamone** 5-(methylamino)-2-phenyl-4-[3-(trifluoromethyl)phenyl]-3(2H)-furanone;
**fomesafen** 5-[2-chloro-4-(trifluoromethyl)phenoxy]-N-(methylsulfonyl)-2-nitrobenzamide;
**fosamine** ethyl hydrogen carbamoylphosphonate;
**furyloxyfen** 3-[5-[2-chloro4-(trifluoromethyl)-phenoxy]-2-nitrophenoxy]-tetrahydrofuran;
**glufosinate** 4-[hydroxy(methyl)phosphinoyl]-homoalanine;
**glyphosate** N-(phosphonomethyl)glycine;
**halosaten** 5-[6-chloro-2-fluoro-4-(trifluoromethyl)-phenxyl]-N-(ethylsulfonyl)-2-nitrobenzamide;
**haloxyfop** 2-[4 [[3-chloro-5-(trifluoromethyl)-2-pyridinyl]-oxy]-phenoxy]-propanoic acid;
**hexazinone** 3-cyclohexyl-6-(dimethylamino)-1-methyl-1,3,5-triazine-2,4(1H,3H)-dione;
**Hw** 52 N-(2,3-dichlorophenyl)-4-(ethoxymethoxy)benzamide;
**imazamethabenz-methyl** 6-(4-isopropyl-4-methyl-5-oxo-2-imidazolin-2-yl)-m-toluic acid, methyl ester and 6-(4-isopropyl-4-methyl-5-oxo-2-imidazolin-2-yl)-p-toluic acid, methyl ester;
**imazapyr** 2-[4,5-dihydro-4-methyl-4-(1-methylethyl)-5-oxo-1H-imidazol-2-yl]-3-pyridinecarboxylic acid;
**imazaquin** 2-[4,5-dihydro-4-methyl-4-(1-methylethyl)-5-oxo-1H-imidazol-2-yl]-3-quinolinecarboxylic acid;
**imazethapyr** 2-[4,5-dihydro-4-methyl-4-(1-methylethyl)-5-oxo-1H-imidazol-2-yl]-5-ethyl-3-pyridinecarboxylic acid;
**imazosulfuron** 2-chloro-N-[[(4,6-dimethoxy-2-pyrimidinyl)-amino]-carbonyl]-imidazo[1,2-a]pyridine-3-sulfonamide;
**ioxynil** 4-hydroxy-3,5-diiodobenzonitrile;
**isocarbamid** N-(2-methylpropyl)-2-oxo-1-imidazolidinecarboxamide;
**isopropalin** 4-(1-methylethyl)-2,6-dinitro-N,N-dipropylbenzenamine;
**isoproturon** N-[4-(methylethyl)phenyl]-N',N'-dimethylurea;
**isouron** N'-[5-(1,1-dimethylethyl)-3-isoxazolyl]-N,N-dimethylurea;
**isoxaben** N-[3-(1-ethyl-1-methylpropyl)-5-isoxazolyl]-2,6-dimethoxybenzamide;
**isoxapyrifop** 2-[2-[4-[(3,5-dichloro-2-pyridinyl)oxy]phenoxy]-1-oxopropyl]isoxazolidine;
**karbutilate** 3-[[(dimethylamino)carbonyl]-amino]-phenyl (1,1-dimethylethyl)-carbamate;
**lactofen** 2-ethoxy-1-methyl-2-oxoethyl 5-[2-chloro-4-(trifluoromethyl)phenoxy]-2-nitrobenzoate;
**lenacil** 3-cyclohexyl-6,7-dihydro-1H-cyclopentapyrimidine-2,4(3H,5H)-dione;
**linuron** N'-(3,4-dichlorophenyl)-N-methoxy-N-methylurea;
**MCPA** (4-chloro-2-methylphenoxy)acetic acid;
**MCPB** 4-(4-chloro-2-methylphenoxy)butanoic acid;
**mecoprop** 2-(4-chloro-4-methylphenoxy)propanoic acid;
**mefenacet** 2-benzothiazol-2-yloxy-N-methylacetanilide;
**mefluidide** N-[2,4-dimethyl-5-[[(trifluoromethyl)-sulfonyl]-amino]phenyl]-acetamide;
**metamitron** 4-amino-3-methyl-6-phenyl-1,2,4-triazin-5(4H)-one;
**metazachlor** 2-chloro-N-(2,6-dimethylphenyl)-N-(1(H)-pyrazol-1-ylmethyl)acetamide;
**methabenzthiazuron** 1,3-dimethyl-3-(2-benzothiazolyl)urea;
**metham** methylcarbamodithioic acid;
**methazole** 2-(3,4-dichlorophenyl)-4-methyl-1,2,4-oxadiazolidine-3,5-dione;
**methoxyphenone** (4-methoxy-3-methylphenyl)(3-methylphenyl)methanone;
**methyldymron** N-methyl-N'-(1-methyl-1-phenylethyl)-N-phenylurea;
**metobromuron** N'-(4-bromophenyl)-N-methoxy-N-methylurea;
**metolachlor** 2-chloro-N-(2-ethyl-6-methylphenyl)-N-(2-methoxy-1-methylethyl)-acetamide;
**metoxuron** N'-(3-chloro-4-methoxyphenyl)-N,N-dimethylurea;
**metribuzin** 4-amino-6-(1,1-dimethylethyl)-3-(methylthio)-1,2,4-triazin-5(4H)-one;
**metsulfuron-methyl** 2-[[[[(4-methoxy-6-methyl-1,3,5-triazin-2-yl)amino]-carbonyl]-ammo]-sulfonyl]-benzoic acid, methyl ester;
**MH** 1,2-dihydro-3,6-pyridazinedione;
**molinate** S-ethyl hexahydro-1H-azepine-1-carbothioate;
**monalide** N-(4-chlorophenyl)-2,2-dimethylpentanamide;
**monolinuron** 3-(4-chlorophenyl)-1-methoxy-1 -methylurea;
**monuron** N'-(4-chlorophenyl)-N,N-dimethylurea;
**MT 128** 6-chloro-N-(3-chloro-2-prcpenyl)-5-methyl-N-phenyl-3-pyridazinamine;
**MT 5950** N-[3-chloro-4-(1-methylethyl)phenyl]-2-methylpentanamide;
**naproanilide** 2-(2-naphthalenyloxy)-N-phenylpropanamide;
**napropamide** N,N-diethyl-2-(1-naphthalenyloxy)propanamide;
**naptalam** 2-[(1-naphthalenylamino)carbonyl]benzoic acid;
**NC 310** 4-(2,4-dichlorobenzoyl)-1-methyl-5-benzyloxypyrazole;
**neburon** 1-butyl-3-(3,4-dichlorophenyl)-1-methylurea;
**nicosulfuron** 2-[[[[(4,6-dimethoxy-2-pyrimidinyl)-amino]-carbonyl]-amino]sulfonyl]-N,N-dimethyl-3-pyridinecarboxamide;
**nipyraclophen** 5-amino-1-(2,6-dichloro-4-(trifluoromethyl)-phenyl)-4-nitropyrazole;
**nitralin** 4-(methylsulfonyl)-2,6-dinitro-N,N-dipropylaniline;
**nitrofen** 2,4-dichloro-1-(4-nitrophenoxy)benzene;
**nitrofluorfen** 2-chloro-1-(4-nitrophenoxy)-4-(trifluoromethyl)benzene;
**norflurazon** 4-chloro-5-(methylamino)-2-[3-(trifluoromethyl)phenyl]-3(2H)-pyridazinone;
**orbencarb** S-[2-(chlorophenyl)methyl] diethylcarbamothioate;
**oryzalin** 4-(dipropylamino)-3,5-dinitrobenzenesulfonamide;
**oxadiazon** 3-[2,4-dichloro-5-(1-methylethoxy)-phenyl]-5-(1,1-dimethylethyl)-1,3,4-oxadiazol-2(3H)-one;
**oxyfluorfen** 2-chloro-1-(3-ethoxy-4-nitrophenoxy)-4-(trifluoromethyl)-benzene;
**paraquat** 1,1'-dimethyl-4,4'-dipyridinium ion;
**pebulate** S-propyl butylethylcarbamothioate;
**pendimethalin** N-(1-ethylpropyl)-3,4-dimethyl-2,6-dinitrobenzenamme;
**perfluidone** 1,1,1-trifluoro-N-[2-methyl-4-(phenylsulfonyl) phenyl]-methanesulfonamide;
**phenisopham** 3-[[(1-methylethoxy)carbonyl]amino]phenyl ethylphenylcarbamate;
**phenmedipham** 3-[(methoxycarbonyl)amino]phenyl (3-methylphenyl)carbamate;
**picloram** 4-amino-3,5,6-trichloro-2-pyridinecarboxylic acid;
**piperophos** S-[2-(2-methyl-1-piperidinyl)-2-oxoethyl] O,O-dipropyl phosphorodithioate;
**pirifenop-butyl** 2-[4-[(3,5-dichloro-2-pyridinyl)oxy]phenoxy]propanoic acid, butyl ester;
**PPG-1013** 5-[2-chloro-4-(trifluoromethyl)phenoxy]-2-nitroacetophenone oxime-O-acetic acid, methyl ester;
**pretilachlor** 2-chloro-N-(2,6-diethylphenyl)-N-(2-propoxyethyl)-acetamide;
**primisulfuron-methyl** 2-[[[[[4,6-bis(difluoromethoxy)pyrimidin-2-yl]-amino]-carbonyl]-amino]-sulfonyl]-benzoic acid, methyl ester;
**procyazine** 2-[[4-chloro-6-(cyclopropylamino)-,3,5-triazine-2-yl]amino]-2-methylpropane-nitrile;
**prodiamine** 2,4-dinitro-N³,N³-dipropyl-6-(trifluoromethyl)-1,3-benzenediamine;
**profluralin** N-(cyclopropylmethyl)-2,6-dinitro-N-propyl-4-(trifluoromethyl)-benzenamine;
**proglinazine-ethyl** N-[4-chloro-6-[(1-methylethyl)-amino]-1,3,5-triazin-2-yl]-glycine, ethyl ester;
**prometon** 6-methoxy-N,N'-bis( 1 -methylethyl)-1,3,5-triazine-2,4-diamine;
**prometryn** N,N'-bis(1-methylethyl)-6-(methylthio)-1,3,5-triazine-2,4-diamine;
**propachlor** 2-chloro-N-(1-methylethyl)-N-phenylacetamide;
**propanil** N-(3,4-dichlorophenyl)propanamide;
**propaquizafop** 2-[4-[(6-chloro-2-quinoxalinyl)oxy]phenoxy]propanoic acid, 2-[[(1-methylethylidene)amino]oxy]ethyl ester;
**propazine** 6-chloro-N,N'-bis(1-methylethyl)-1,3,5-triazine-2,4-diamine;
**propham** 1-methylethyl phenylcarbamate;
**propyzamide** 3,5-dichloro-N-(1,1-dimethyl-2-propynyl)benzamide;
**prosulfalin** N-[[4-(dipropylamino)-3,5-dinitrophenyl]-sulfonyl]-S,S-dimethylsulfilimine;
**prosulfocarb** S-(phenyl)methyl dipropylcarbamothioate;
**prynachlor** 2-chloro-N-(1-methyl-2-propynyl)acetanilide;
**pyrazolinate** [4-(2,4-dichlorobenzoyl)-1,3-dimethylpyrazol-5-yl]toluene-4-sulfonate;
**pyrazon** 5-amino-4-chloro-2-phenyl-3(2H)-pyridazinone;
**pyrazosulfuron-ethyl** 1-(4,6-dimethoxypyrimidin-2-yl)-3-[[(1-methyl)4-(ethoxycarbonyl)pyrazol-5-yl]sulfonyl]urea;
**pyrazoxyfen** 2-[[4-(2,4-dichlorobenzoyl)-1,3-dimethyl-1H-pyrazol-5-yl]oxy]-1-phenylethanone;
**pyributicarb** O-[3-(1,1-dimethylethyl)-phenyl]-(6-methoxy-2-pyridinyl)-methylcarbamothioate;
**pyridate** O-(6-chloro-3-phenyl-4-pyridazinyl)S-octyl carbonothioate;
**quinclorac** 3,7-dichloro-8-quinolinecarboxylic acid;
**quinmerac** 7-chloro-3-methyl-8-quinolinecarboxylic acid;
**quizalofop-ethyl** 2-[4-[(6-chloro-2-quinoxalinyl)oxy]phenoxy]propanoic acid, ethyl ester;
**S 275** 2-[4-chloro-2-fluoro-5-(2-propynyloxy)-phenyl]-4,5,6,7-tetrahydro-2H-indazole;
**S 482** 2-[7-fluoro-3,4-dihydro-3-oxo4-(2-propynyl)-2H-1,4-benzoxazin-6-yl]-4,5,6,7-tetrahydro-1H-isoindole-1,3(2H)-dione;
**secbumeton** N-ethyl-6-methoxy-N'-(1-methylpropyl)-1,3,5-triazine-2,4-diamine;
**sethoxydim** 2-[1-(ethoxyimino)butyl]-5-[2-(ethylthio)propyl]-3-hydroxy-2-cyclohexen-1-one;
**siduron** N-(2-methylcyclohexyl)-N'-phenylurea;
**simazine** 6-chloro-N,N'-diethyl-1,3,5-triazine-2,4-diamine;
**simetryn** N,N'-diethyl-6-(methylthio)-1,3,5-triazine-2,4-diamine;
**SN 106279** 2-[[7-[2-chloro-4-(trifluoromethyl)-phenoxy]-2-naphthalenyl]-oxy]-propanoic acid, methyl ester;
**sulfometuron-methyl** 2-[[[[(4,6-dimethyl-2-pyrimidinyl)-amino]-carbonyl]-amino]-sulfonyl]-benzoic acid, methyl ester;
**TCA** trichloroacetic acid;
**tebutam** 2,2-dimethyl-N-(1-methylethyl)-N-(phenylmethyl)propanamide;
**tebuthiuron** N-[5-(1,1-dimethylethyl)-1,3,4-thiadiazol-2-yl]-N,N'-dimethylurea;
**terbacil** 5-chloro-3-(1,1-dimethylethyl)-6-methyl-2,4(1 H,3H)-pyrimidinedione;
**terbucarb** 2,6-bis(1,1-dimethylethyl)-4-methylphenyl methylcarbamate;
**terbuchlor** N-(butoxymethyl)-2-chloro-N-[2-(1,1-dimethylethyl)-6-methylphenyl]-acetamide;
**terbumeton** N-(1,1-dimethylethyl)-N'-ethyl-6-methoxy-1,3,5-triazine-2,4-diamine;
**terbuthylazine** 6-chloro-N-(1,1-dimethylethyl)-N'-ethyl-1,3,5-triazine-2,4-diamine;
**terbutryn** N-(1,1-dimethylethyl)-N'-ethyl-6-(methylthio)-1,3,5-triazine-2,4-diamine;
**TFH 450** N,N-diethyl-3-[(2-ethyl-6-methylphenyl)-sulfonyl]-1H-1,2,4-triazole-1-carboxamide;
**thiazafluron** N,N'-dimethyl-N-[5-(trifluoromethyl)-1,3,4-thiadiazol-2-yl]-urea;
**thifensulfuron-methyl** 3-[[[[(4-methoxy-6-methyl-1,3,5-triazin-2-yl)-amino]-carbonyl]-amino]-sulfonyl]-thiophene-carboxylic acid, methyl ester;
**thiobencarb** S-[(4-chlorophenyl)-methyl]-diethylcarbamothioate;
**tiocarbazil** S-(phenylmethyl)-bis(1-methylpropyl)-carbamothioate;
**tralkoxydim** 2-[1-(ethoxyimino)-propyl]-5-[2,4,6-trimethylphenyl]-3-hydroxy-2-cyclohexen-1-one;
**tri-allate** S-(2,3,3-trichloro-2-propenyl) bis(1-methylethyl)carbamothioate;
**triasulfuron** 1-(4-methoxy-6-methyl-1,3,5-triazin-2-yl)-3-[2-(2-chloroethoxy)-phenylsulfonyl]-urea;
**triazofenamide** 1-(3-methylphenyl)-5-phenyl-1,2,4-triazole-2-carboxamide;
**tribenuron-methyl** 2-[[[N-(4-methoxy-6-methyl-1,3,5-triazin-2-yl)-N-methylarmino]-carbonyl]-amino]-sulfonyl]-benzoic acid, methyl ester;
**triclopyr** [(3,5,6-trichloro-2-pyridinyl)oxy]acetic acid;
**tridiphane** 2-(3,5-dichlorophenyl)-2-(2,2,2-trichloroethyl)-oxirane;
**trietazine** 6-chloro-N,N,N'-triethyl-1,3,5-triazine-2,4-diamine;
**trifluralin** 2,6-dinitro-N,N-dipropyl-4-(trifluoromethyl)-benzenamine;
**trimeturon** 1-(4-chlorophenyl)-2,3,3-trimethylpseudourea;
**vernolate** S-propyl dipropylcarbamothioate;
**WL 110547** 5-phenoxy-1-[3-(trifluoromethyl)phenyl]-1H-tetrazole.

Der Wirkstoffgehalt der Anwendungsformen der Wirkstoffe kann in weiten Bereichen variieren, beispielsweise von 0,0001 bis zu 100 Gew.-% Wirkstoff, vorzugsweise von 0,001 bis 99 Gew.-% Wirkstoff.

Die agrochemischen Zubereitungen (Formulierungen) enthalten in der Regel 0,1 bis 99 Gewichtsprozent, insbesondere 0,1 bis 95 Gew.-%, Herbizid-Wirkstoff und 1 bis 99,9 Gew.-%, vorzugsweise 5 bis 99,9 Gew.-% unter den Lager- und Anwendungsbedingungen inerte Formulierungshilfsmittel.

Die Anwendung geschieht in einer den Anwendungsformen angepaßten üblichen Weise.

Beispielsweise werden die in handelsüblicher Form vorliegenden Formulierungen zur Anwendung gegebenenfalls in üblicher Weise verdünnt, z.B. bei Spritzpulvem, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der erfindungsgemäßen Verbindungen der Formel (I). Sie kann innerhalb weiter Grenzen variiert werden, z.B. zwischen 0,001 und 10,0 kg/ha oder mehr Aktivsubstanz, vorzugsweise liegt sie jedoch zwischen 0,005 und 5 kg/ha.

### A. Chemische Beispiele

### Beispiel 1: N-tert.Butyl-(2-iodo-3-methoxycarbonyl)benzolsulfonamid

Zu 59.3 g 2-lodo-3-methoxycarbonylbenzolsulfochlorid in 300 ml Dichlormethan tropft man bei Raumtemp. eine Lösung aus 24.1 g *tert*.-Butylamin in 30 ml Dichlormethan. Man rührt 3 h bei Raumtemp. nach, wäscht mit 2 N Salzsäure, trocknet über Na₂SO₄ und evaporiert das Solvens. Der Rückstand wird in Ether digeriert. Man erhält so 30.0 g N-*tert*. Butyl-(2-iodo-3-methoxycarbonyl)benzolsulfonamid als farblose Kristalle vom Schmp. 148-9°C.

### Beispiel 2: 2-Iodo-3-methoxycarbonylbenzolsulfonamid

27.9 g N-*tert*.Butyl-(2-iodo-3-methoxycarbonyl)benzolsulfonamid werden 4 h bei Raumtemp. mit 100 ml Trifluoressigsäure gerührt, man erhitzt 2 h zum Sieden und dampft dann die organische Phase i.Vak. ein. Der Rückstand wird in Dichlormethan/Wasser aufgenommen und bis zur Neutralreaktion mit Natriumcarbonat versetzt. Die Phasen werden getrennt und die wässrige Phase noch zweimal mit Dichlormethan extrahiert. Die vereinigten organischen Phasen werden über Na₂SO₄ getrocknet und das Solvens eingedampft. Nach Verrühren des Rückstands mit Ether erhält man 17.4 g 2-Iodo-3-methoxycarbonylbenzolsulfonamid vom Schmp. 155-7°C.

### Beispiel 3: Methyl 2-amino-4-iodobenzoat

Eine Lösung aus 16.1 g 2-Acetylamino-4-iodobenzoesäure (Schmp. 233-5°C; dargestellt nach US-Patent US 4,762,838) in 325 ml abs. Methanol wird bei 0°C mit trockenem Chlorwasserstoffgas gesättigt. Man erhitzt 15 h zum Sieden, kühlt auf Raumtemp., sättigt erneut mit trockenem Chlorwasserstoffgas und läßt 24 h bei Raumtemp. stehen. Man dampft das Solvens i.Vak. ein, nimmt den Rückstand in Dichlormethan auf und wäscht die organische Phase mit einer gesättigten wässrigen Natriumhydrogencarbonat-Lösung säurefrei. Die organische Phase wird über Na₂SO₄ getrocknet und i.Vak. eingedampft. Man erhält so 13.8 g Methyl 2-amino-4-iodobenzoat vom Schmp. 63-7°C.

### Beispiel 4: Bis-(2-methoxycarbonyl-5-iodobenzol)disulfid

13.8 g Methyl 2-amino-4-iodobenzoat werden mit 48 ml Eisessig und anschließend mit 86 ml konz. Salzsäure versetzt. Zu dieser auf -5°C gekühlten Suspension tropft man eine Lösung aus 3.8 g Natriumnitrit in 15 ml Wasser langsam zu und rührt 30 min bei dieser Temp. nach. Diese gekühlte Diazoniumsalz-Lösung wird bei 0°C zu einer Lösung aus 20 ml Schwefeldioxid, 60 ml Eisessig, 10 ml Wasser und 3.1 g Kupfer(II)-chlorid Dihydrat getropft und zunächst 1 h bei 0°C, dann über Nacht bei Raumtemp. nachgerührt. Das Reaktionsgemisch wird auf 1 Eiswasser gegossen und das Produkt abgesaugt Man erhält so 12.7 g Bis-(2-methoxycarbonyl-5-iodo-benzol)disulfid vom Schmp. 133-5°C.

### Beispiel 5: 2-Methoxycarbonyl-5-iodobenzolsulfochlorid

Zu 12.2 g Bis-(2-methoxycarbonyl-5-iodobenzol)disulfid in einer Lösung aus 30 ml 1,2-Dichlorethan und 15 ml 2 N Salzsäure wird bei 20-25°C Chlorgas eingeleitet bis zum Ende der exothermen Reaktion. Man saugt ab, extrahiert die wässrige Phase mit Dichlormethan, trocknet die vereinigten organischen Phasen über Na₂SO₄ und dampft das Solvens i.Vak. ab. Man erhält so aus abgesaugtem und extrahierten Produkt eine Gesamtmenge von 15.0 g 2-Methoxycarbonyl-5-iodobenzolsulfochlorid vom Schmp. 119-120°C (Zers.).

### Beispiel 6: 2-Methoxycarbonyl-5-iodobenzolsulfonamid

Zu 15.0 g 2-Methoxycarbonyl-5-iodobenzolsulfochlorid in 100 ml Tetrahydrofuran leitet man so lange bei Raumtemp. Ammoniakgas ein, bis kein Ammoniak mehr aufgenommen wird. Die Lösung wird i.Vak. eingedampft, der Rückstand mit Wasser gut verrührt und das Produkt abgesaugt Nach Trocknung des Filterrückstandes bei 70°C i.Vak. erhält man 10.7 g 2-Methoxycarbonyl-5-iodobenzolsulfonamid als weißes Pulver vom Schmp. 176-7°C.

### Beispiel 7:

### 3-Ethoxycarbonyl-2-iodobenzolsulfochlorid

24.0 g Ethyl 3-amino-2-iodobenzoat werden in 60 ml Eisessig und 120 ml konz. Salzsäure gelöst. Zu dieser auf -5°C gekühlten Suspension tropft man eine Lösung aus 6.9 g Natriumnitrit in 30 ml Wasser langsam zu und rührt 30 min bei dieser Temp. nach. Diese gekühlte Diazoniumsalz-Lösung wird bei 5-10°C zu einer mit Schwefeldioxid bei ca. 10°C gesättigten Lösung aus 70 ml Eisessig, 70 ml konz. Salzsäure und 3.0 g Kupfer(II)-chlorid Dihydrat getropft. Man rührt 3 h bei Raumtemp. und leitet dann Chlorgas ein bis die exotherme Reaktion abklingL Das Reaktionsgemisch wird auf 1 l Eiswasser gegossen, das Produkt abgesaugt und bei 50°C i.Vak. getrocknet Man erhält so 25.3 g 3-Ethoxycarbonyl-2-iodobenzolsulfochlorid vom Schmp. 80-3°C.

### Beispiel 8: 3-Ethoxycarbonyl-2-iodobenzolsulfonamid

Analog Beispiel 6 erhielt man aus 25.3 g 3-Ethoxycarbonyl-2-iodobenzolsulfochlorid und Ammoniak 20.4 g 3-Ethoxycarbonyl-2-iodobenzolsulfonamid vom Schmp. 138-9°C.

### Beispiel 9: 2-[[[(4,6-Dimethoxy-2-pyrimidinyl)-amino]-carbonyl]-amino]sulfonyl]4-iodobenzoesäuremethylester

Zu einer Mischung aus 3.4 g 5-Iodo-2-methoxycarbonylbenzolsulfonamid und 2.8 g O-Phenyl (4,6-dimethoxy-2-pyrimidinyl)carbamat in 50 ml abs. Acetonitril tropft man bei Raumtemp. eine Lösung von 1.7 g 1,8-Diazabicyclo[5.4.0]undec-7-en in 10 ml abs. Acetonitril zu. Man rührt 3 h bei dieser Temp., engt auf ca. 1/3 ein und gießt auf 200 ml Eiswasser. Die wässrige Phase wird mit Diethylether extrahiert, mit konz. Salzsäure auf pH 1-2 angesäuert und das Produkt abgesaugt. Nach Trocknen bei 60°C i.Vak. erhält man 3.3 g 2-[[[(4,6-Dimethoxy-2-pyrimidinyl)-amino]carbonyl]-amino]-sulfonyl]-4-iodo-benzoesäure-methylester vom Schmp. 169-71°C.

### Beispiel 10: 2-Iodo-3-[[[[(4-methoxy-6-methyl,1,3,5-triazin-2-yl)-amino]-carbonyl]-amino]-sulfonyl]-benzoesäure-ethylester

Unter Stickstoff-Schutzgas tropft man zu einer Suspension von 3.6 g 3-Ethoxycarbonyl-2-iodobenzolsulfonamid in 100 ml abs. Dichlormethan 14 mmol Trimethylaluminium ( 7 ml einer 2 M Lösung in Hexan) zu. Nach 30 min Rühren bei Raumtemp. gibt man 2.2 g O-Methyl (4-methyl-6-methoxy-1,3,5-triazin-2-yl)-carbamat in 25 ml Dichlormethan zu und erhitzt 13 h unter Rückfluß. Zur auf Raumtemp. gekühlten Lösung wird unter Eiskühlung 25 ml 2 N Salzsäure zugetropft und die salzsaure Phase zweimal mit Dichlormethan extrahiert. Die org. Phase wird i.Vak. eingeengt und der Rückstand mit Aceton und 100 ml 10%-ige aqu. Natriumacetat-Lösung versetzt. Nach 3 h Rühren wird abgesaugt, mit Diethylether gewaschen, die wässrige Phase mit konz. Salzsäure auf pH 2-3 gestellt und das Produkt nach 15 min Rühren abgesaugt. Nach Trocknen i.Vak. bei 50°C erhält man 1.7 g 2-Iodo-3-[[[[(4-methoxy-6-methyl-1,3,5-triazin-2-yl)-amino]-carbonyl]-amino]-sulfonyl]-benzoesäureethylester vom Schmp. 177-9°C.

### Beispiel 11: 2-Methoxycarbonyl-5-iodobenzolsulfonylisocyanat

50 g des in Beispiel 6 erhalten Sulfonamids werden in 150 ml 1,2-Dichlorethan suspendiert und mit 27,7 ml Thionylchlorid versetzt. Man erhitzt 4 h zum Sieden, kühlt auf 50-55°C ab, versetzt mit 0,5 ml Pyridin und leitet nun in die zum Sieden gebrachte Lösung 3 1/2 Stunden Phosgen ein. Es wird unter Feuchtigkeitsausschluß unter reduziertem Druck eingeengt. Das zurückbleibende rohe Sulfonylisocyanat (52,6 g) kristallisiert beim Stehen.

### Beispiel 12: 2-Iodo-3-methoxycarbonylbenzolsulfonylisocyanat

27,3 g 2-Iodo-3-methoxycarbonylbenzolsulfonamid und 9,0 ml n-Butylisocyanat in 300 ml absolutem Aceton werden beim Raumtemperatur mit 12 ml DBU versetzt und 3 h zum Sieden erhitzt. Man kühlt auf Raumtemperatur ab, engt auf etwa 1/3 des Volumens ein und gießt die Reaktionslösung in 1 1 Wasser. Die Wasserphase wird mit konz. Salzsäure auf pH 1-2 angesäuert und der ausgefallene Niederschlag abgesaugt. Man erhält 31,3 g 2-Iodo-[[[(n-butylamino)-carbonyl]-amino]-sulfonyl]-benzoesäuremethylester vom Schmelzpunktl63-7°C. 29,0 g des so erhaltenen Butylsulfonylharnstoffs werden in 400 ml Chlorbenzol suspendiert und zum Sieden erhitzt. Dann leitet man in der Siedehitze Phosgen ein. Das so entstehende Butylisocyanat wird über eine 20 cm-Vigreux Kolonne während 5 h langsam als Gemisch mit Chlorbenzol abdestilliert. Es wird unter Feuchtigkeitsausschluß i. Vak. eingeengt. Man erhält so 28,4 g 2-Iodo-3-methoxycarbonylbenzolsulfonylisocyanat als Öl.

Die Sulfonamide der Tabellen 1a und 1b werden analog zu den Verfahren der Beispiele 1 bis 8 erhalten.

Die Sulfonylharnstoffe der Tabellen 2-6 werden analog zu den Verfahren der Beispiele 9 und 10 erhalten. In den Tabellen beziehen sich die Abkürzungen auf die der jeweiligen Tabelle vorangestellte allgemeine Formel.

Die Sulfonylisocyanate der Tabellen 1c und 1d werden analog zu den Verfahren der Beispiele 11 und 12 erhalten.

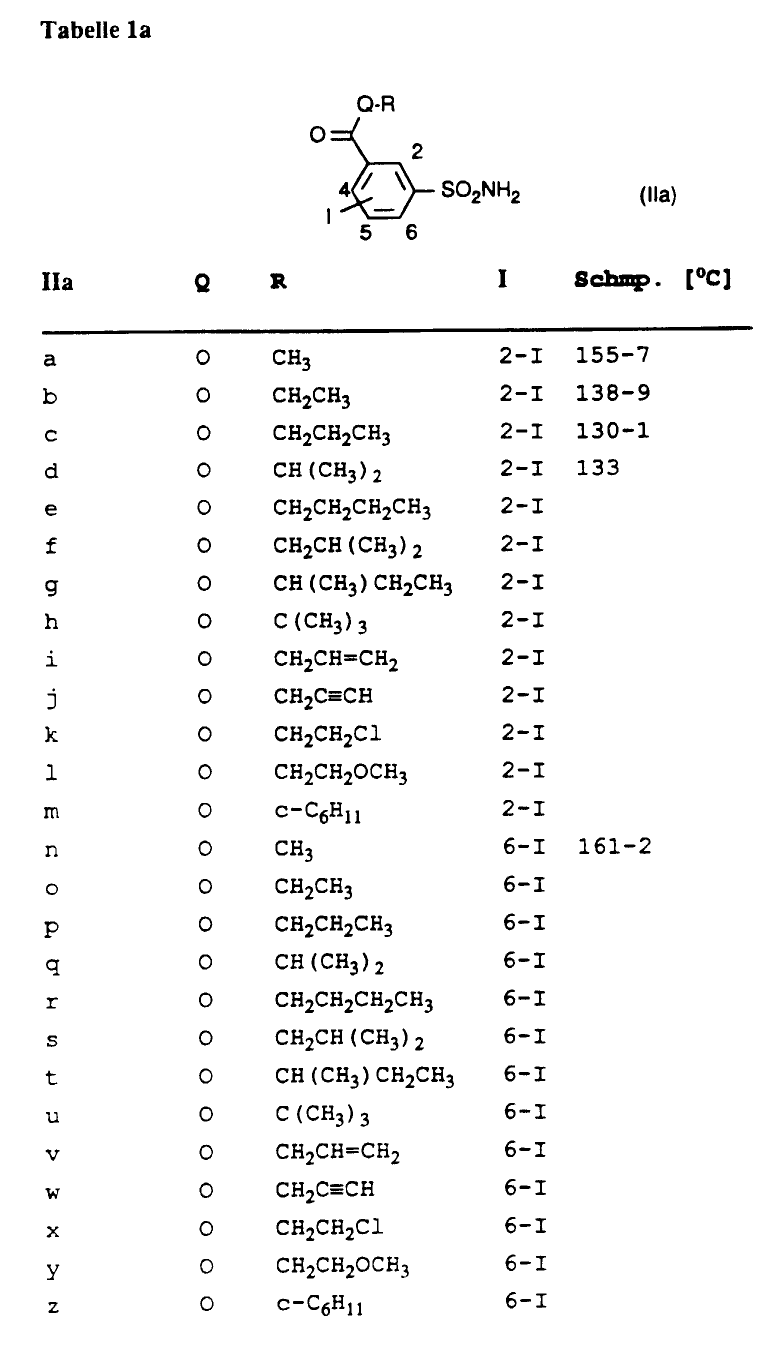

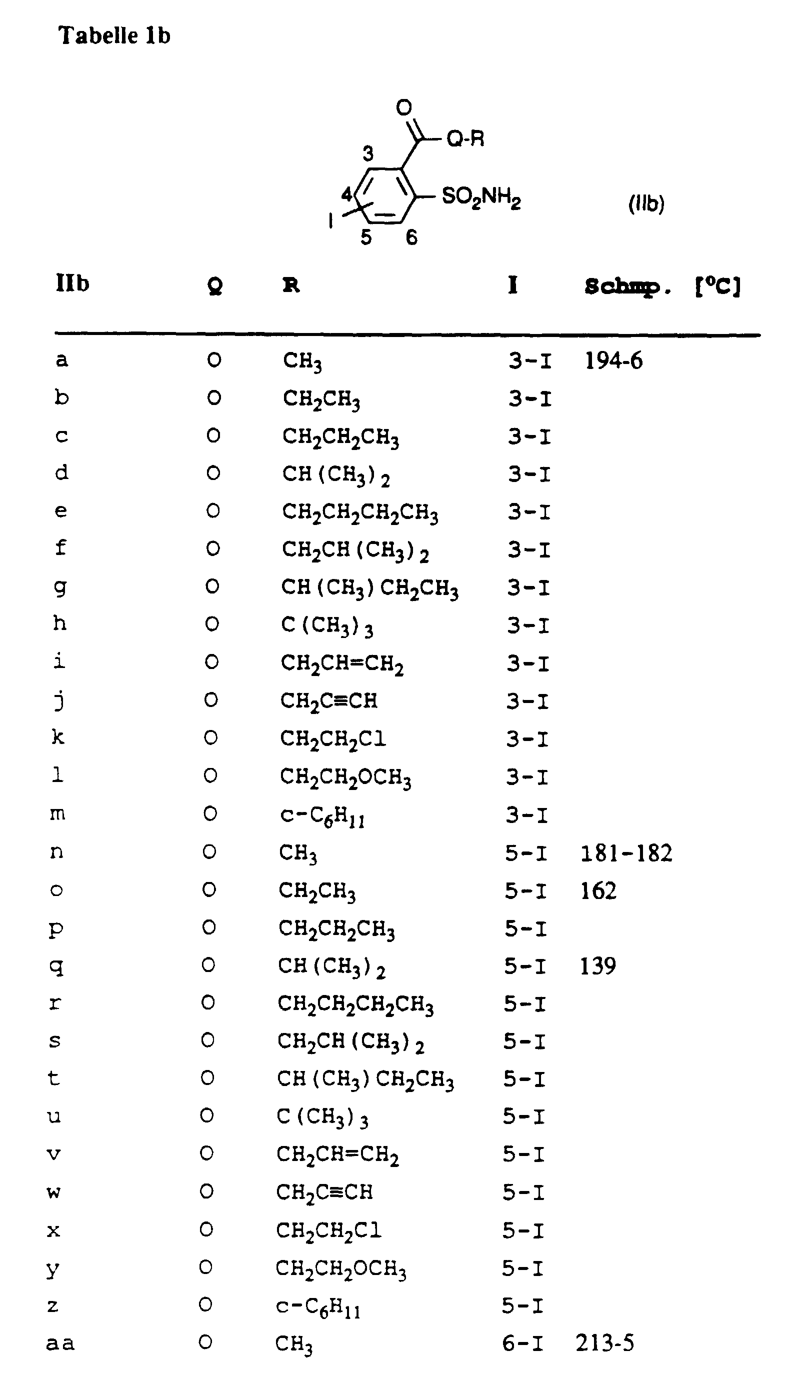

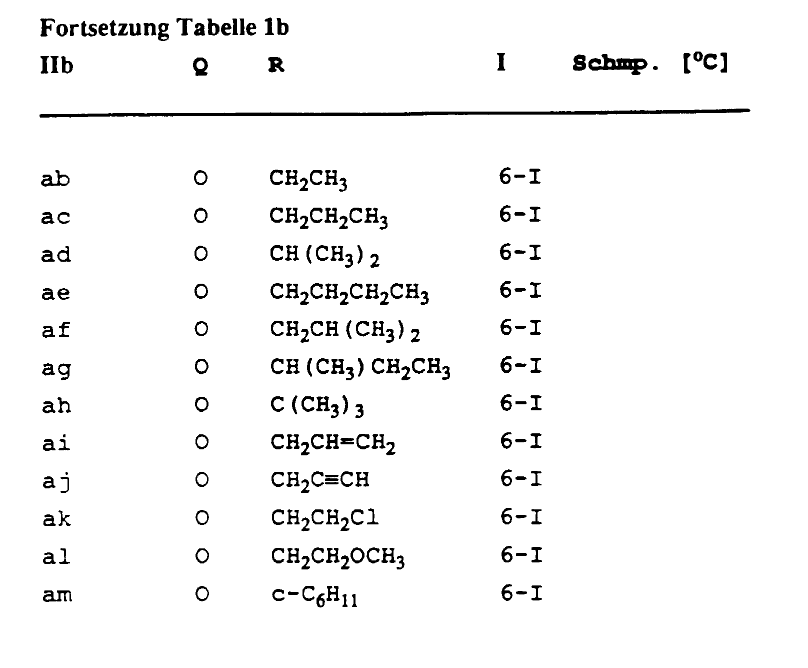

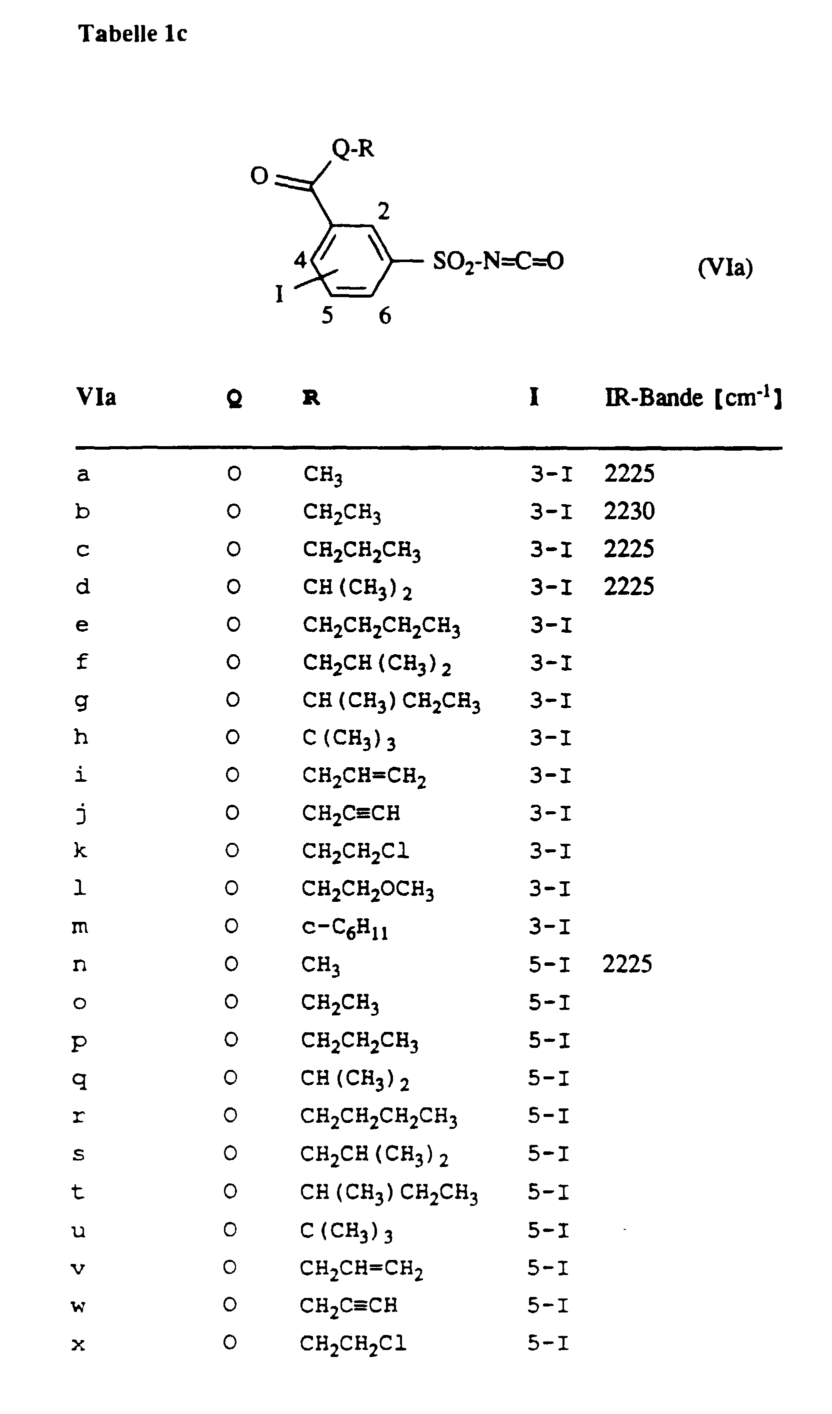

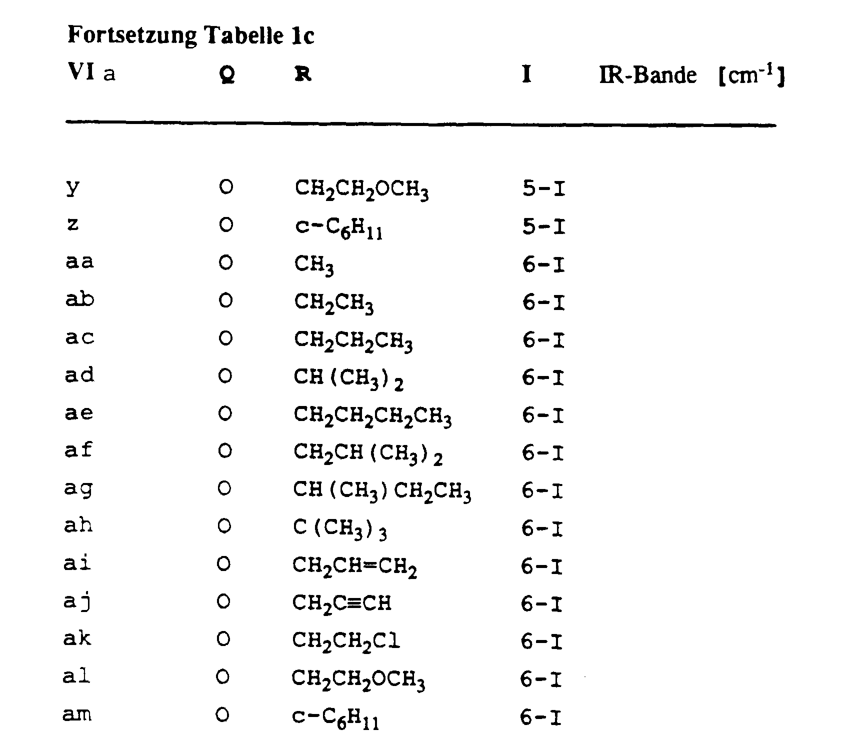

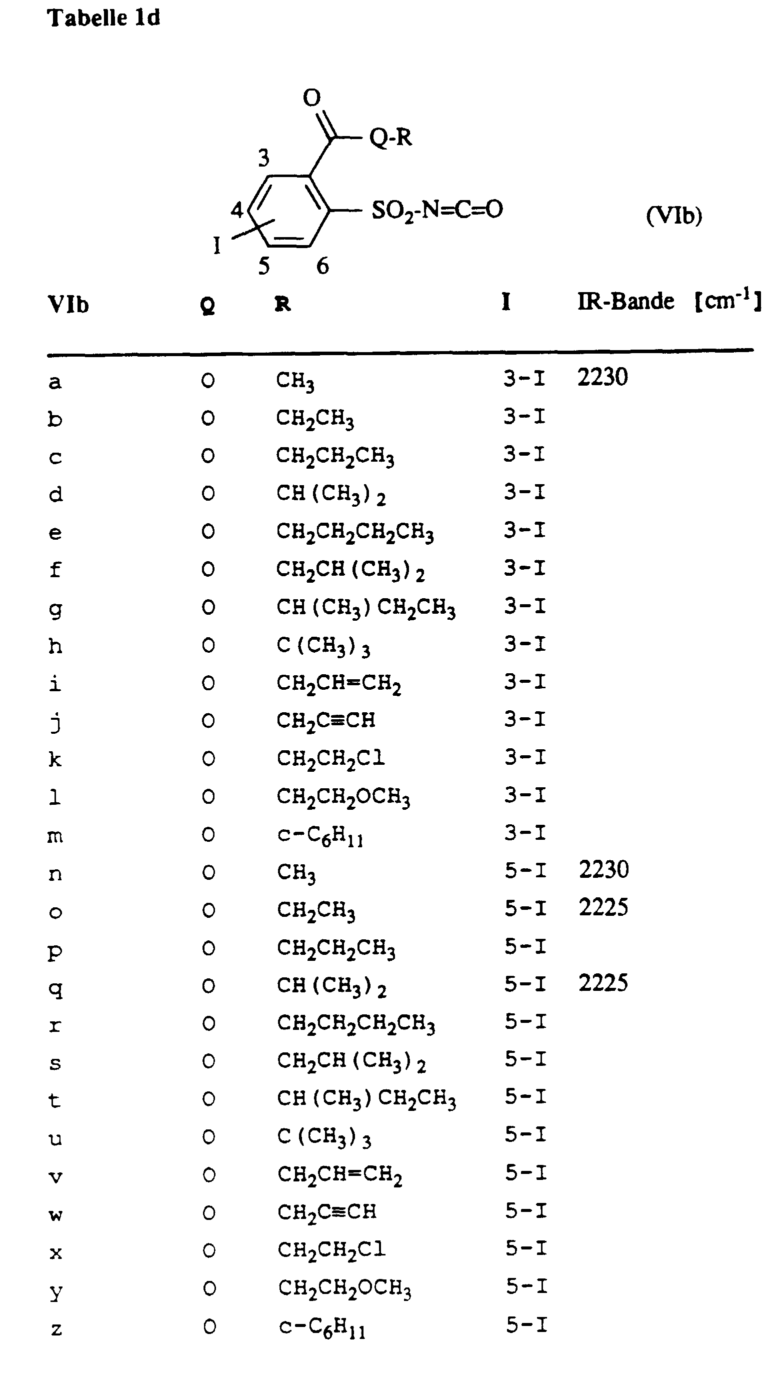

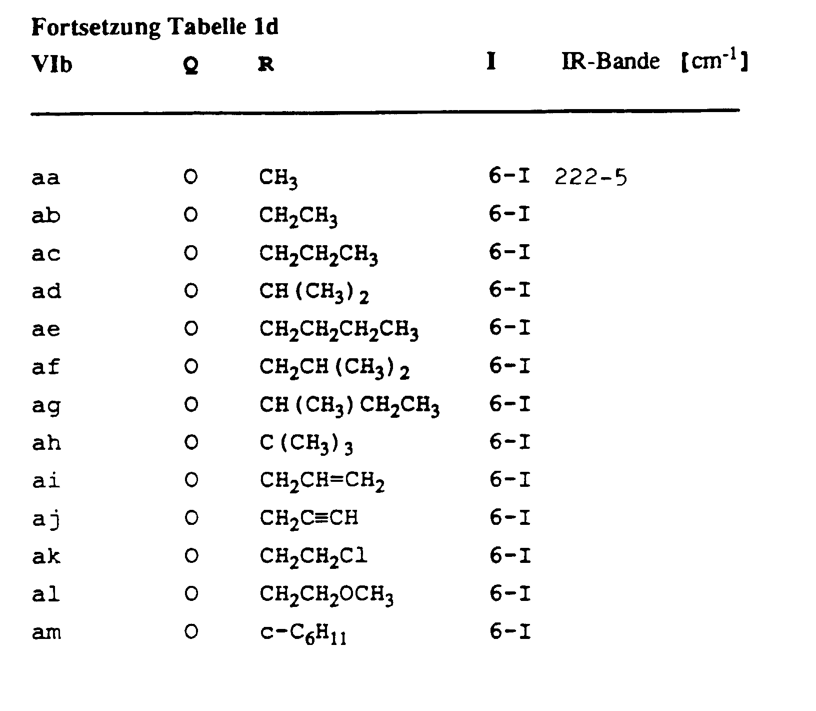

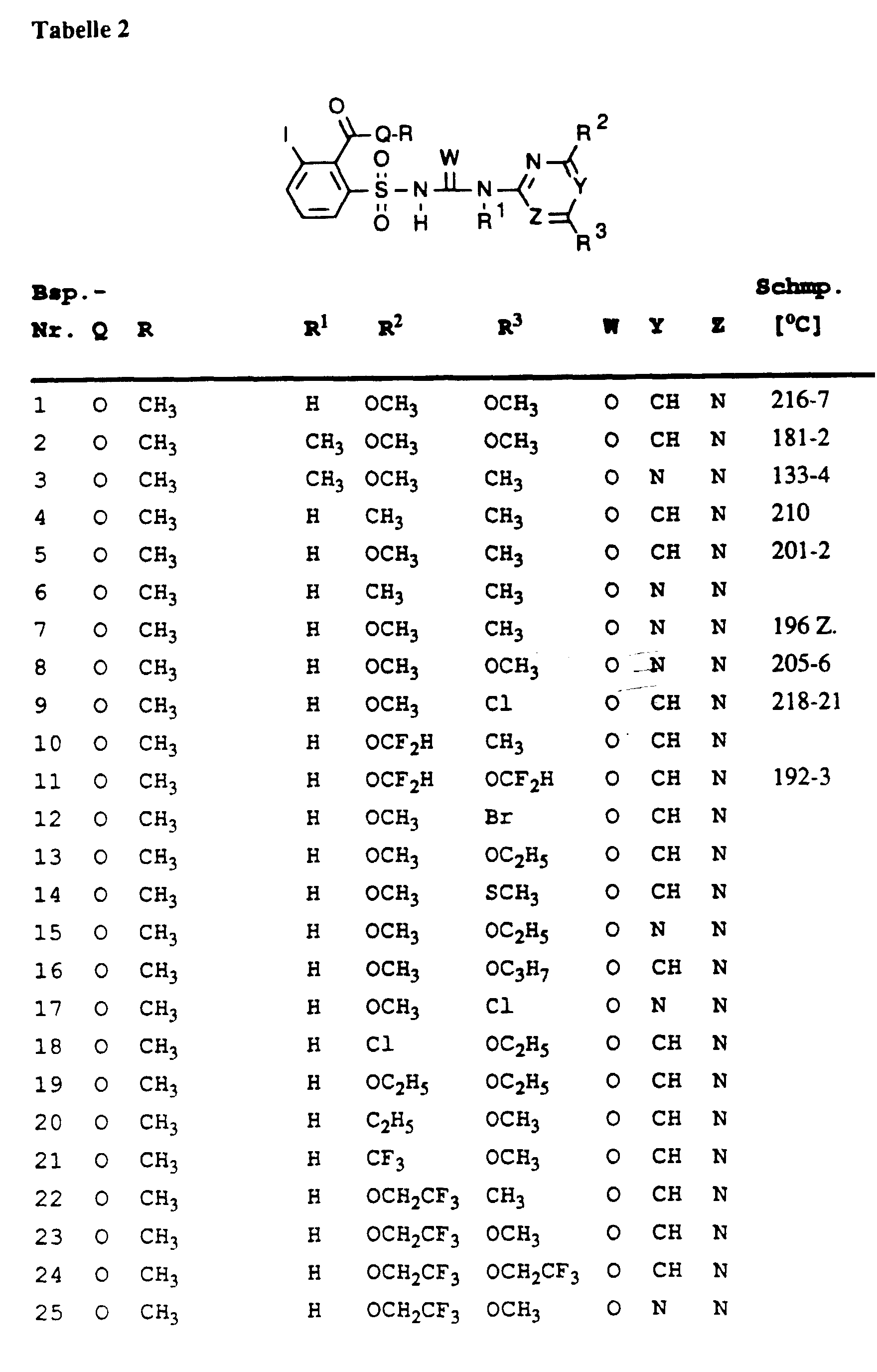

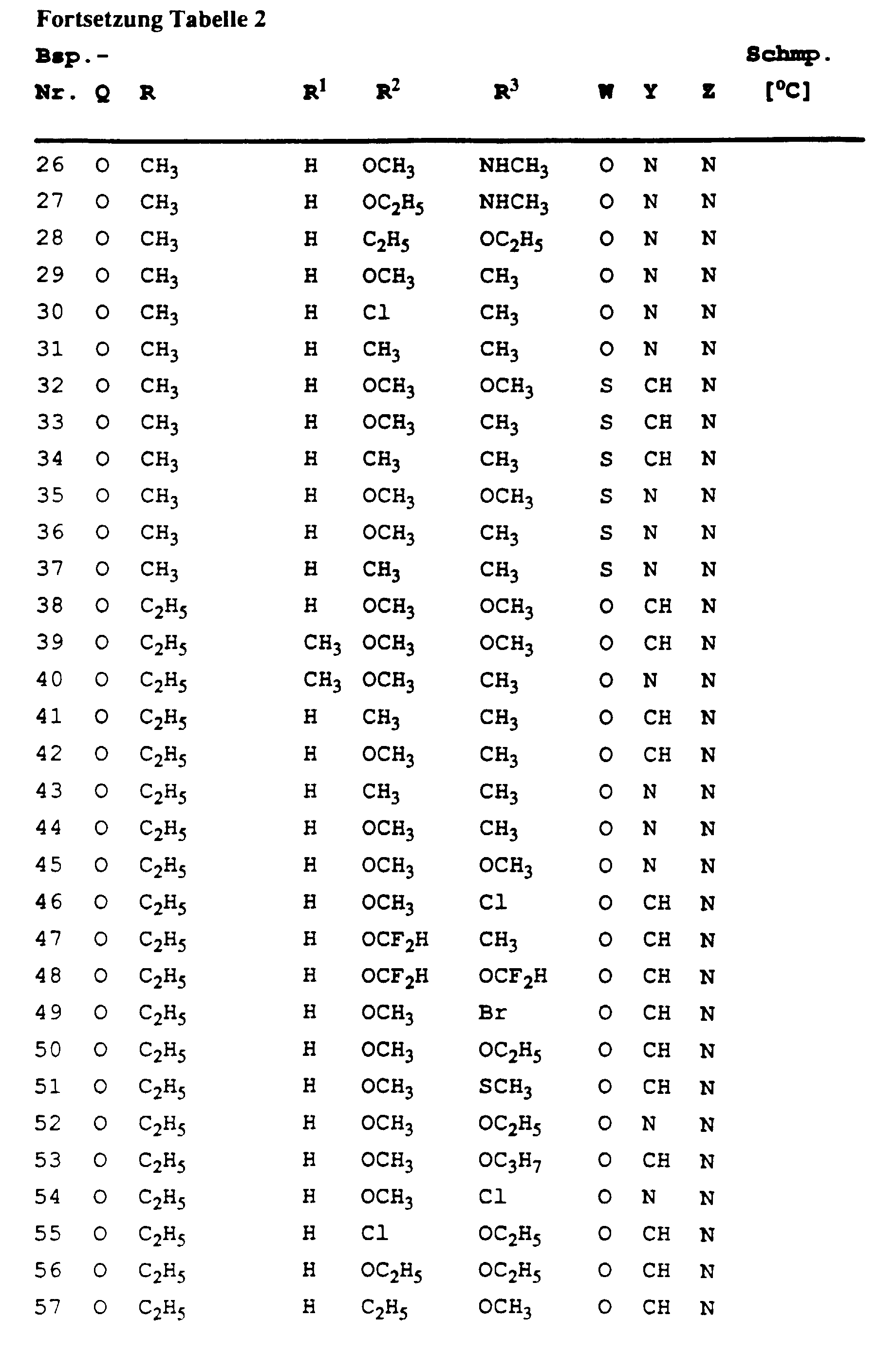

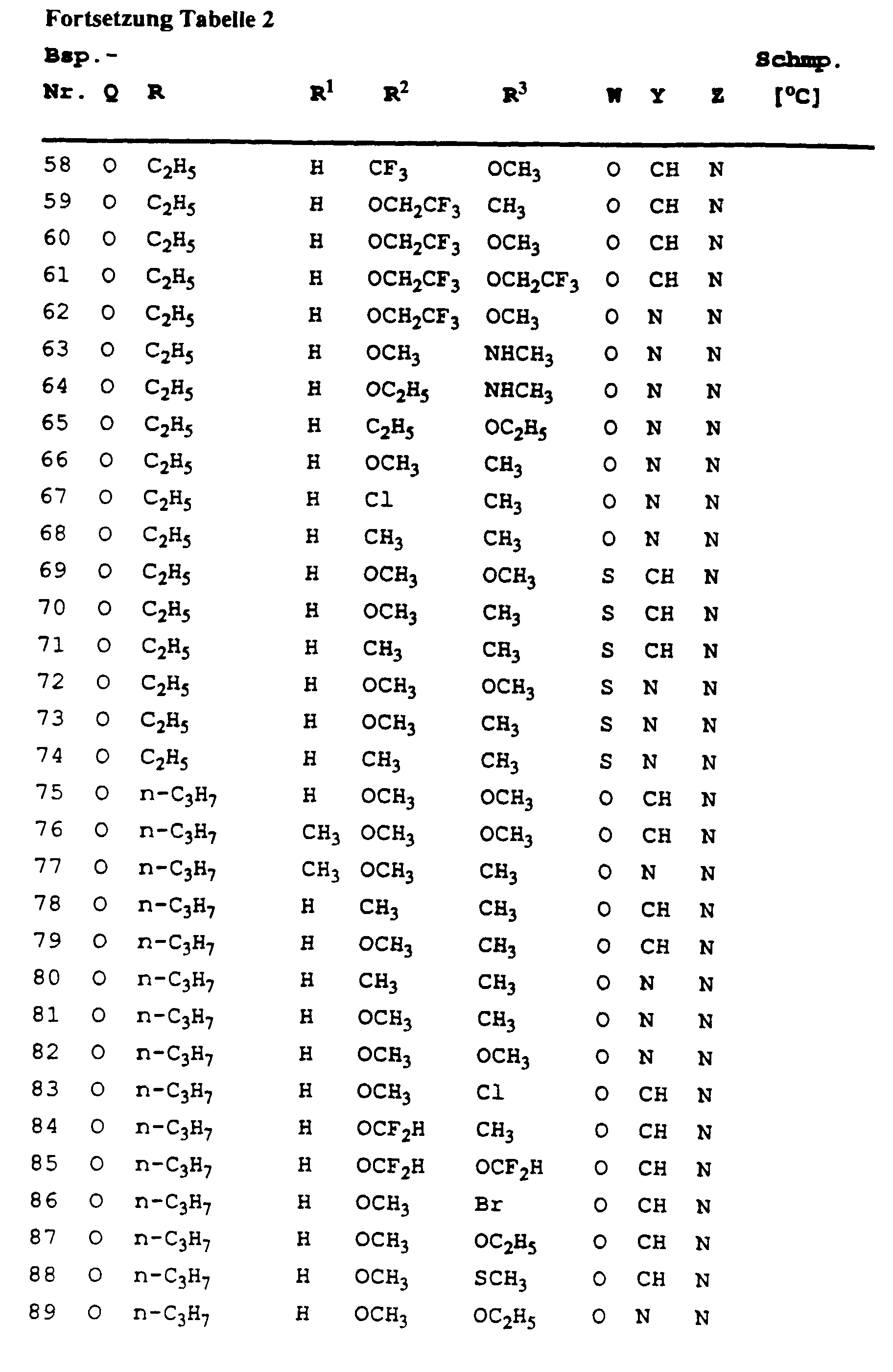

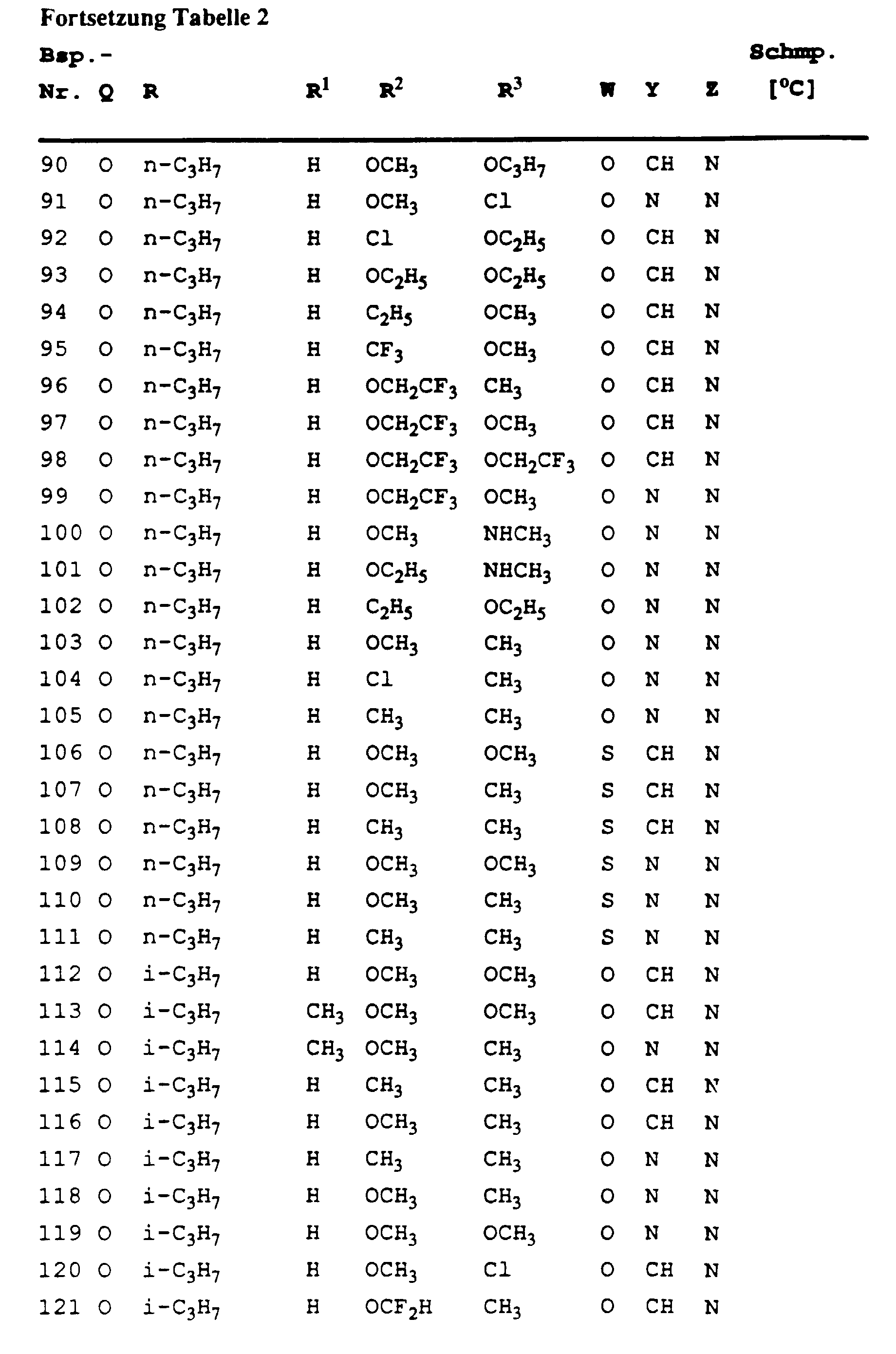

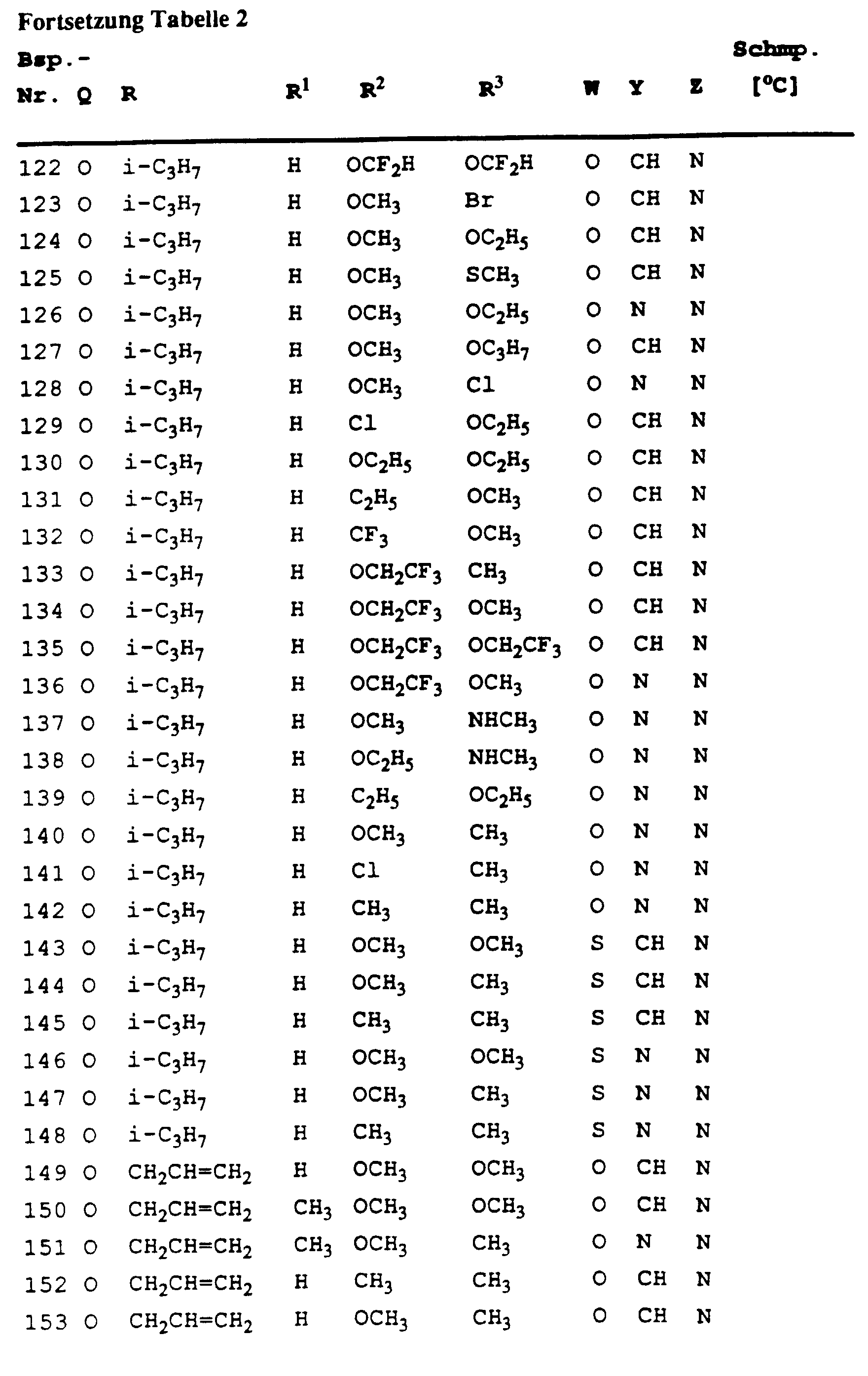

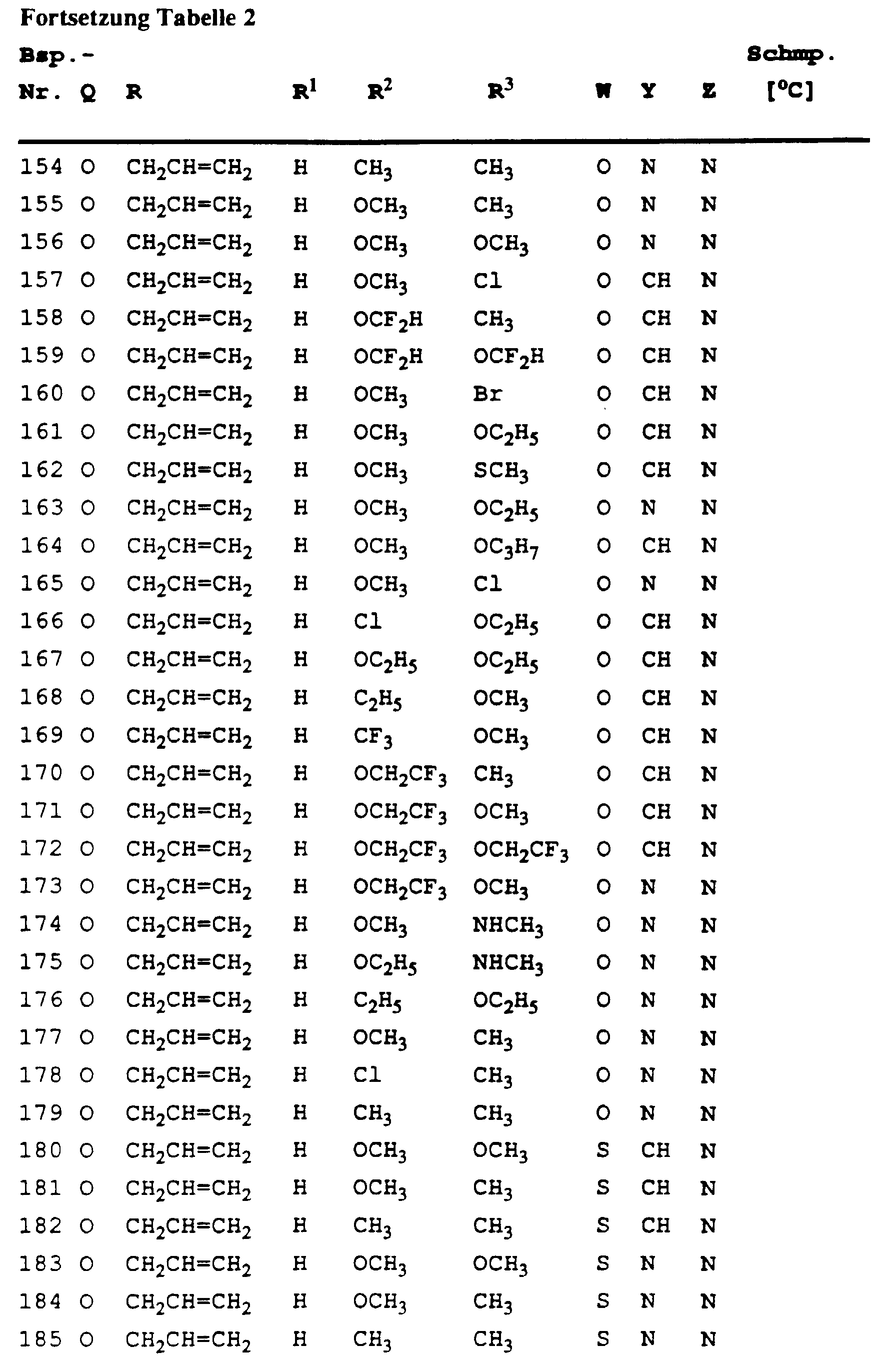

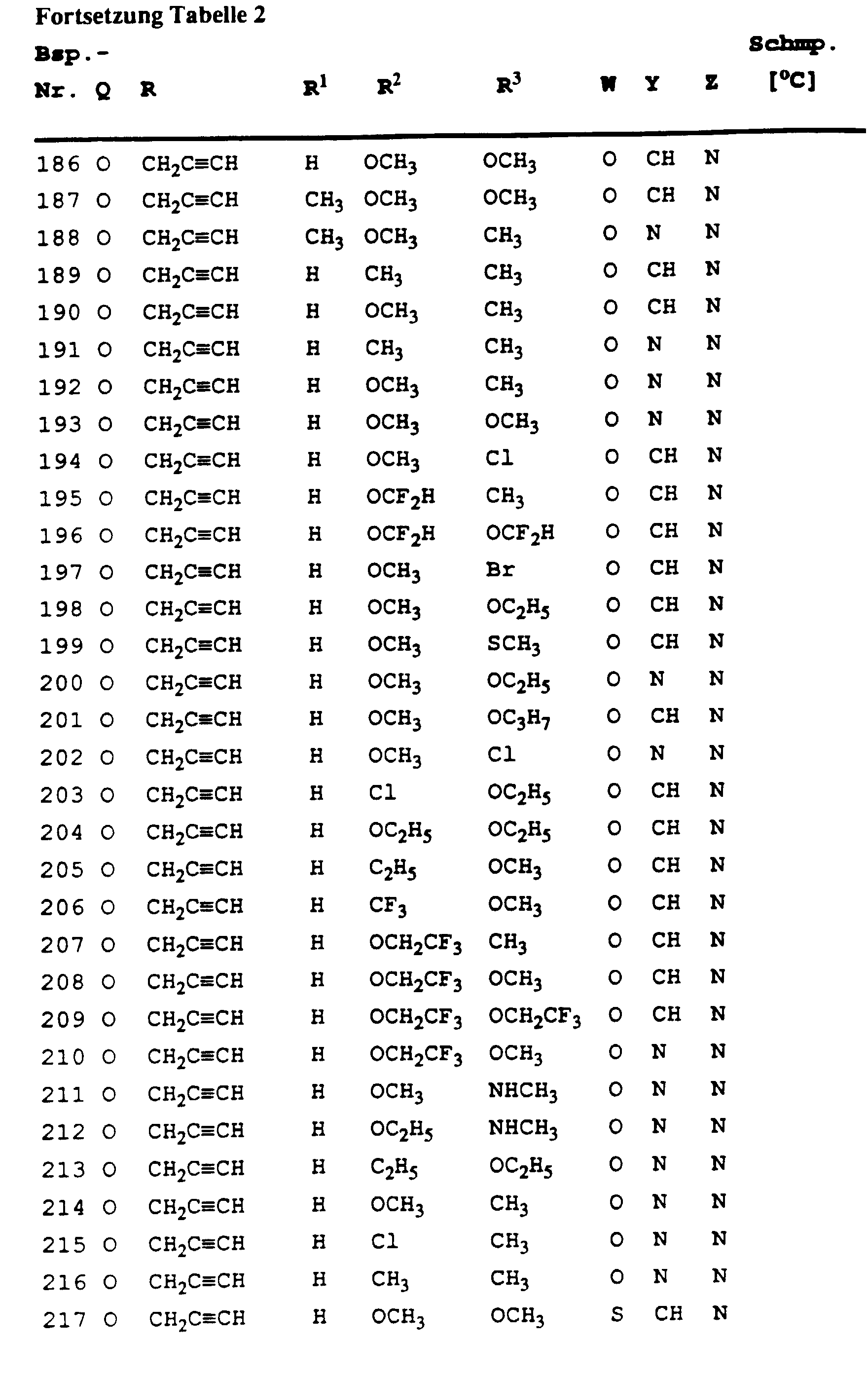

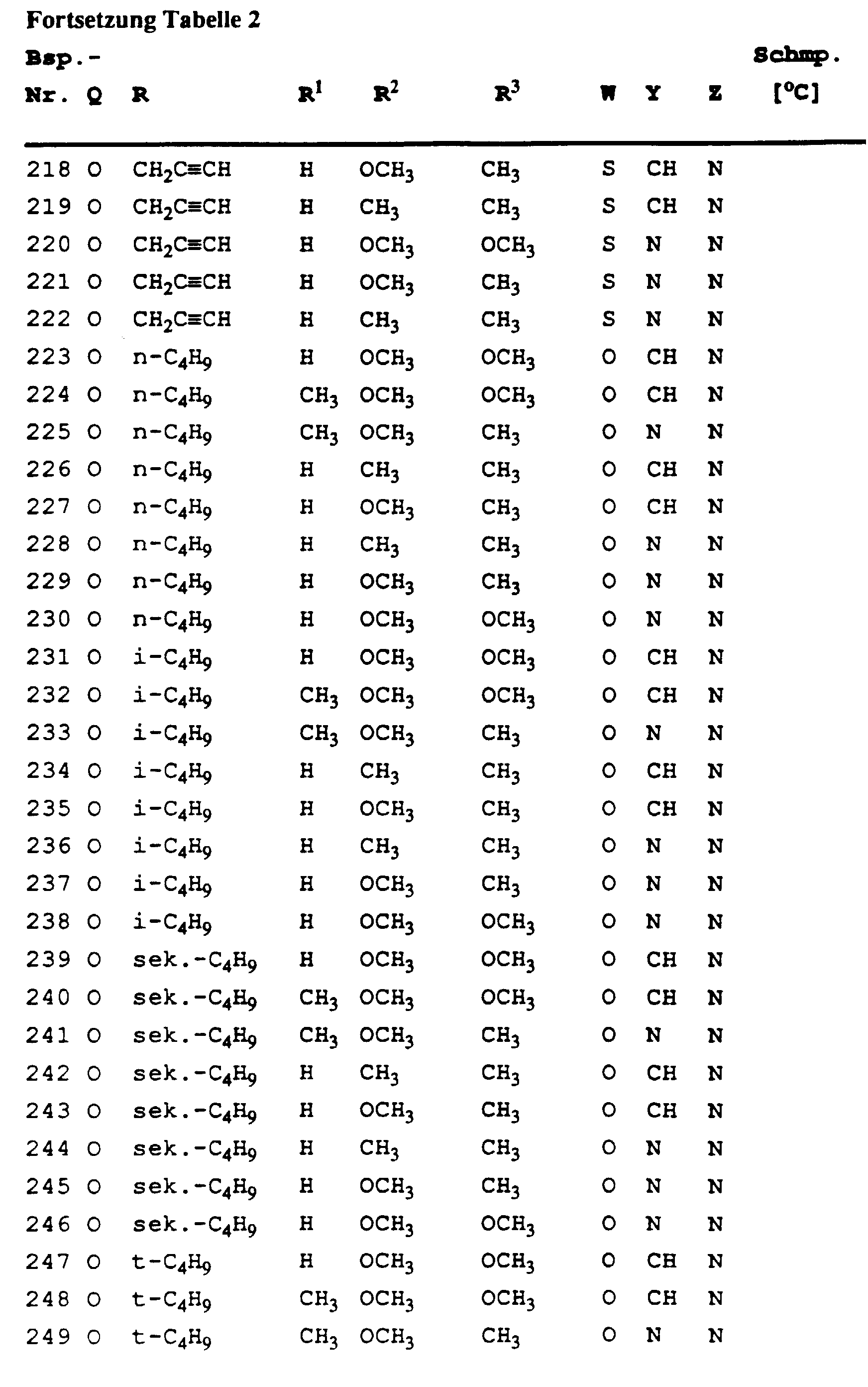

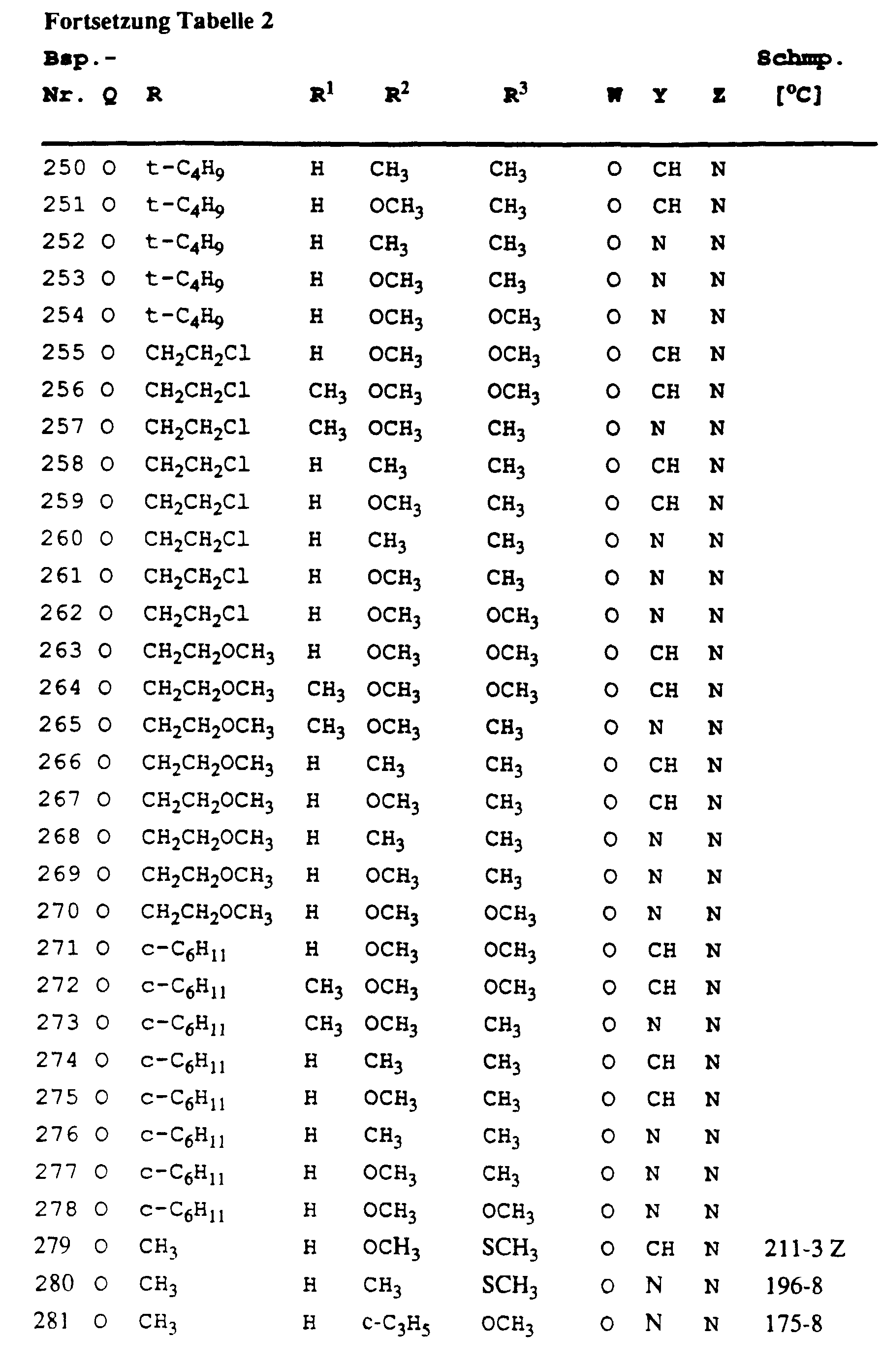

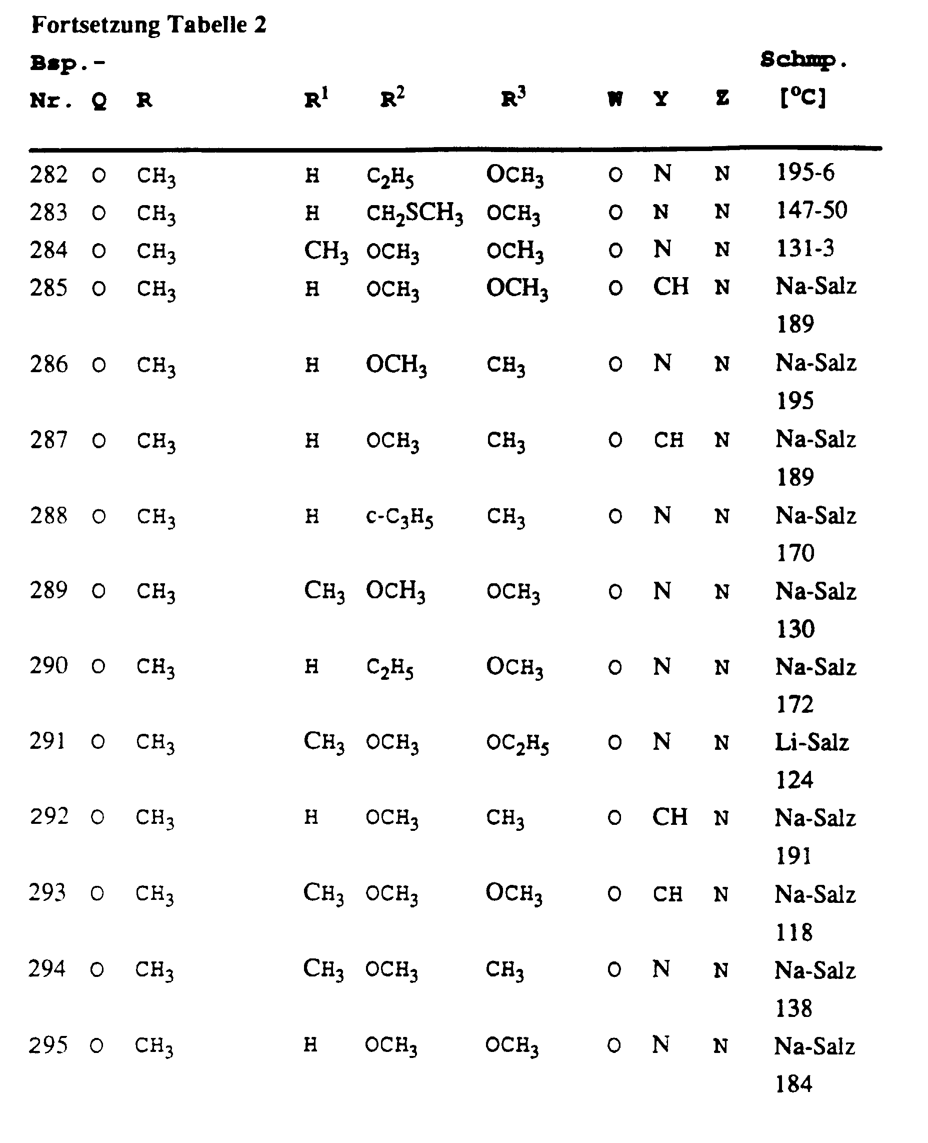

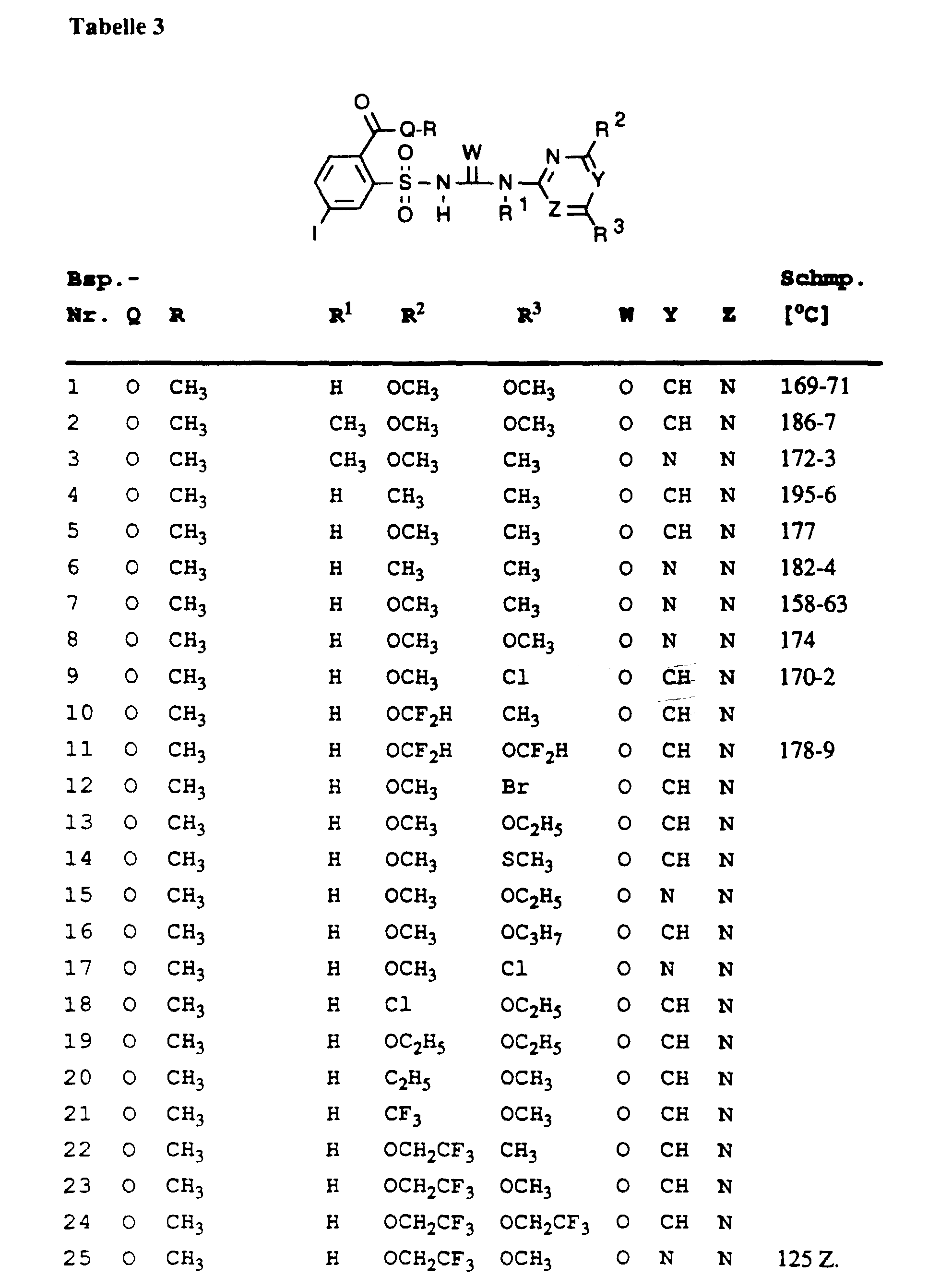

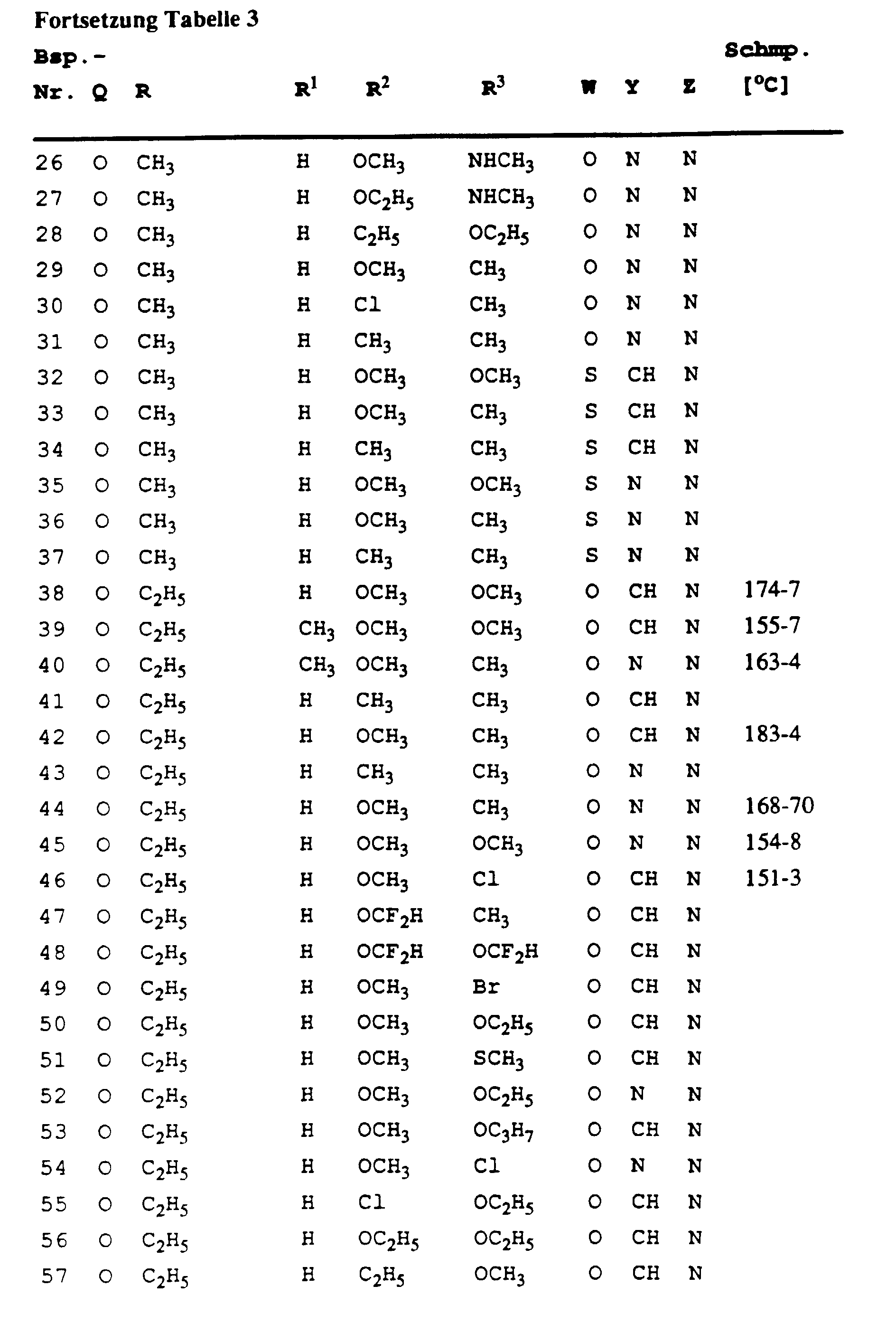

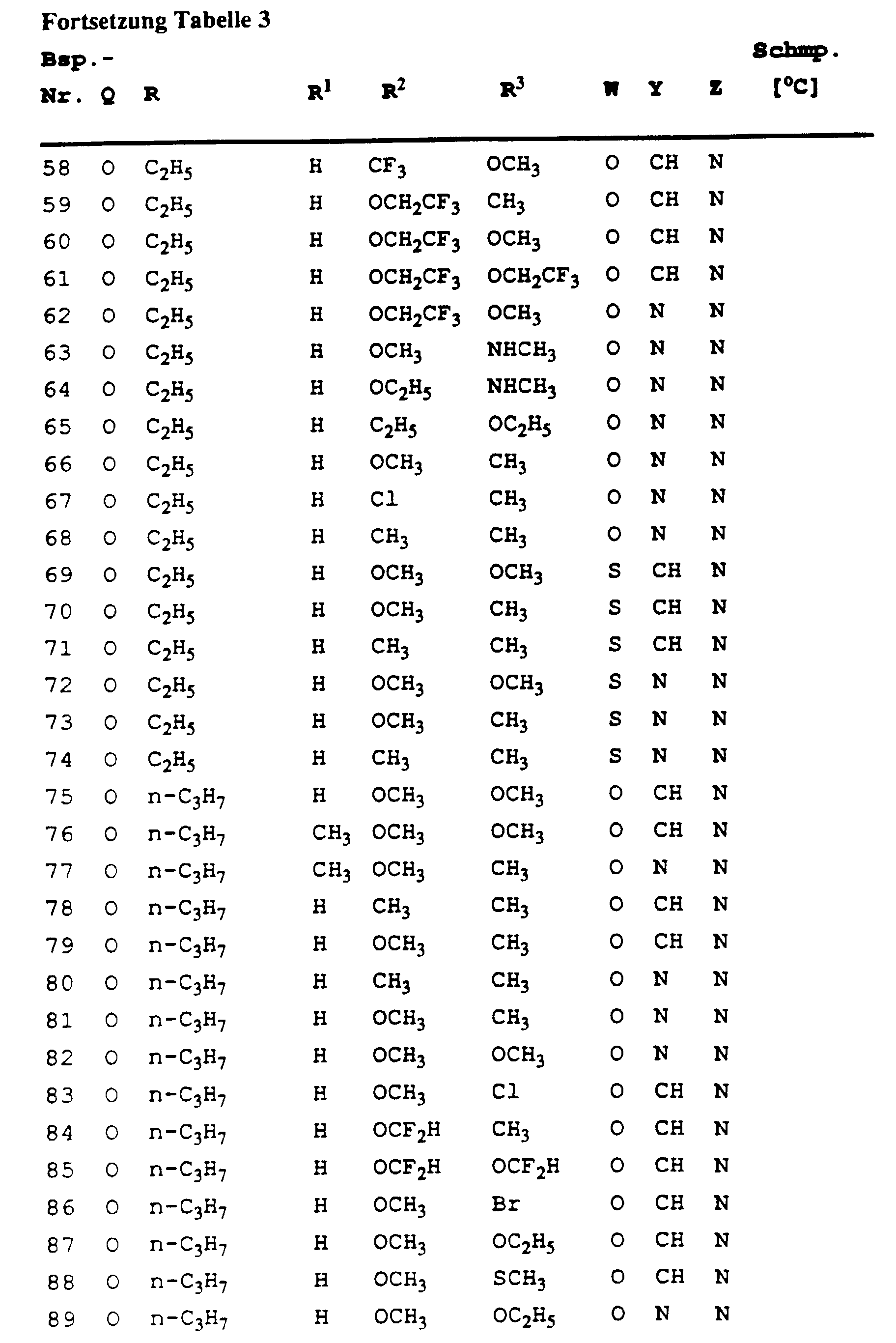

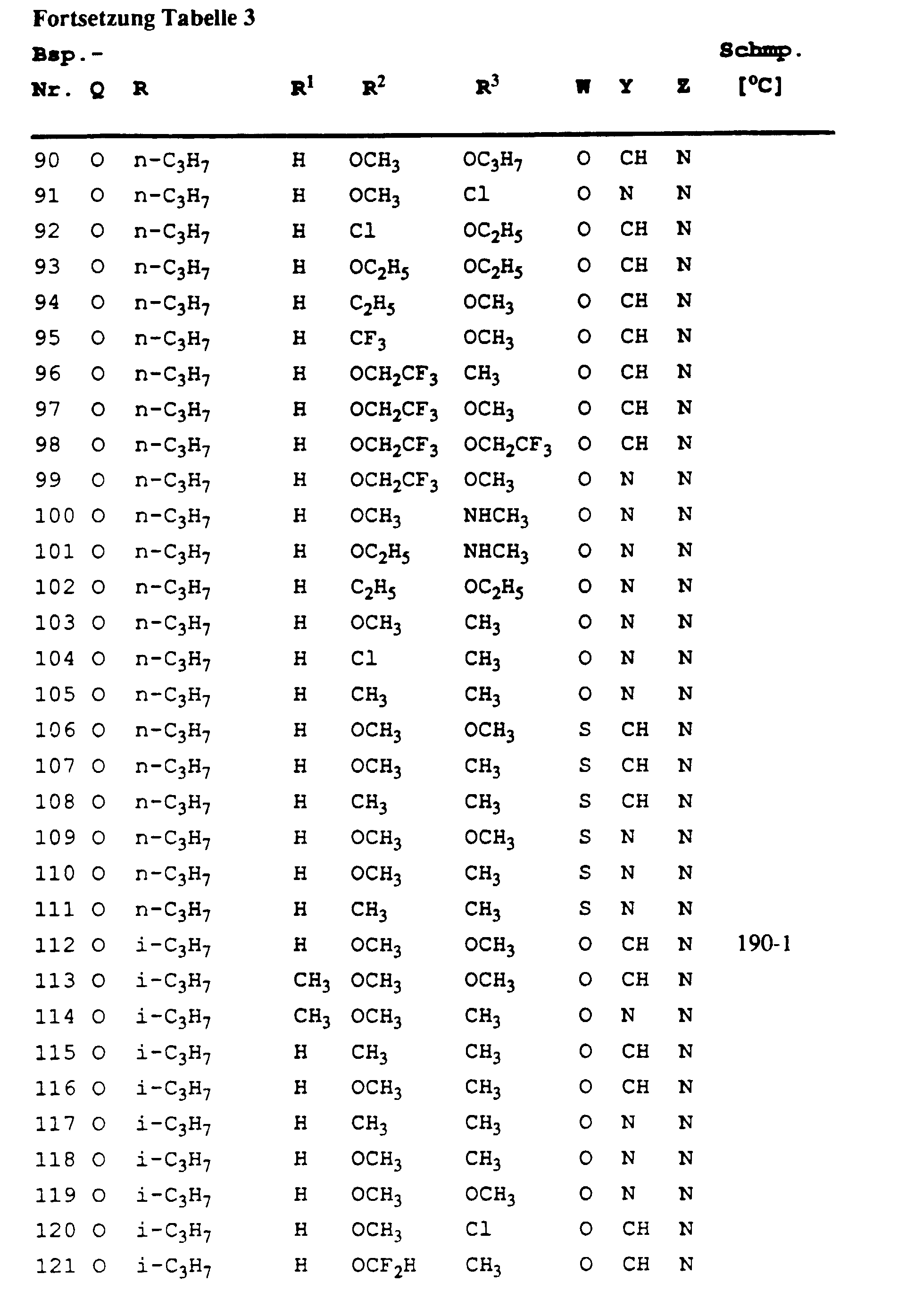

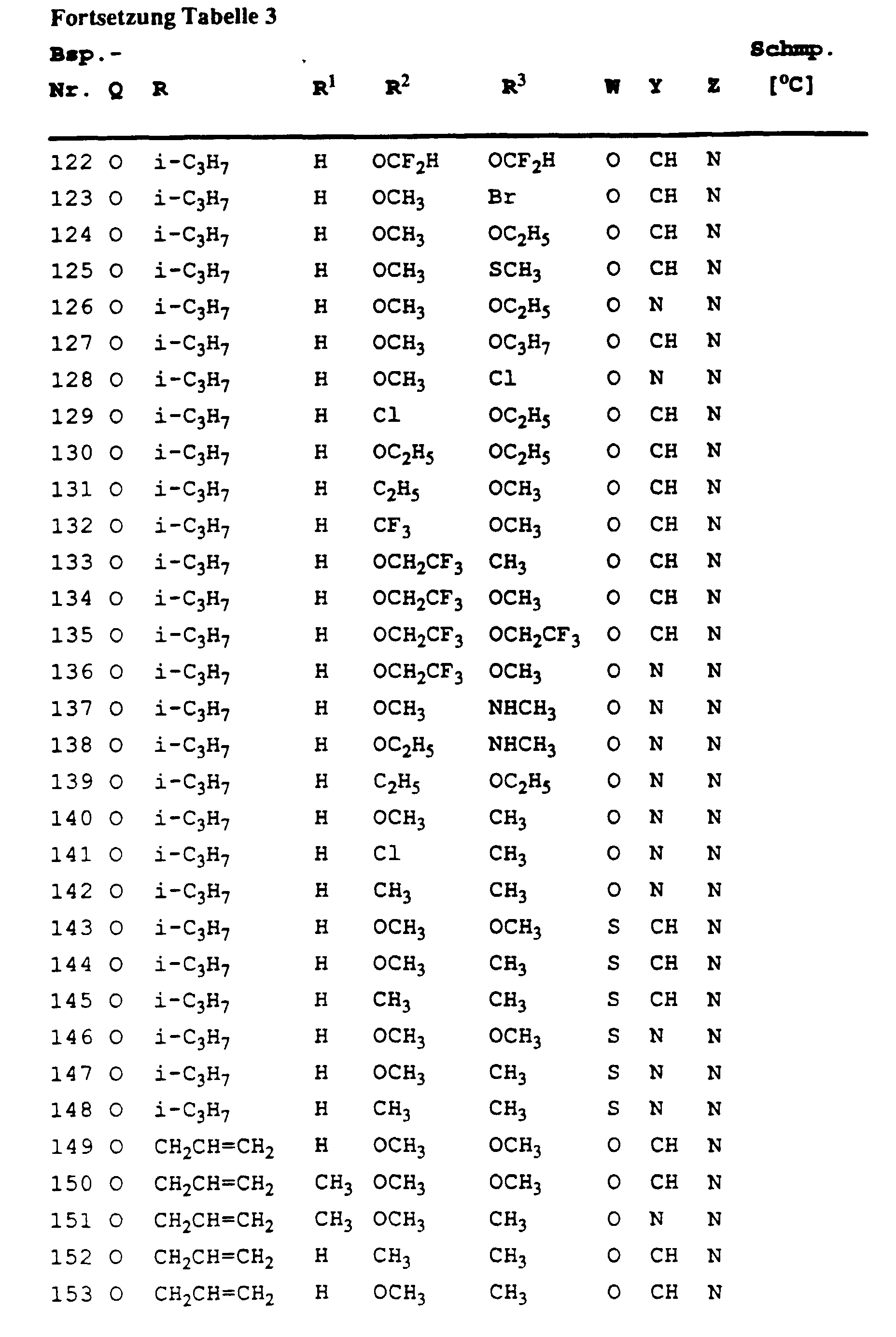

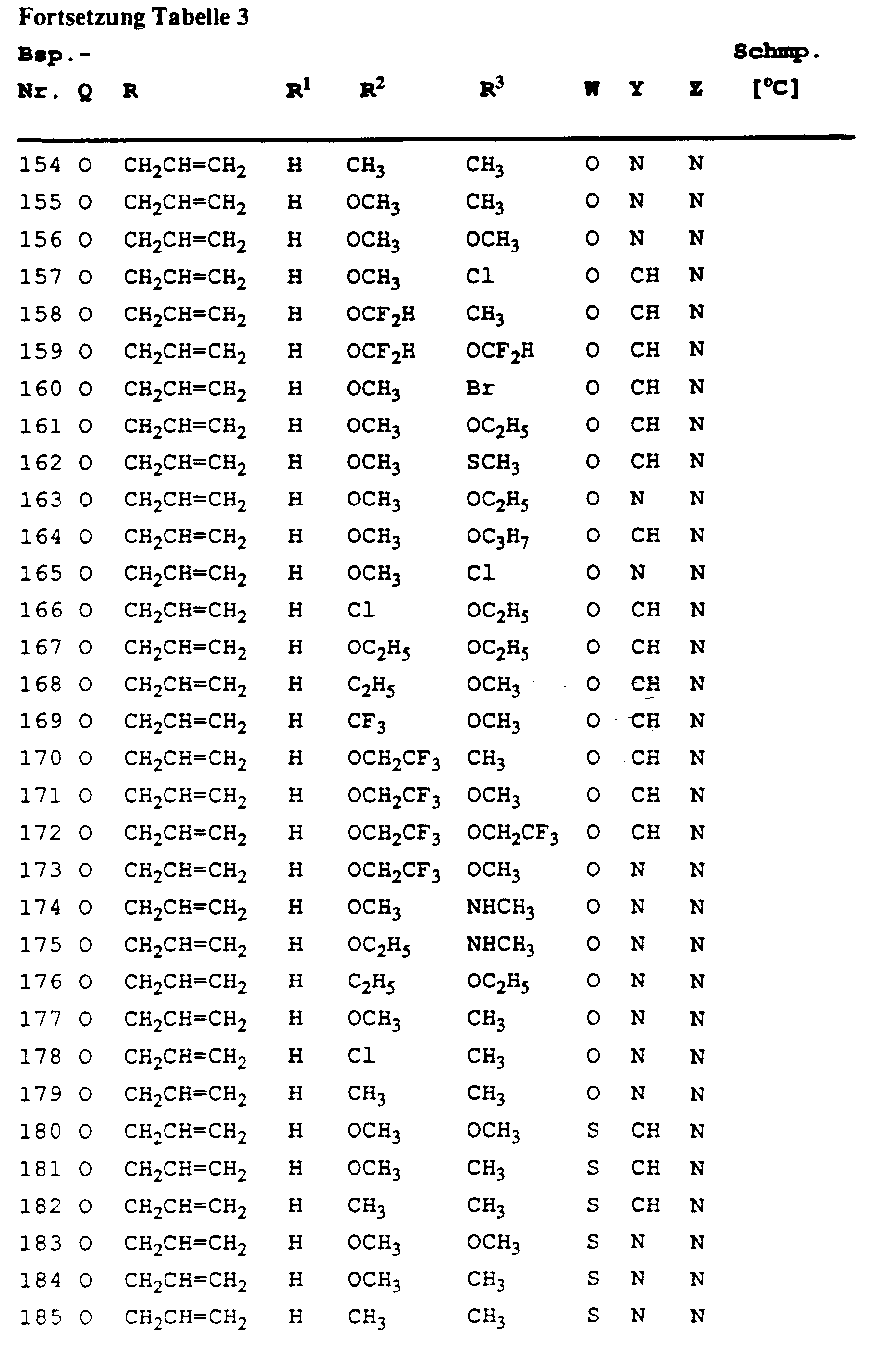

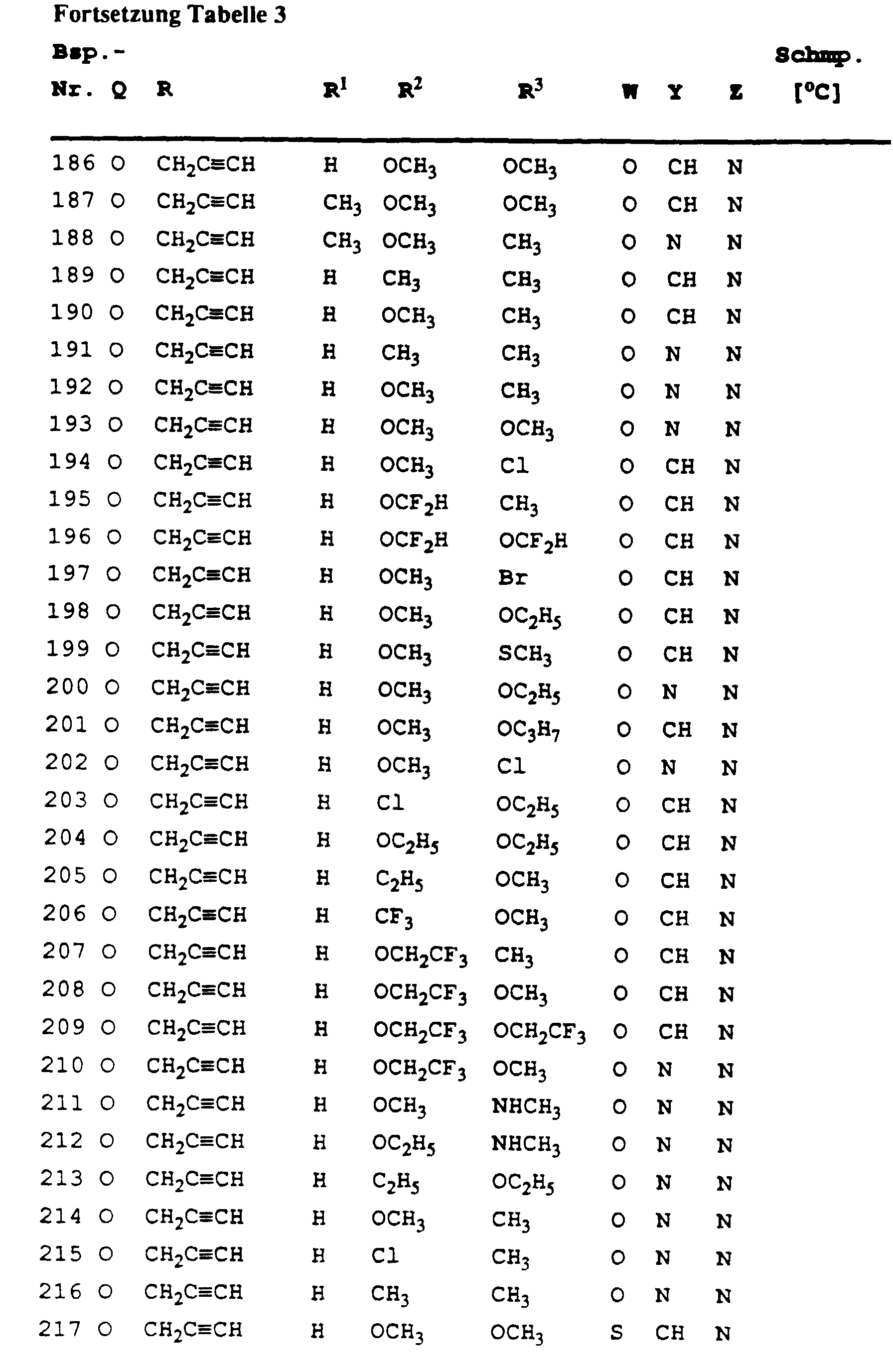

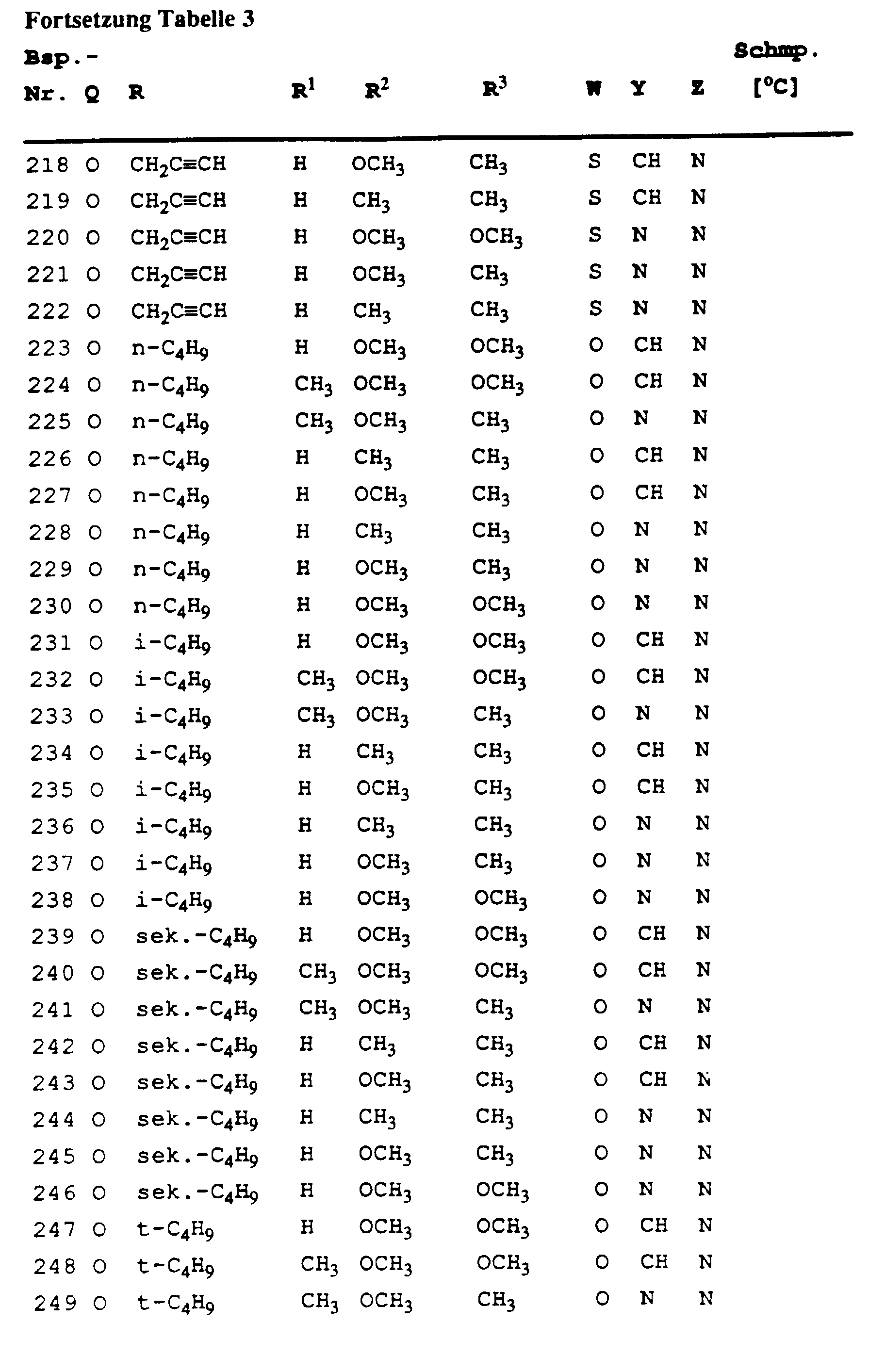

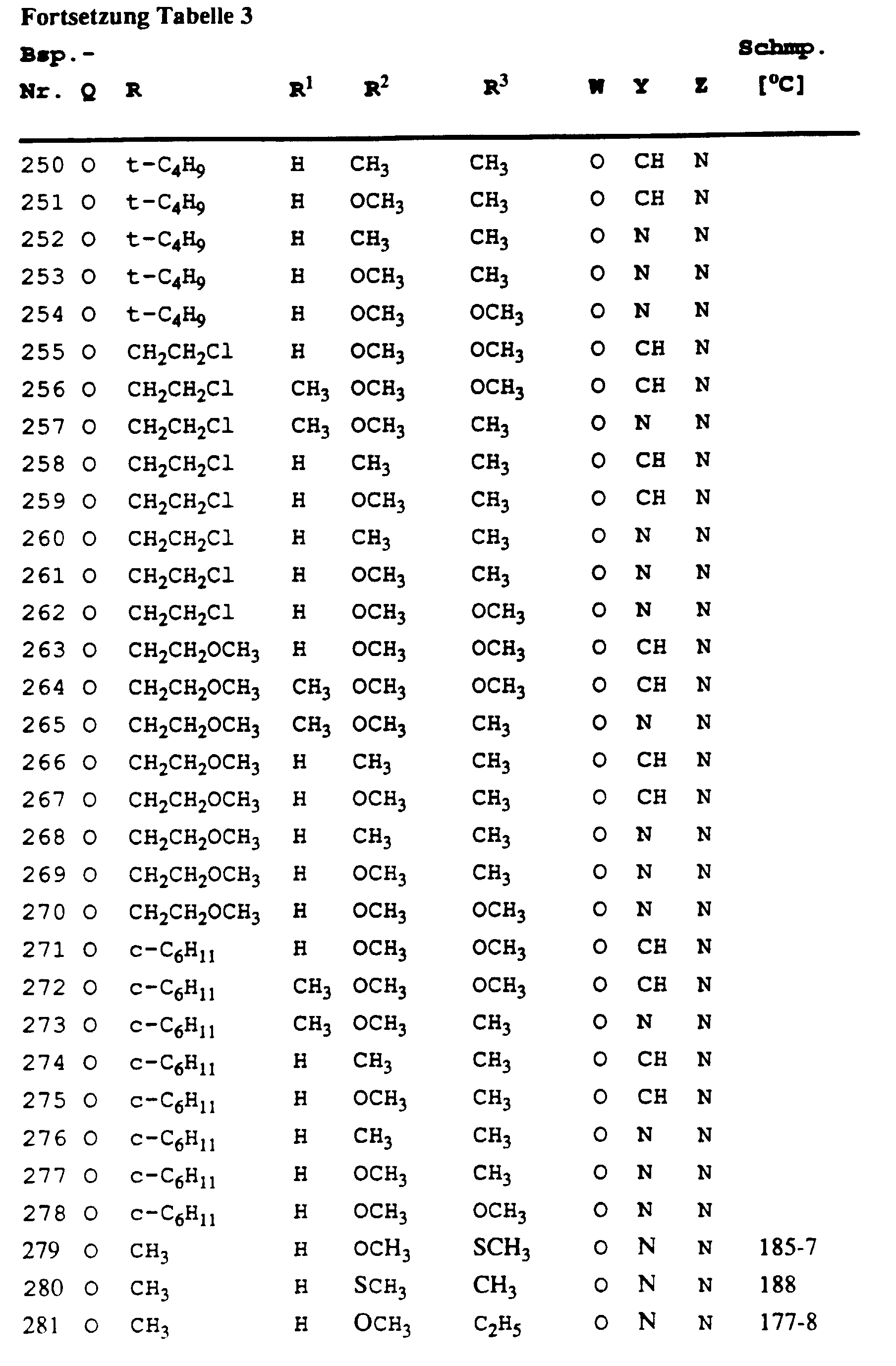

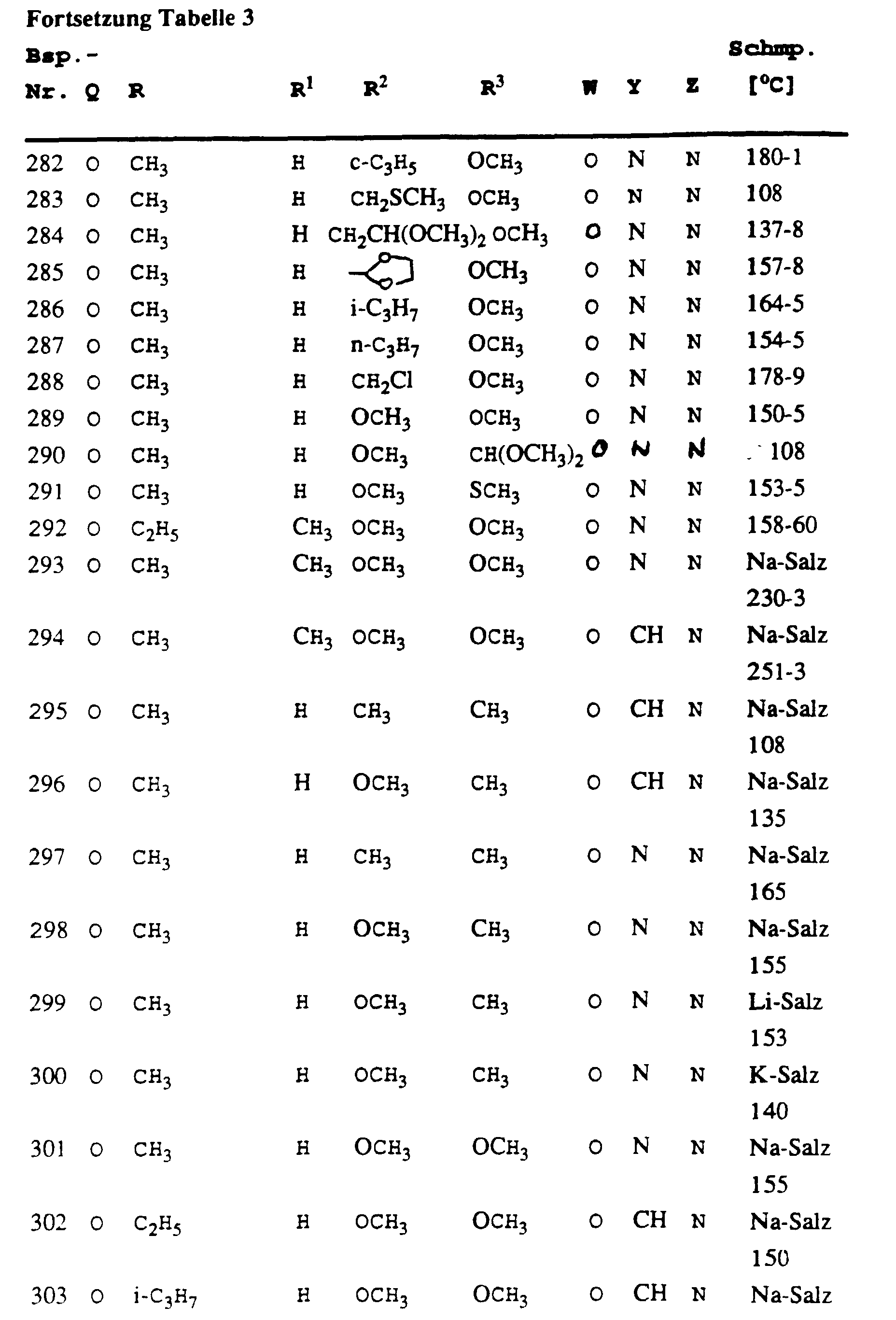

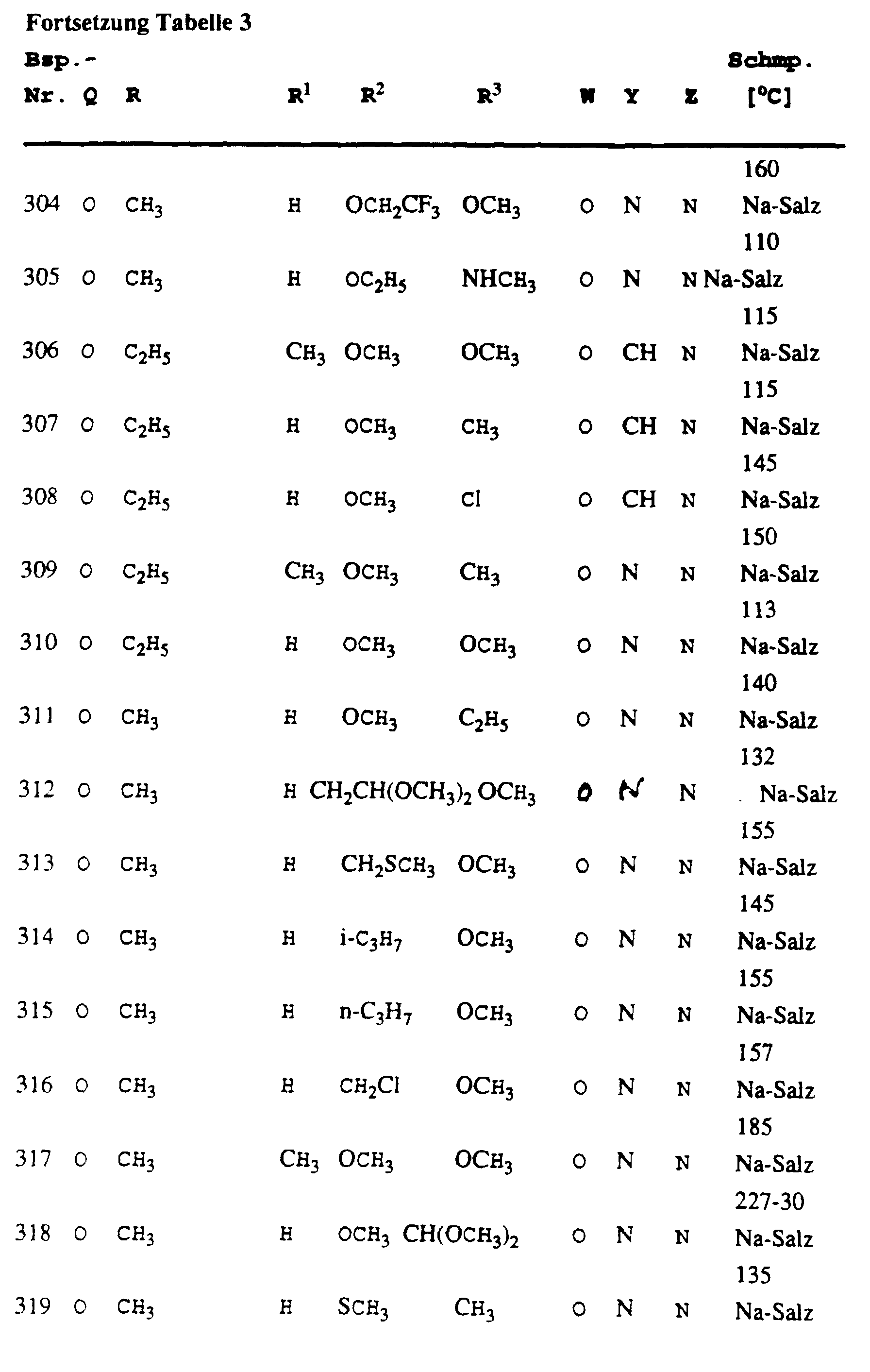

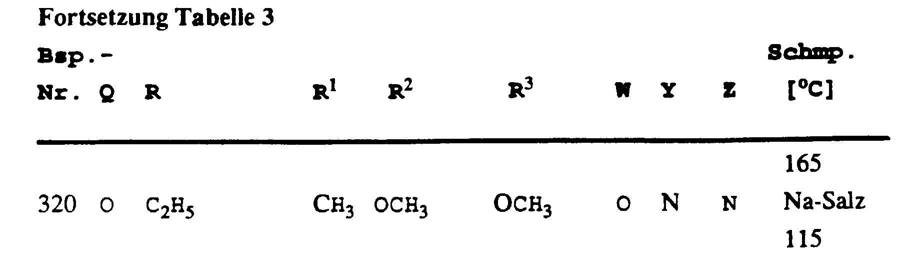

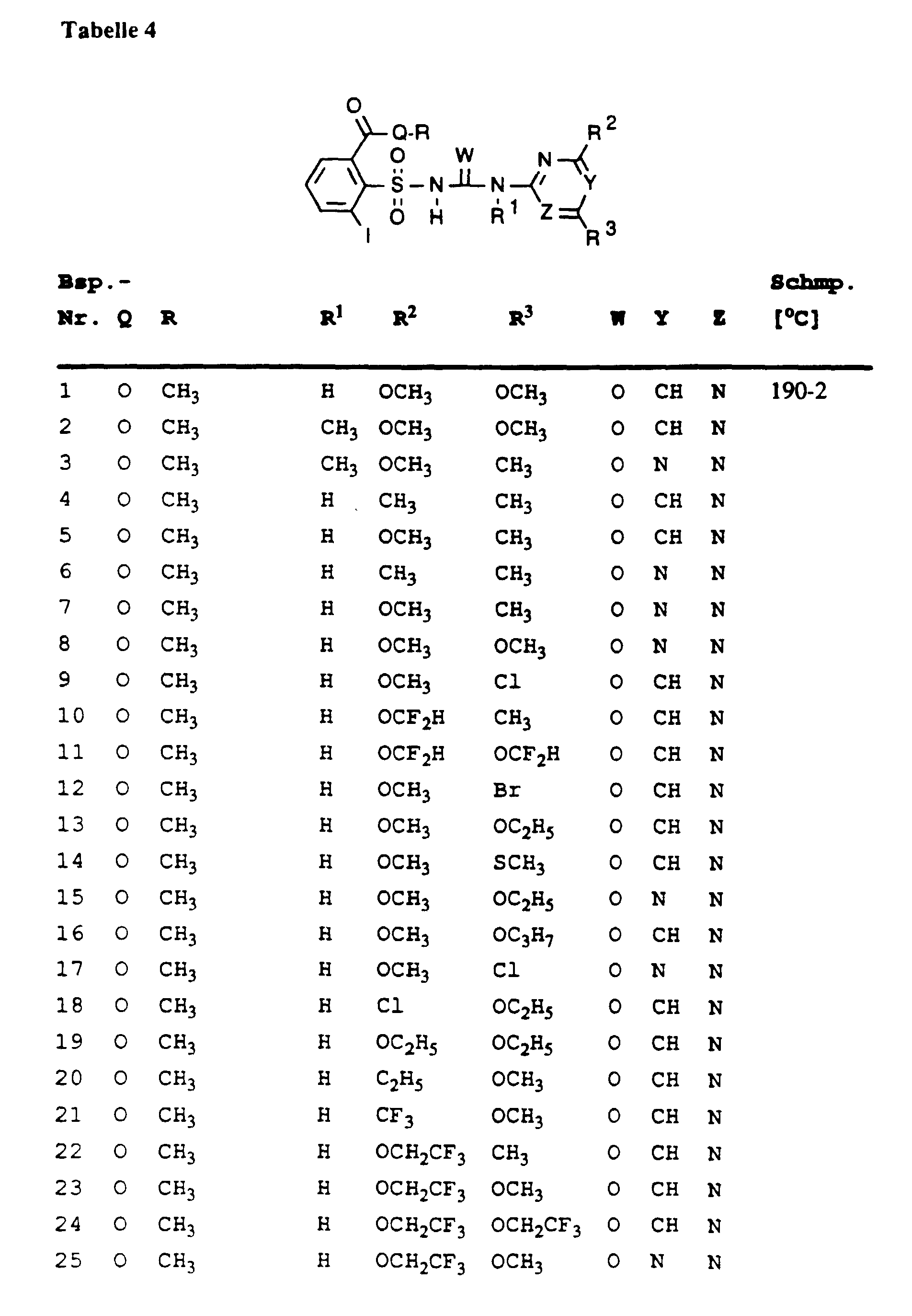

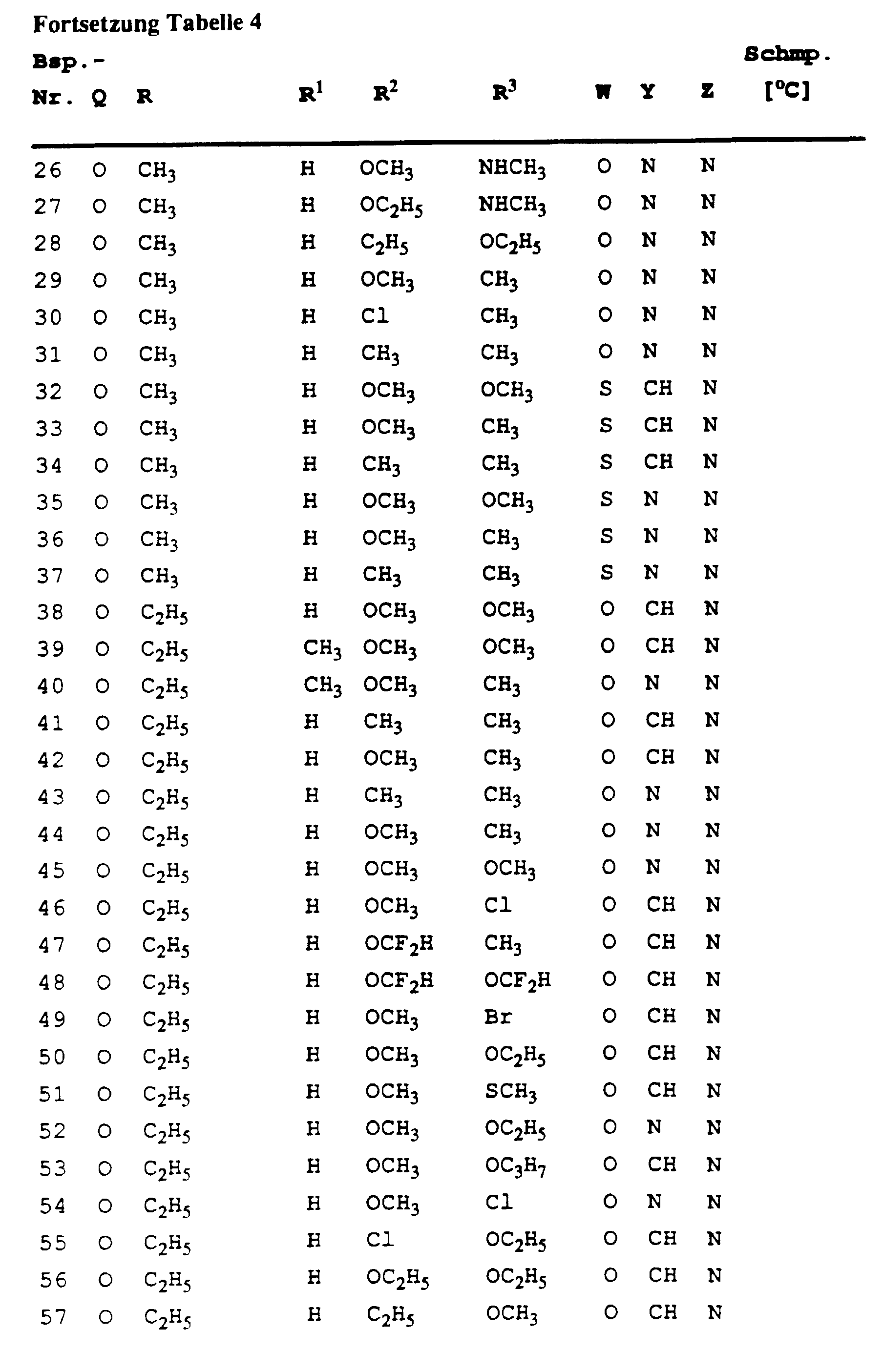

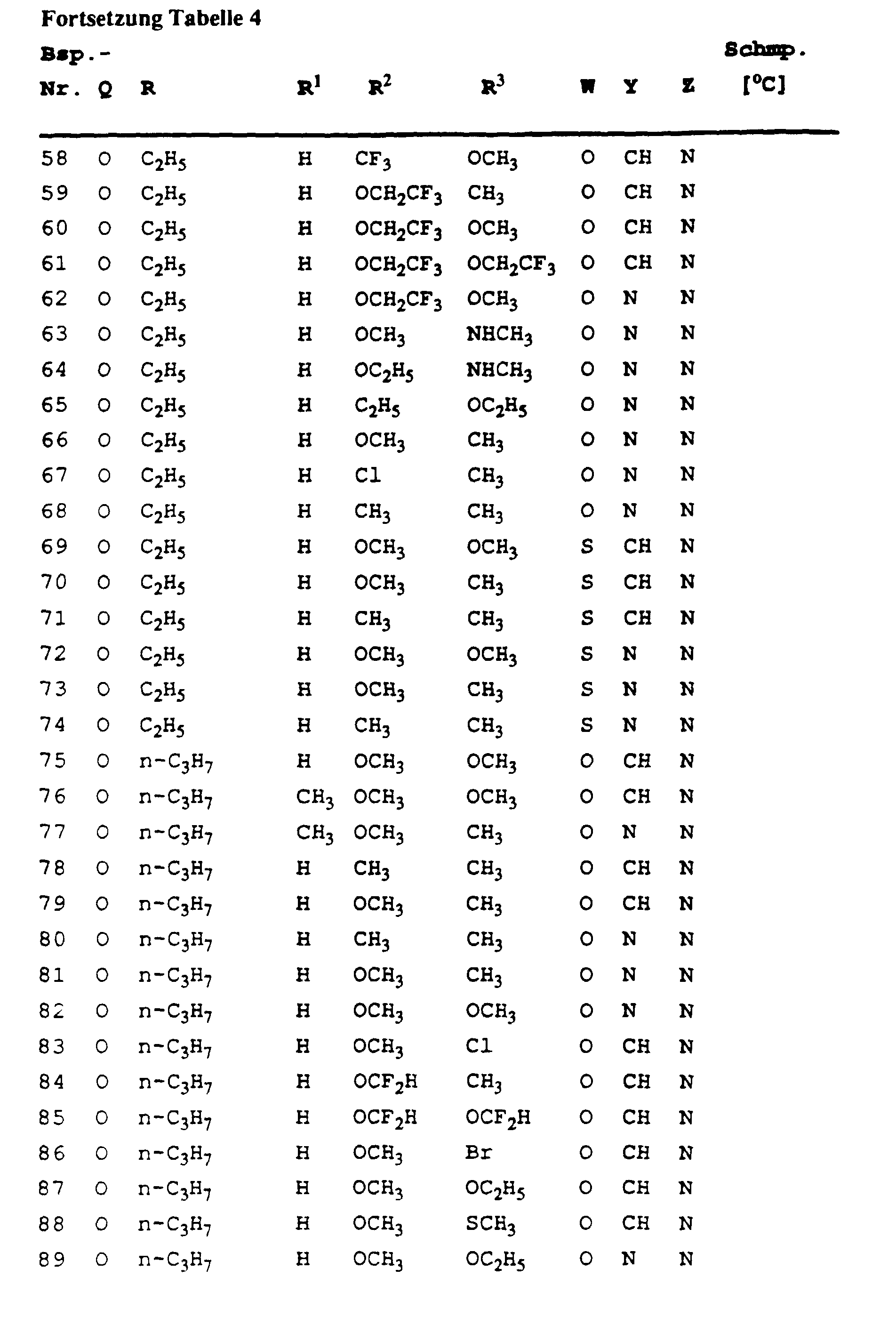

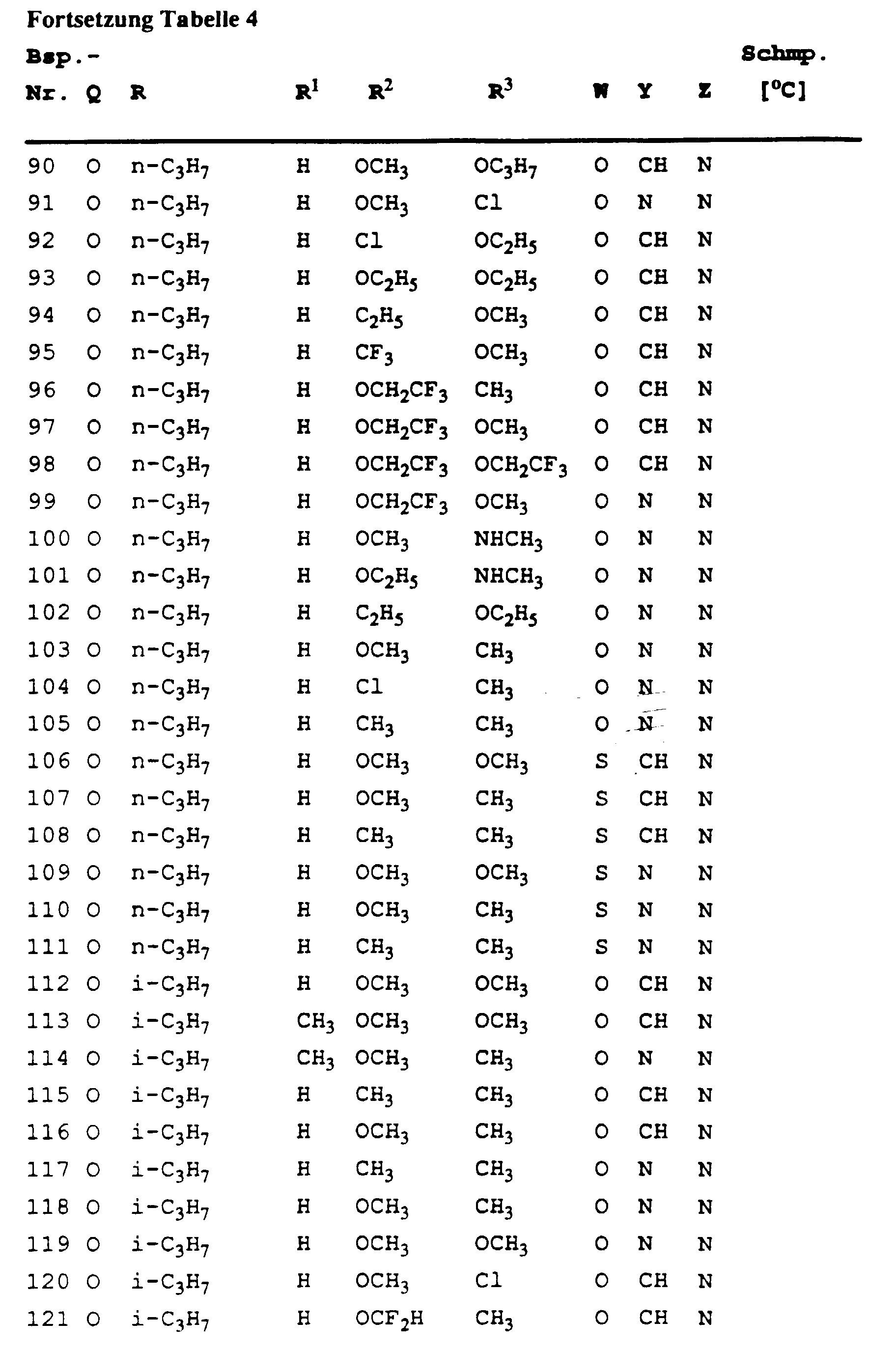

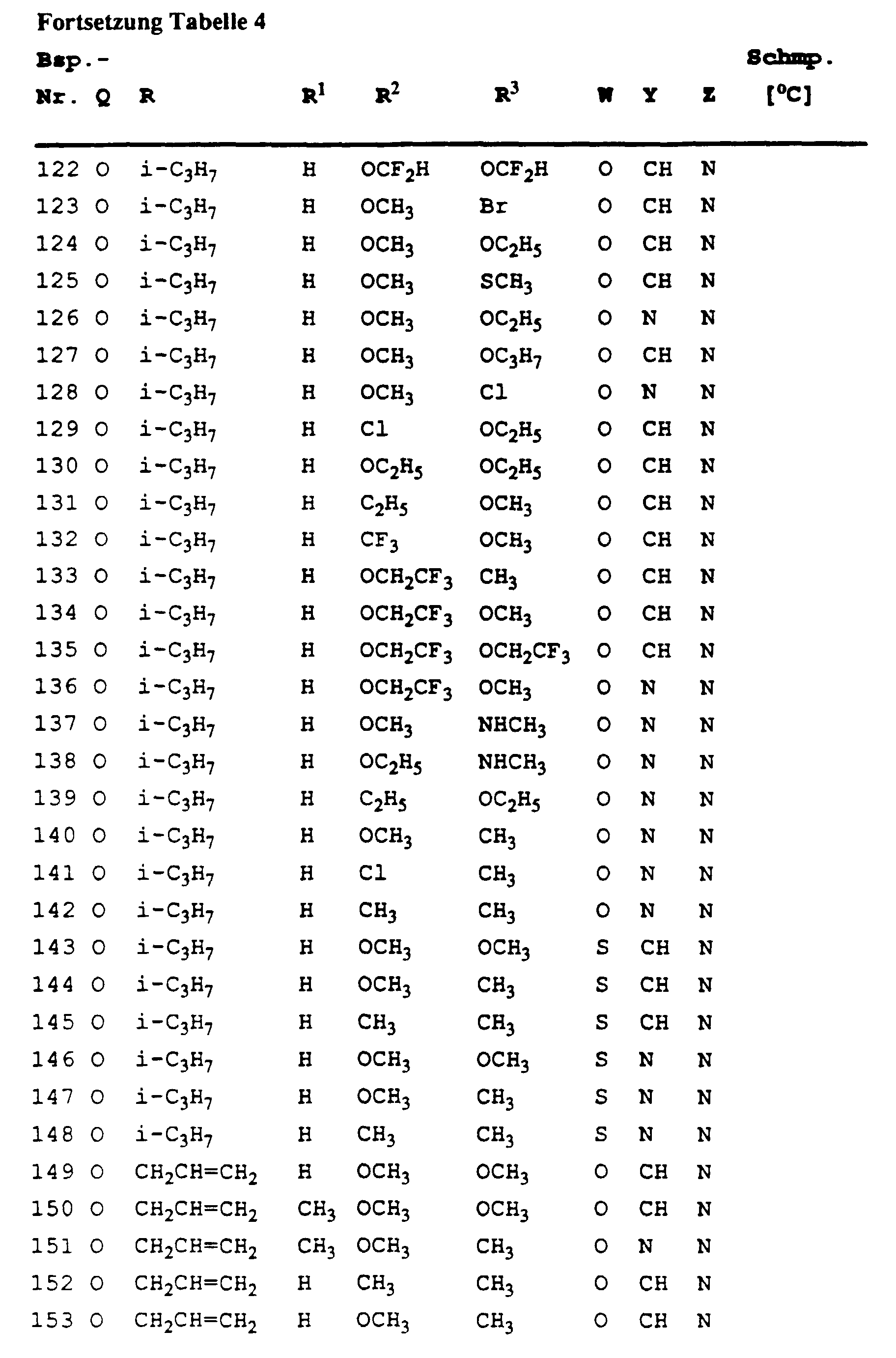

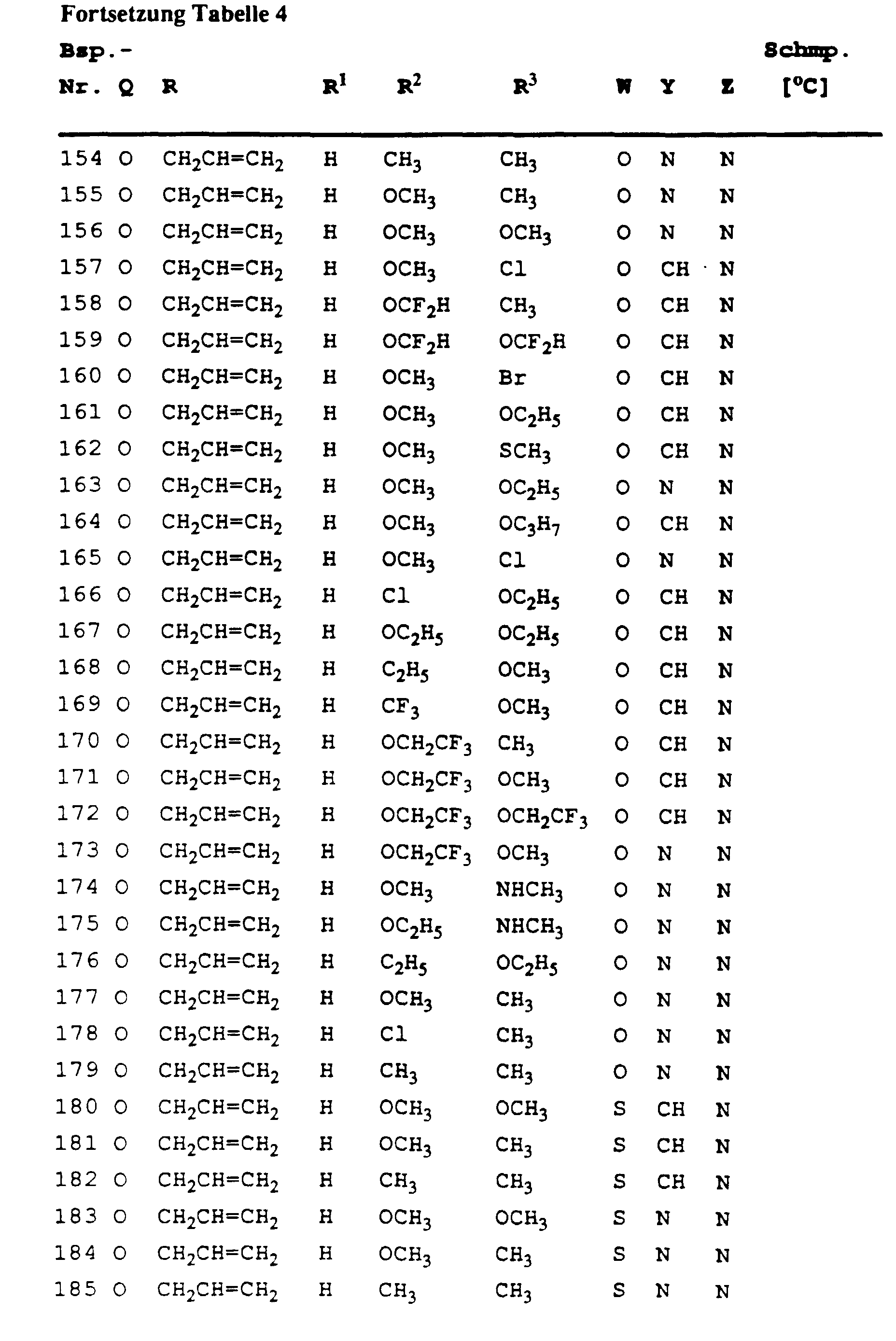

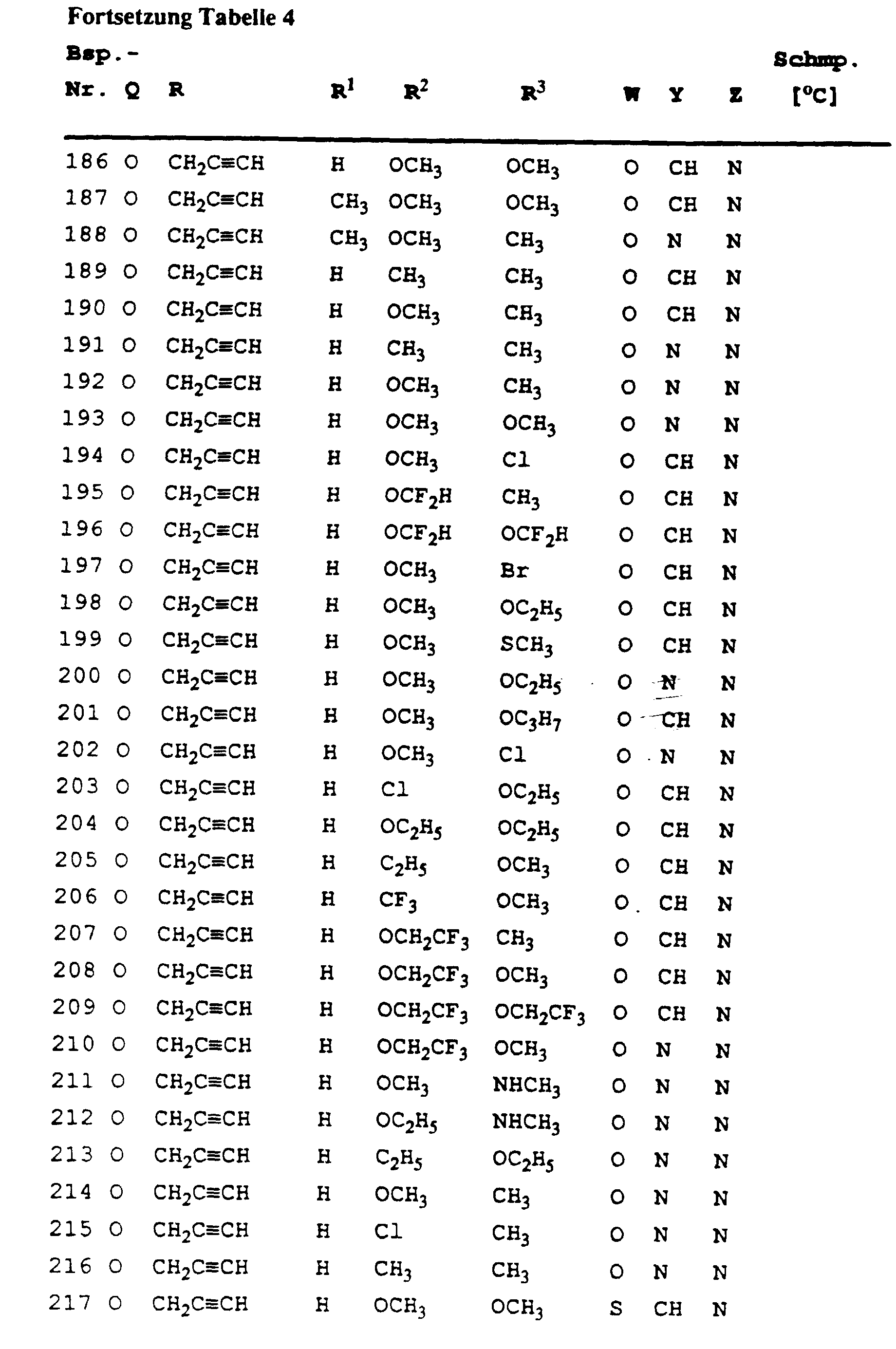

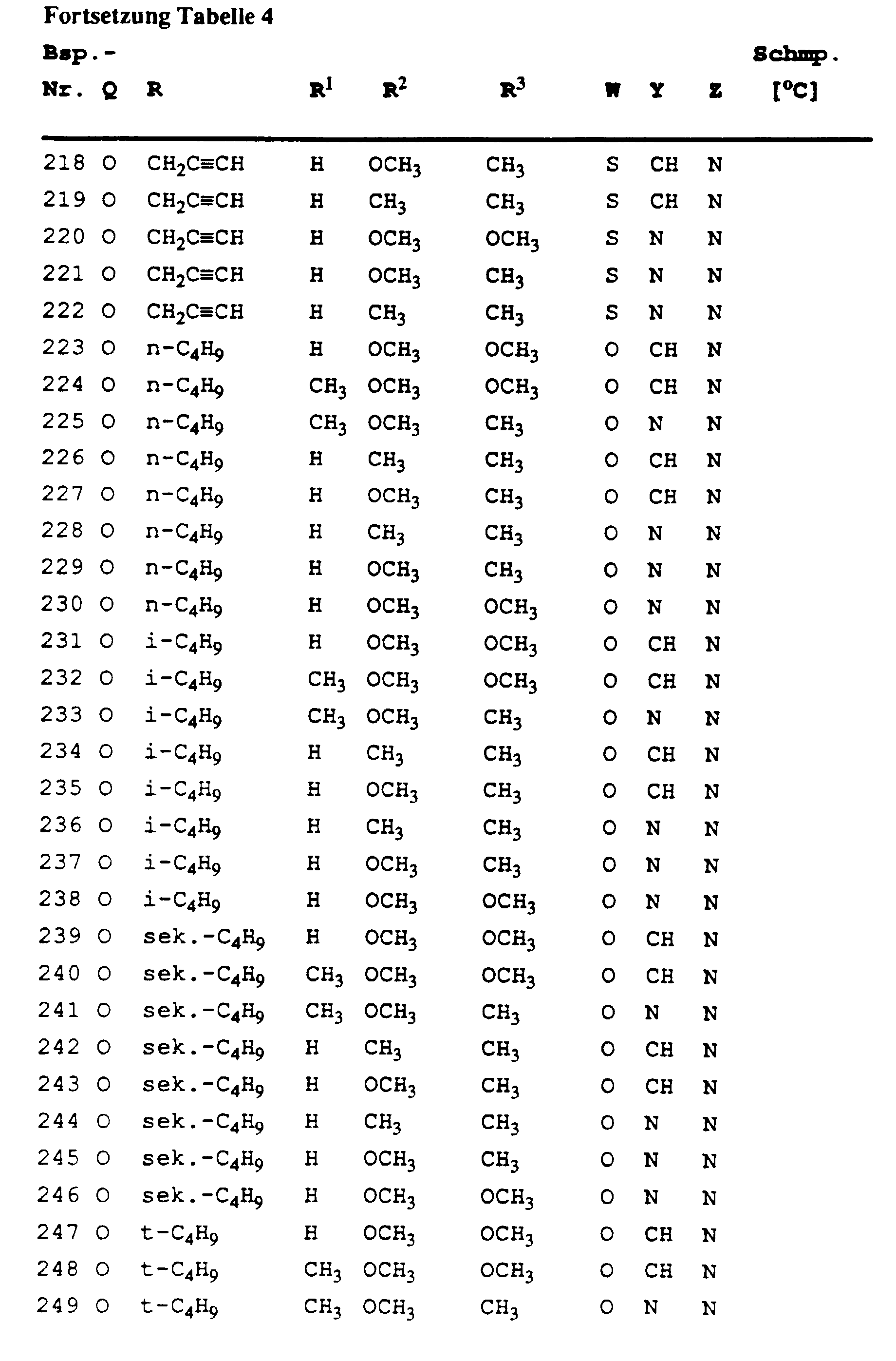

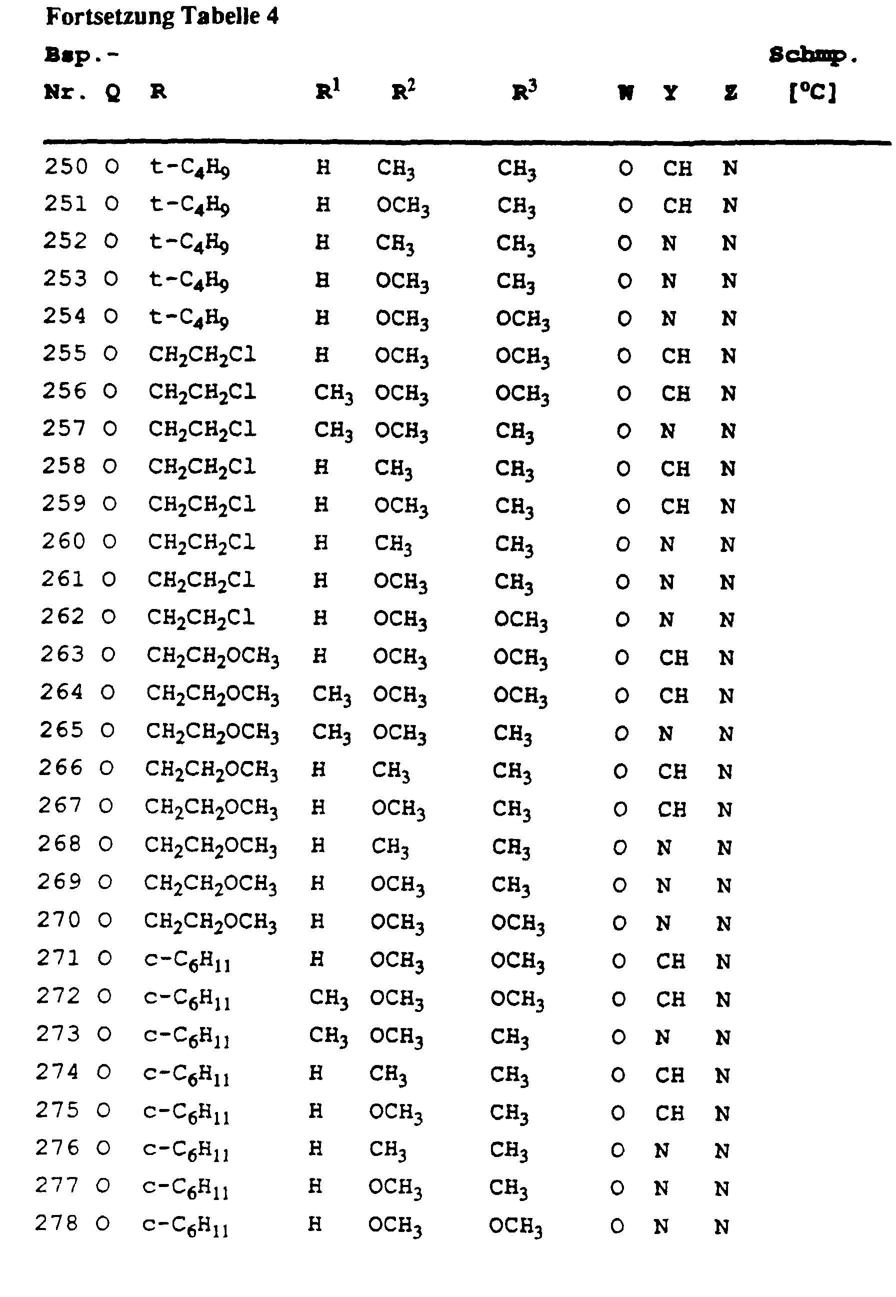

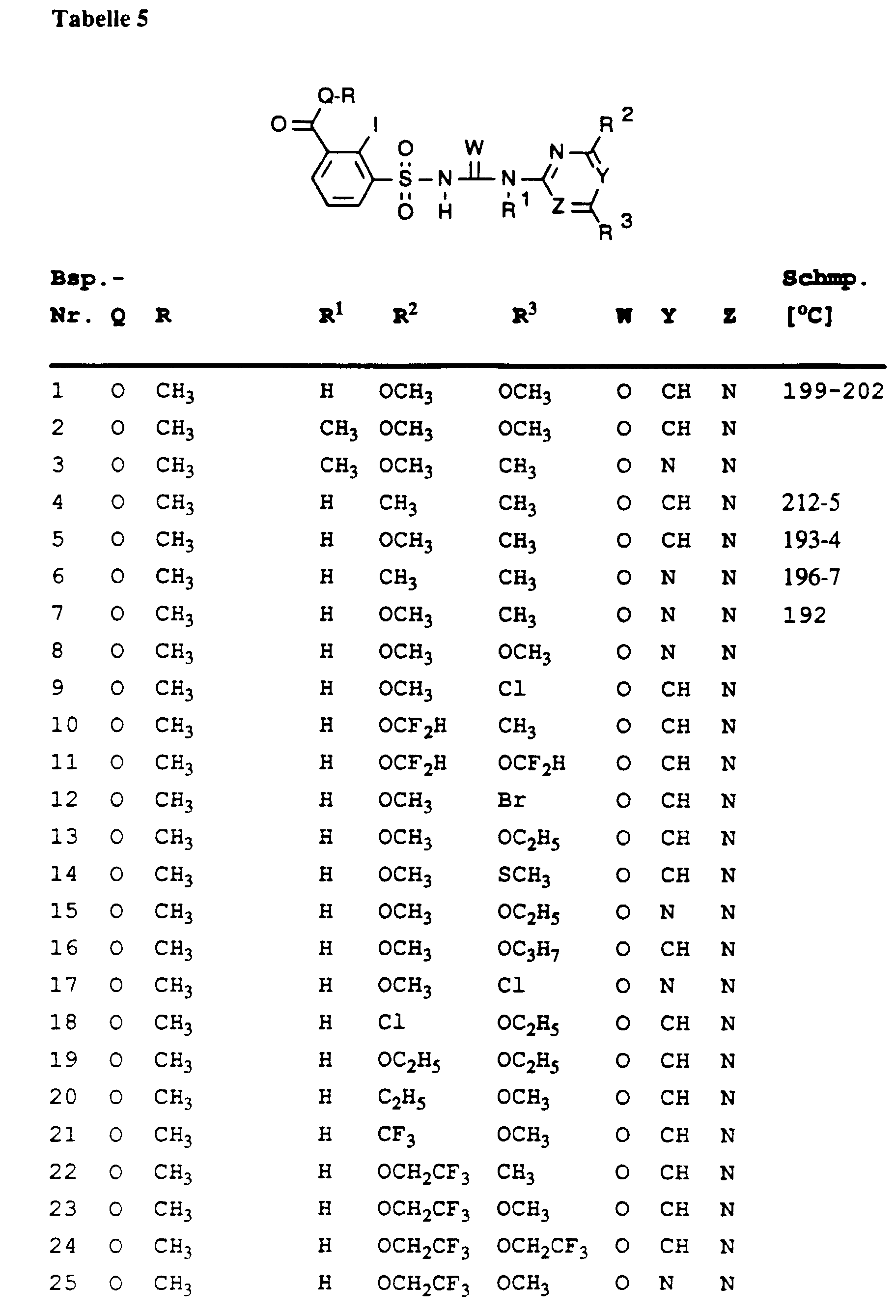

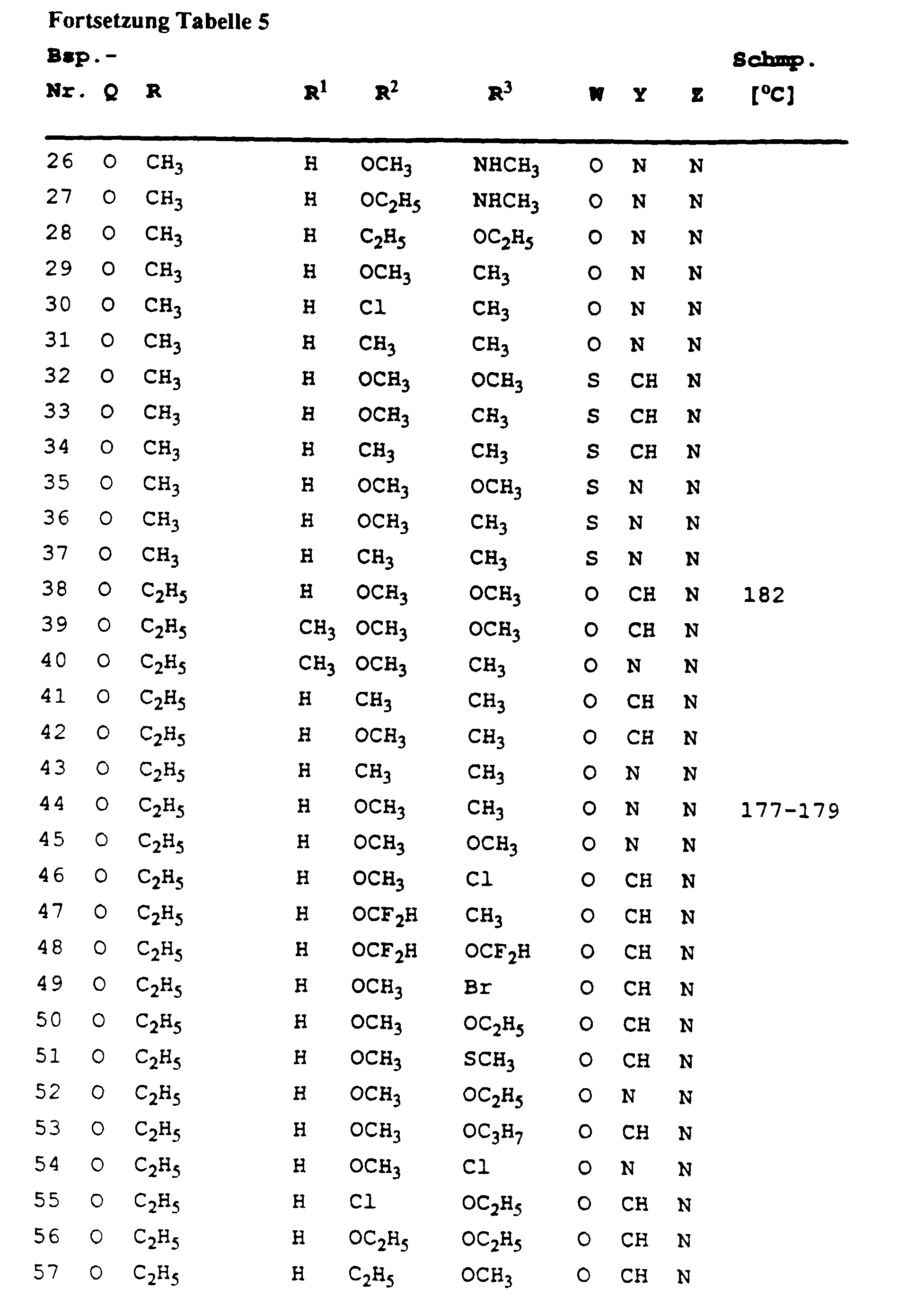

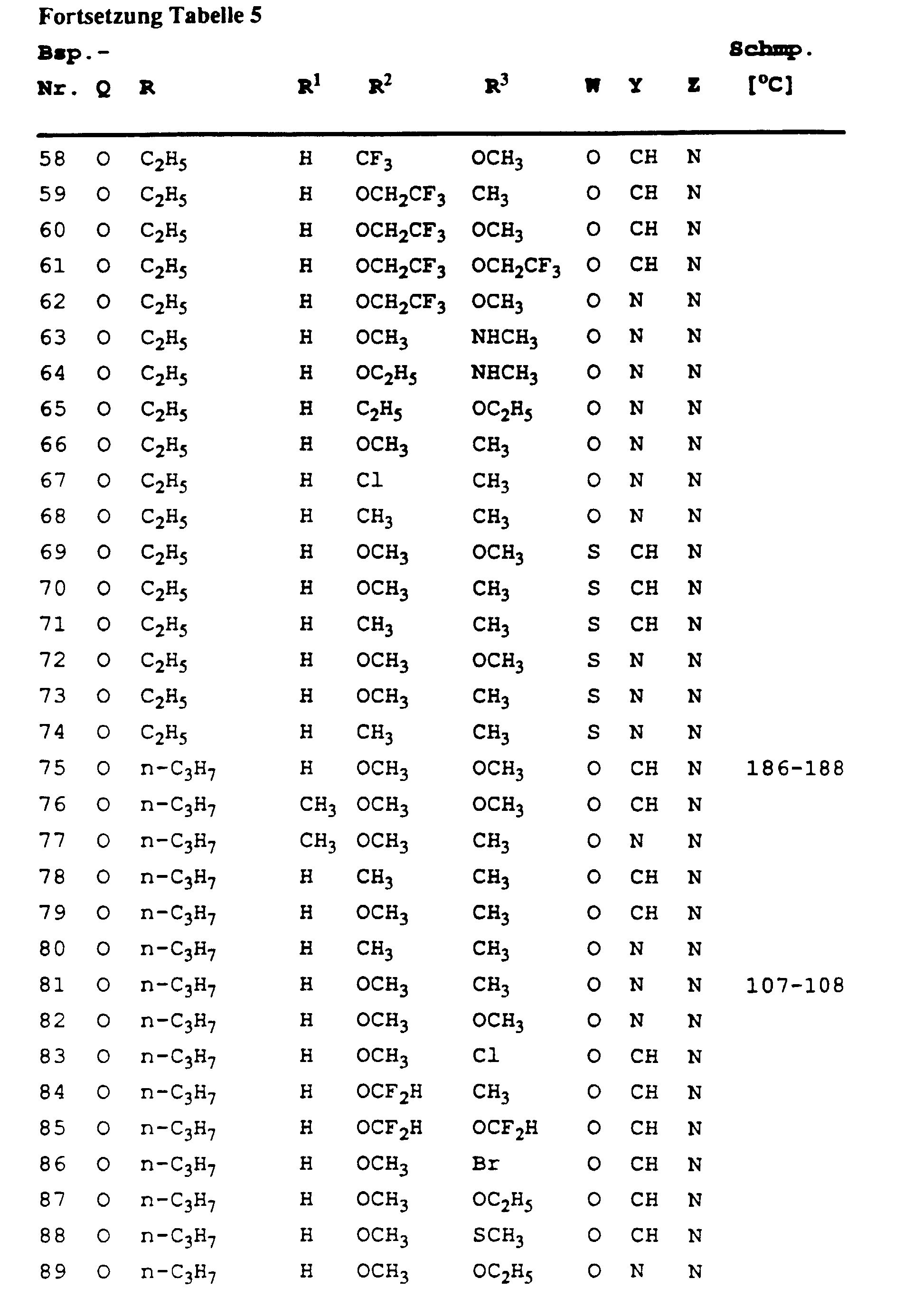

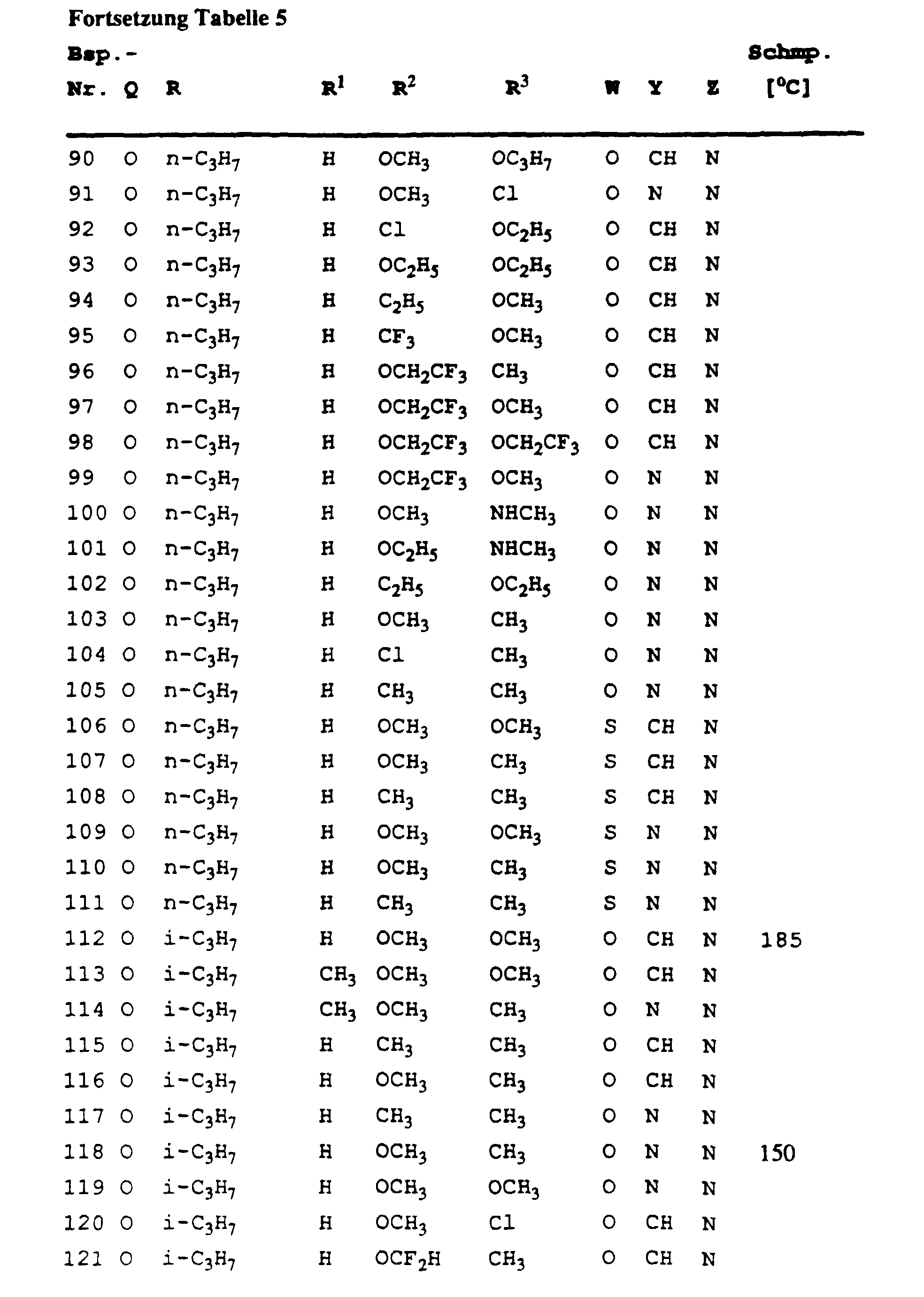

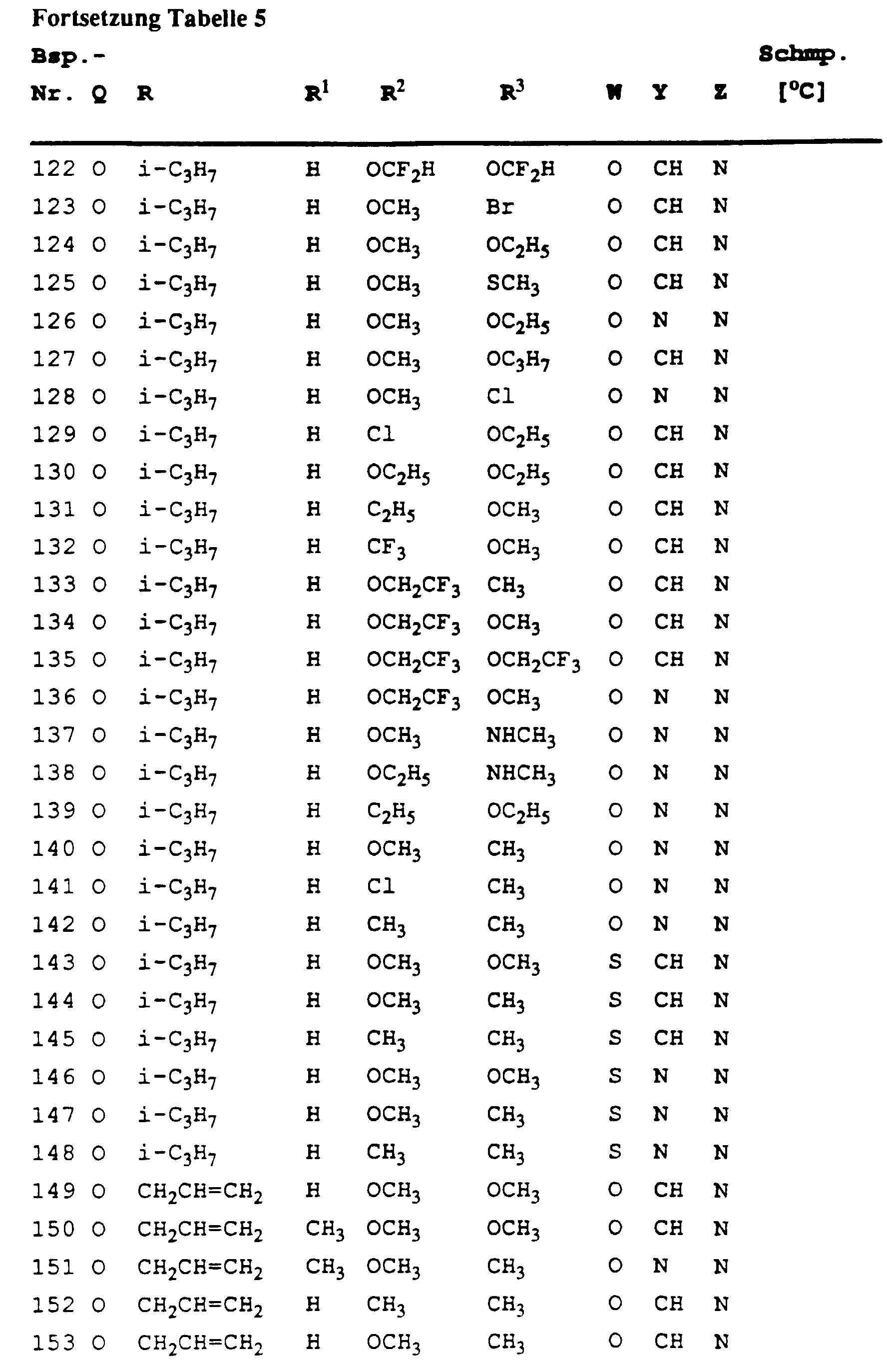

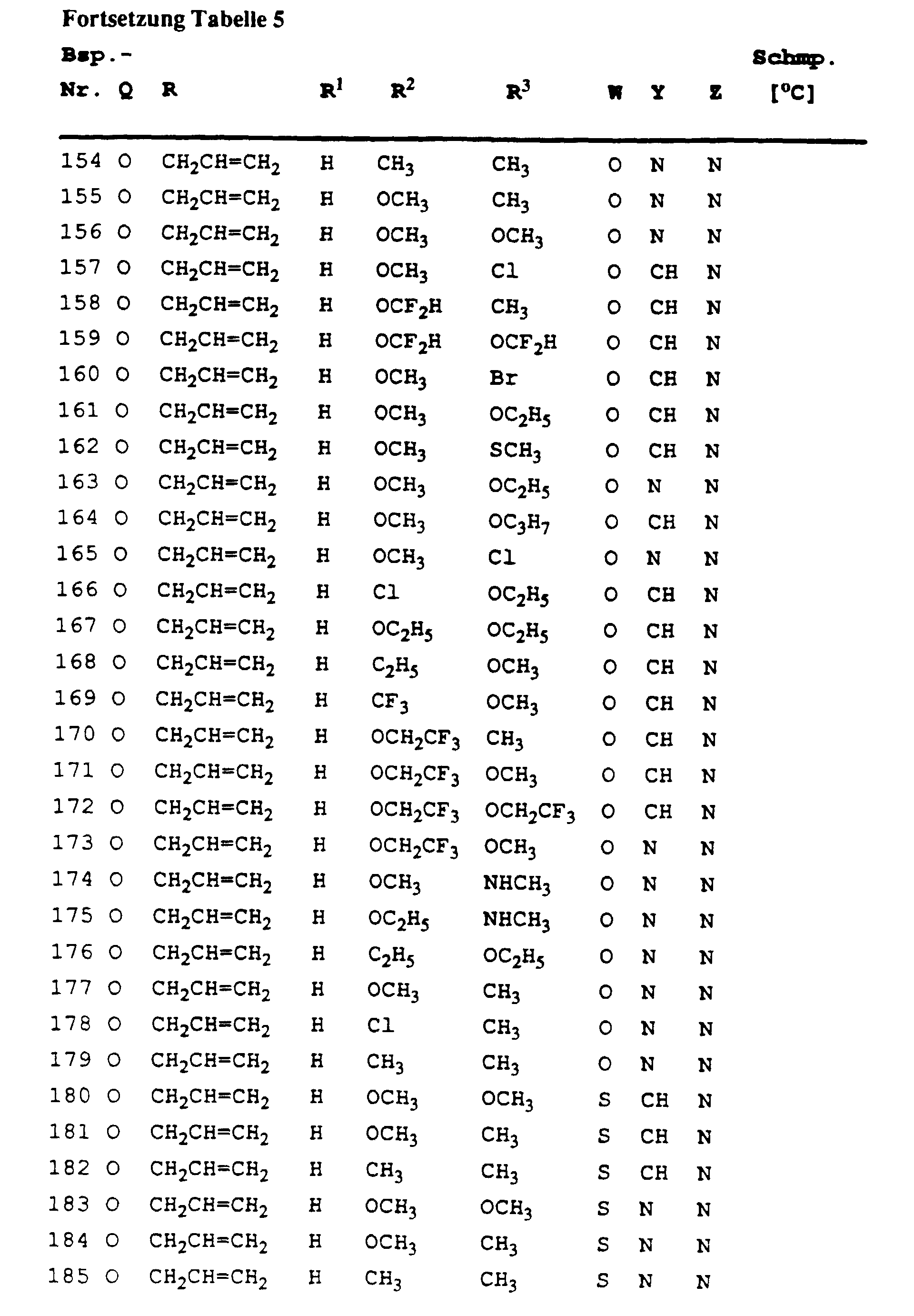

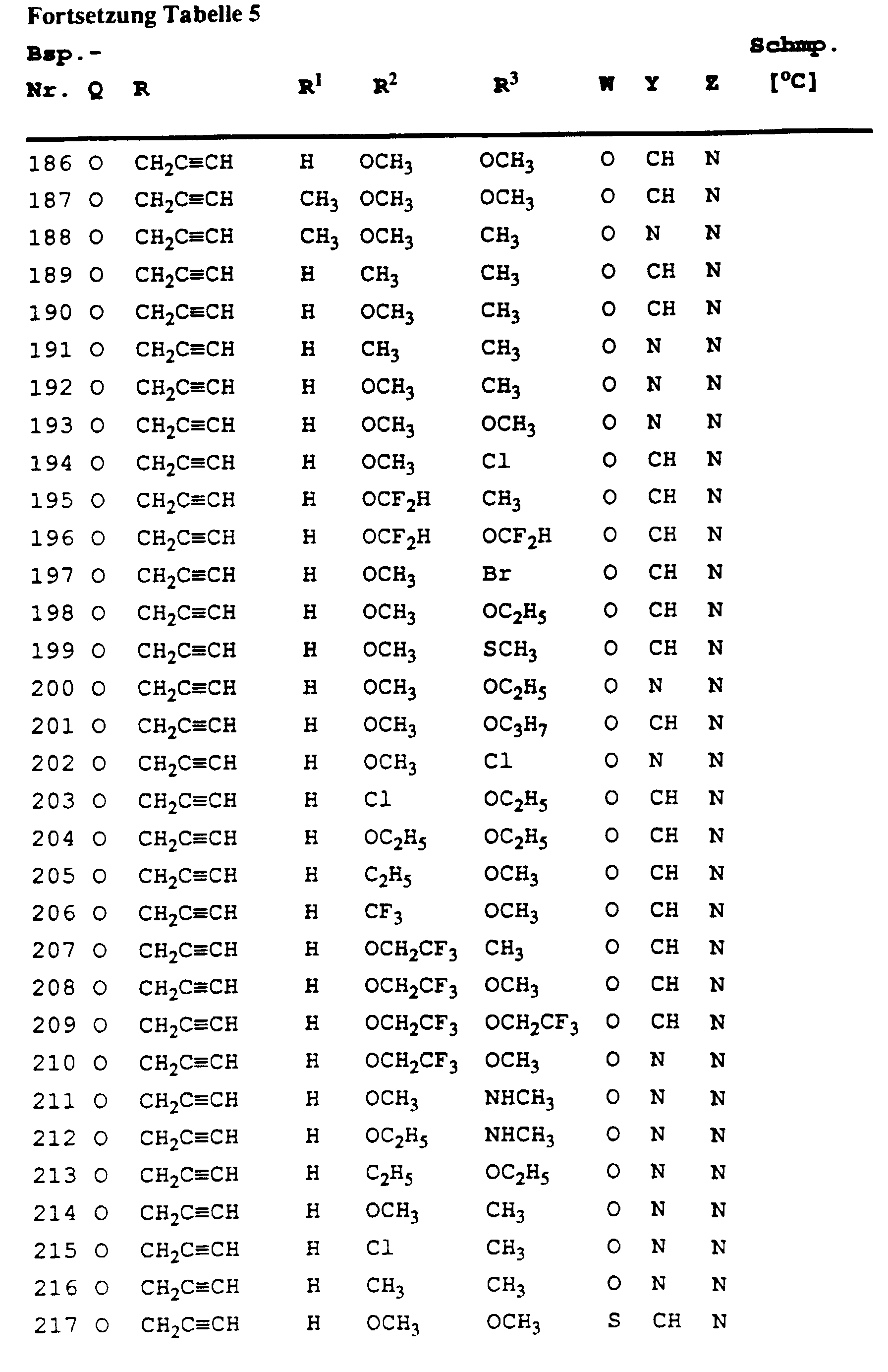

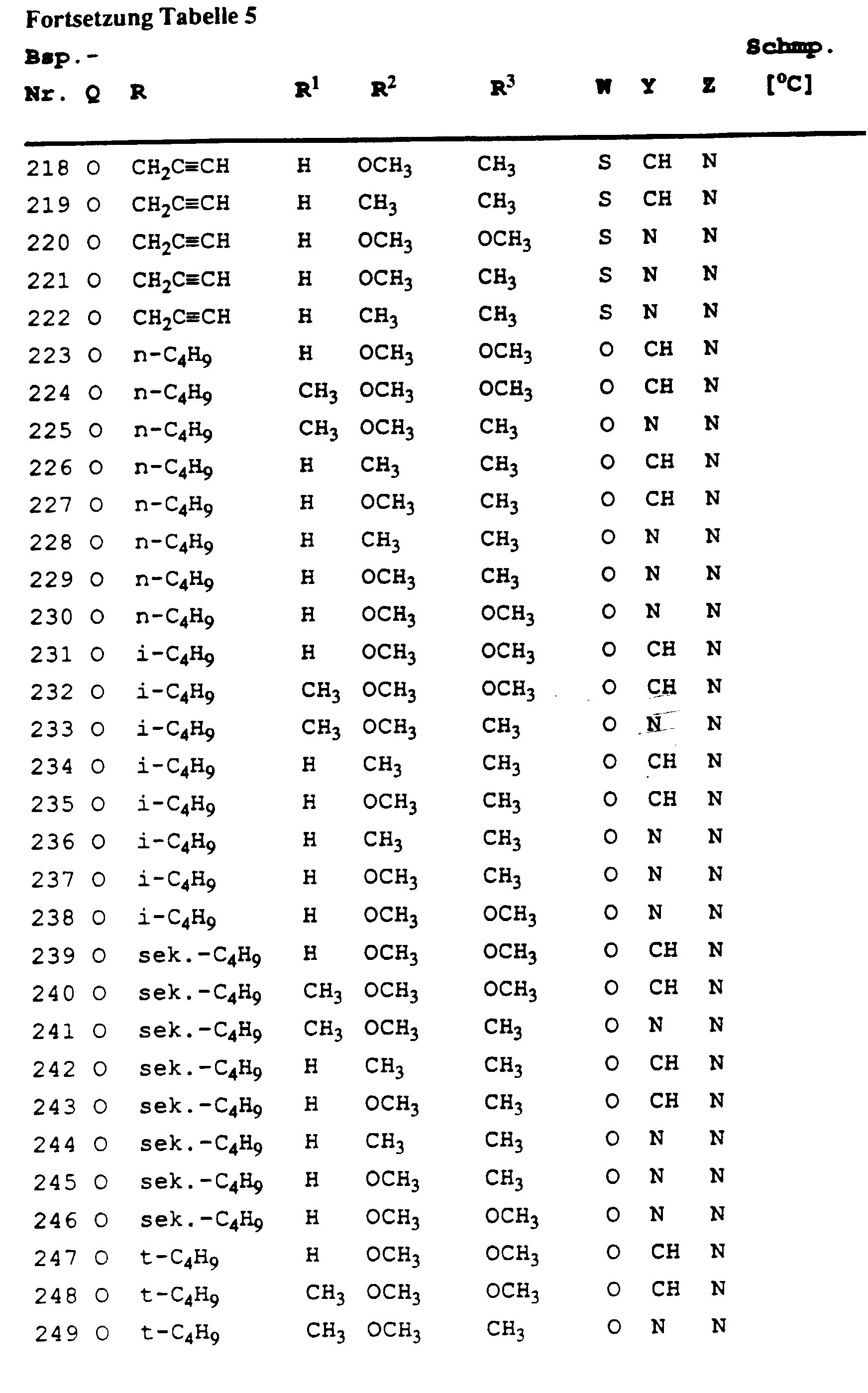

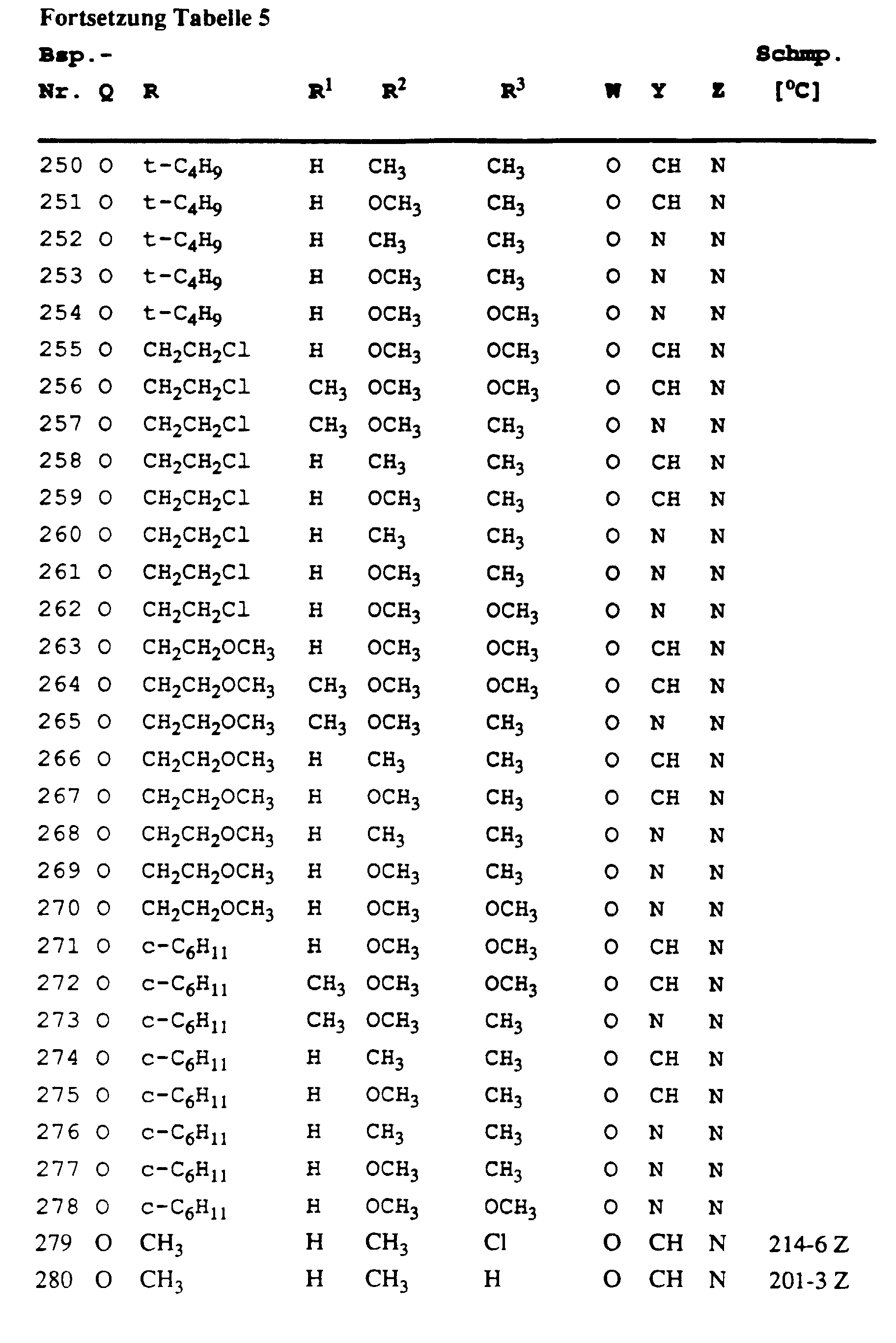

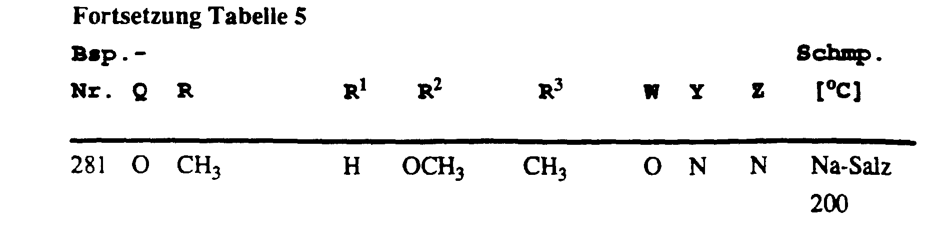

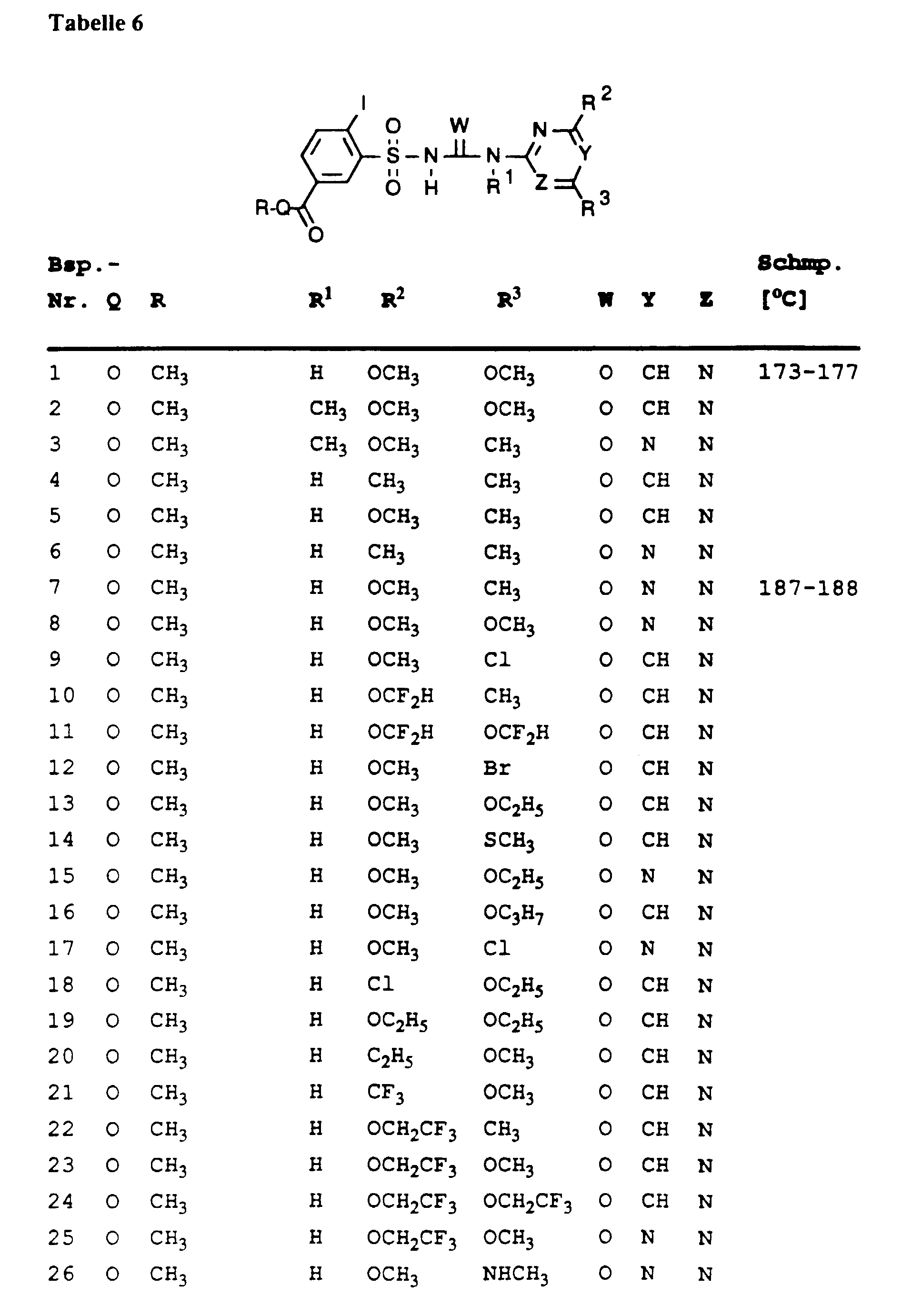

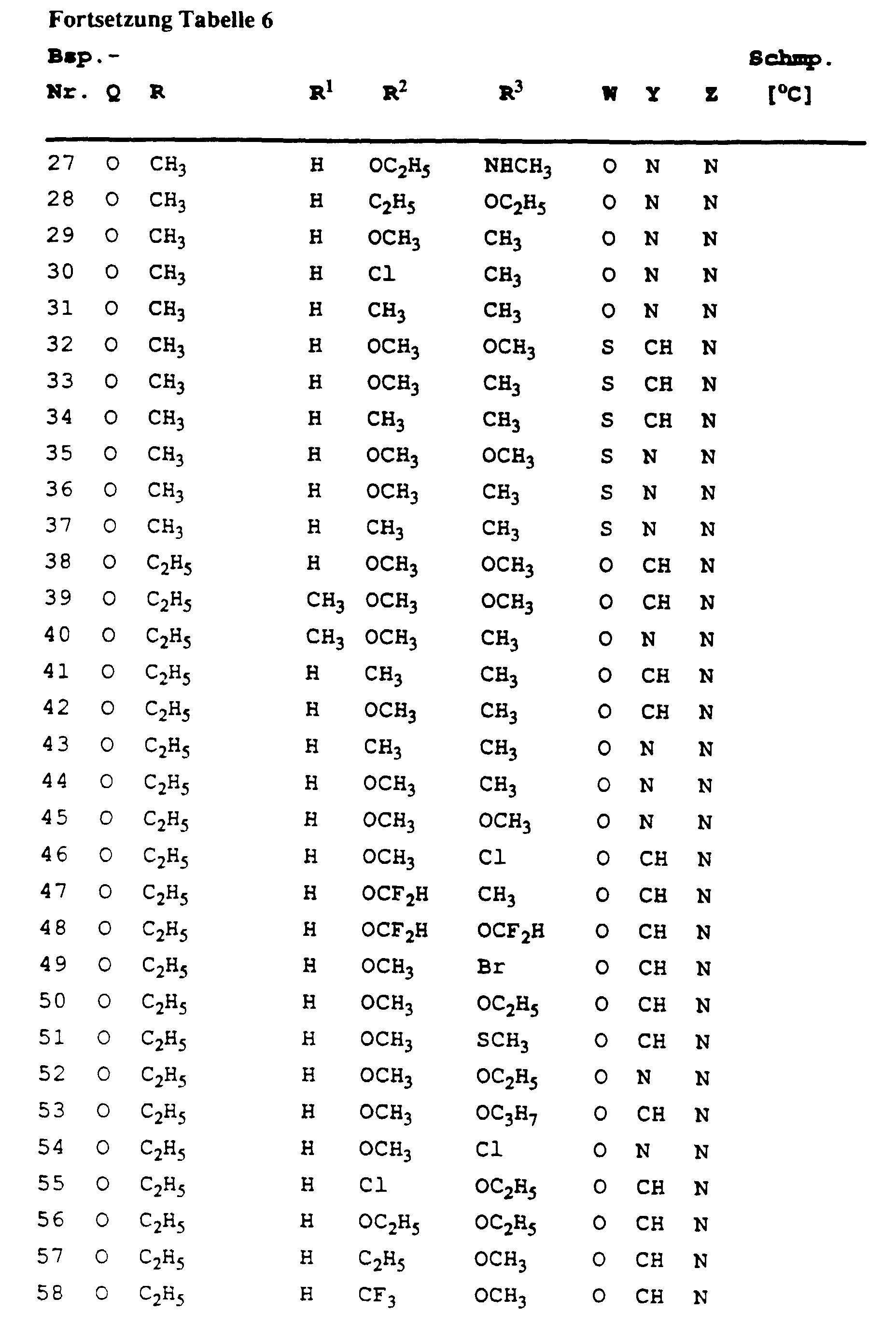

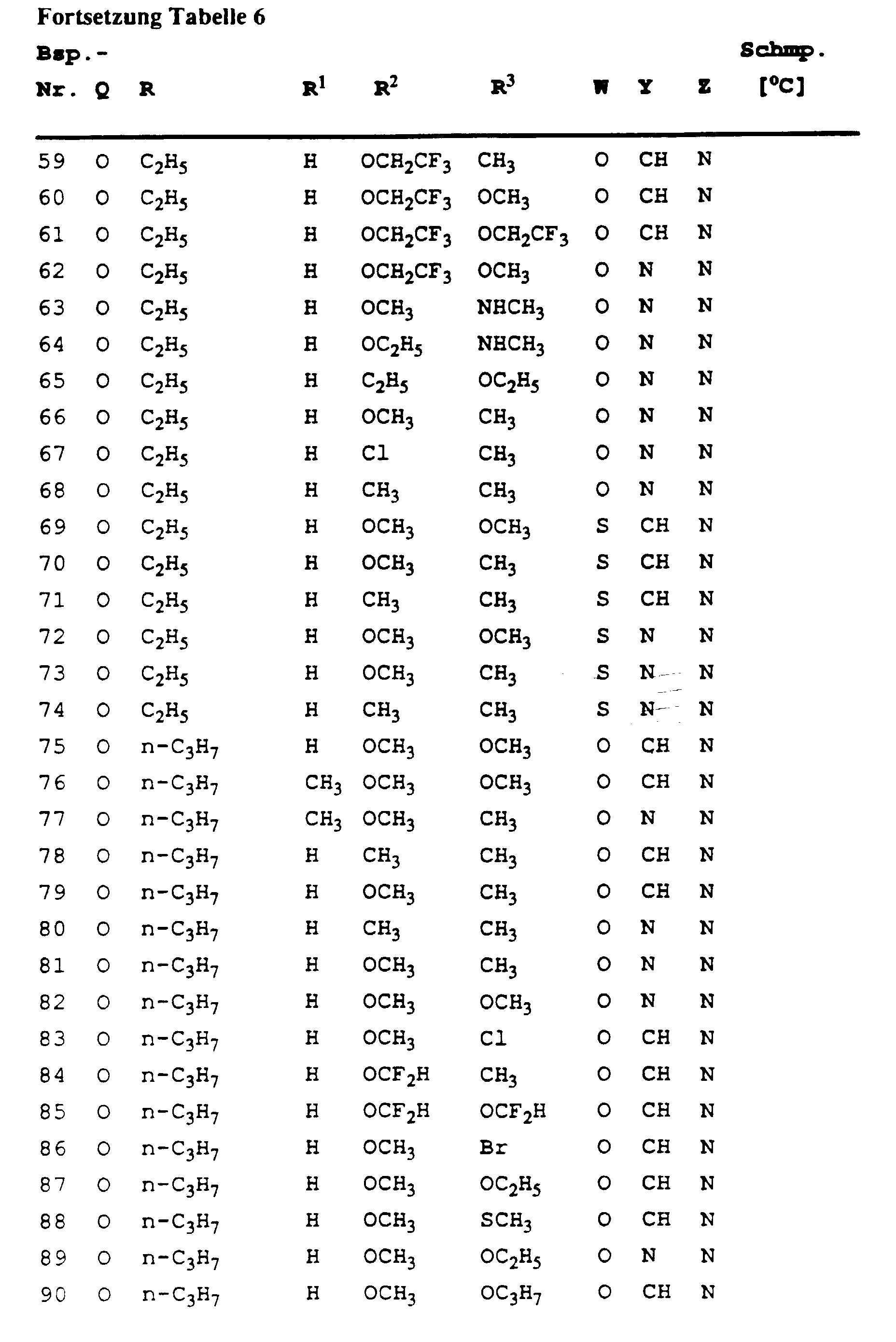

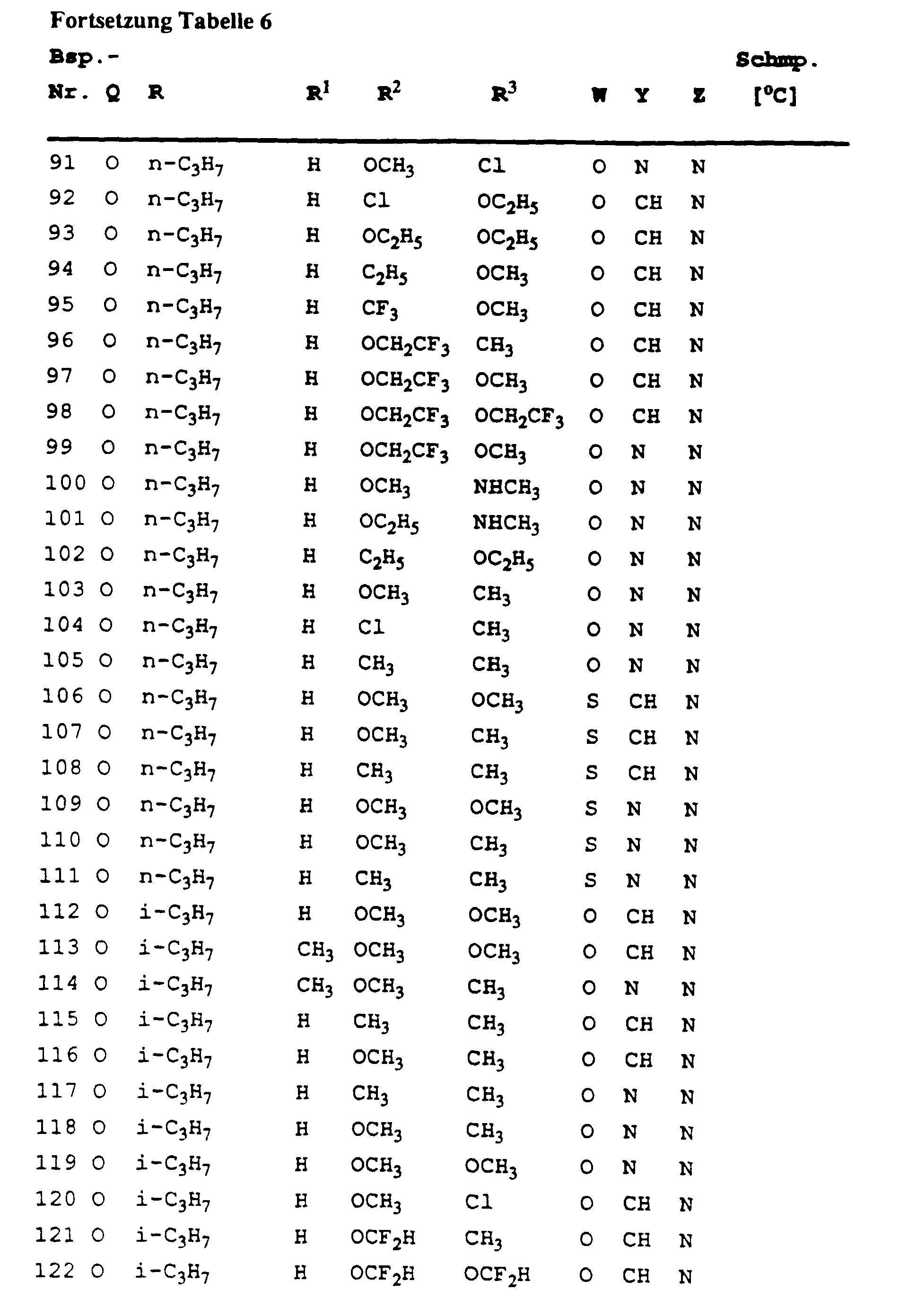

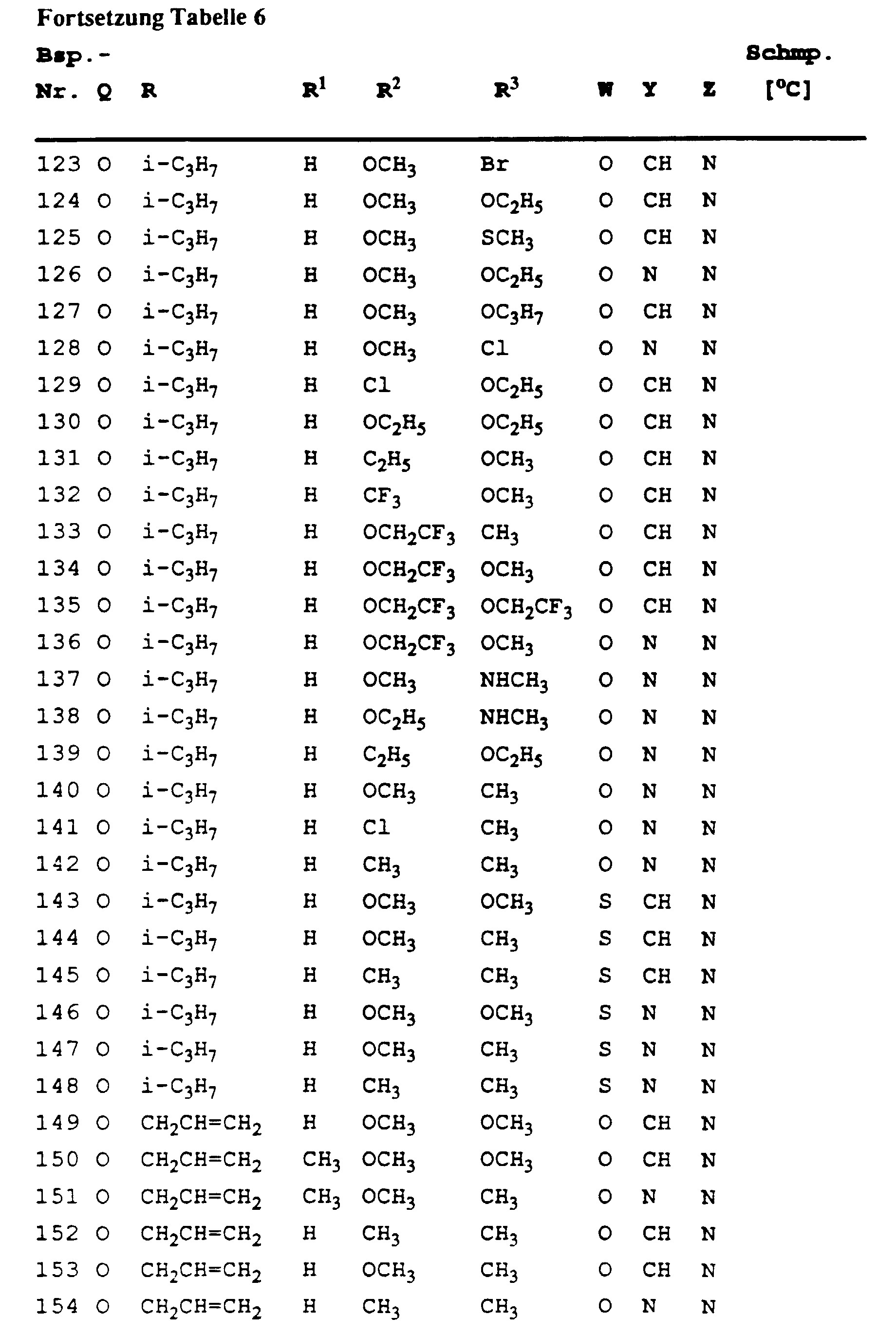

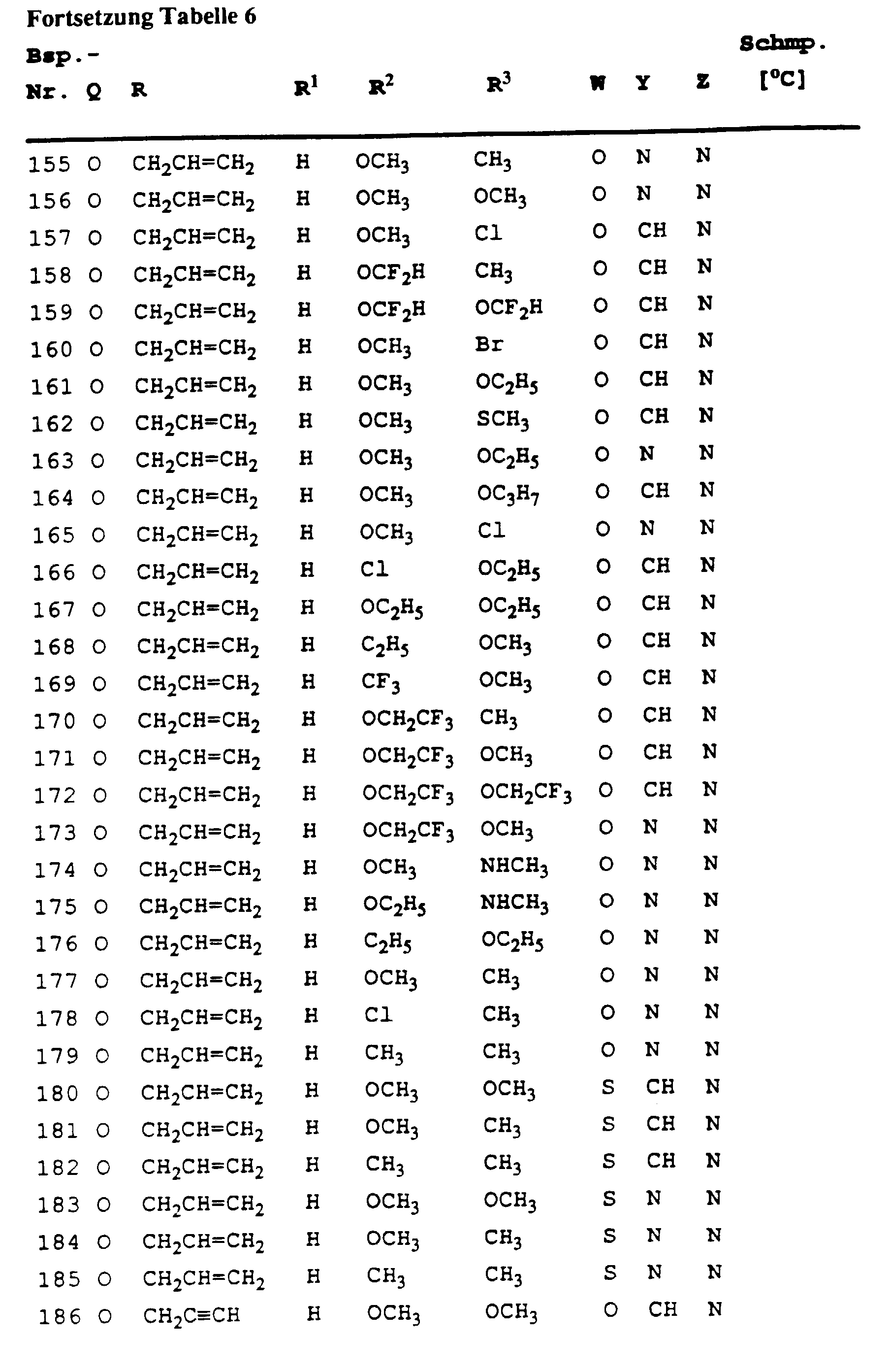

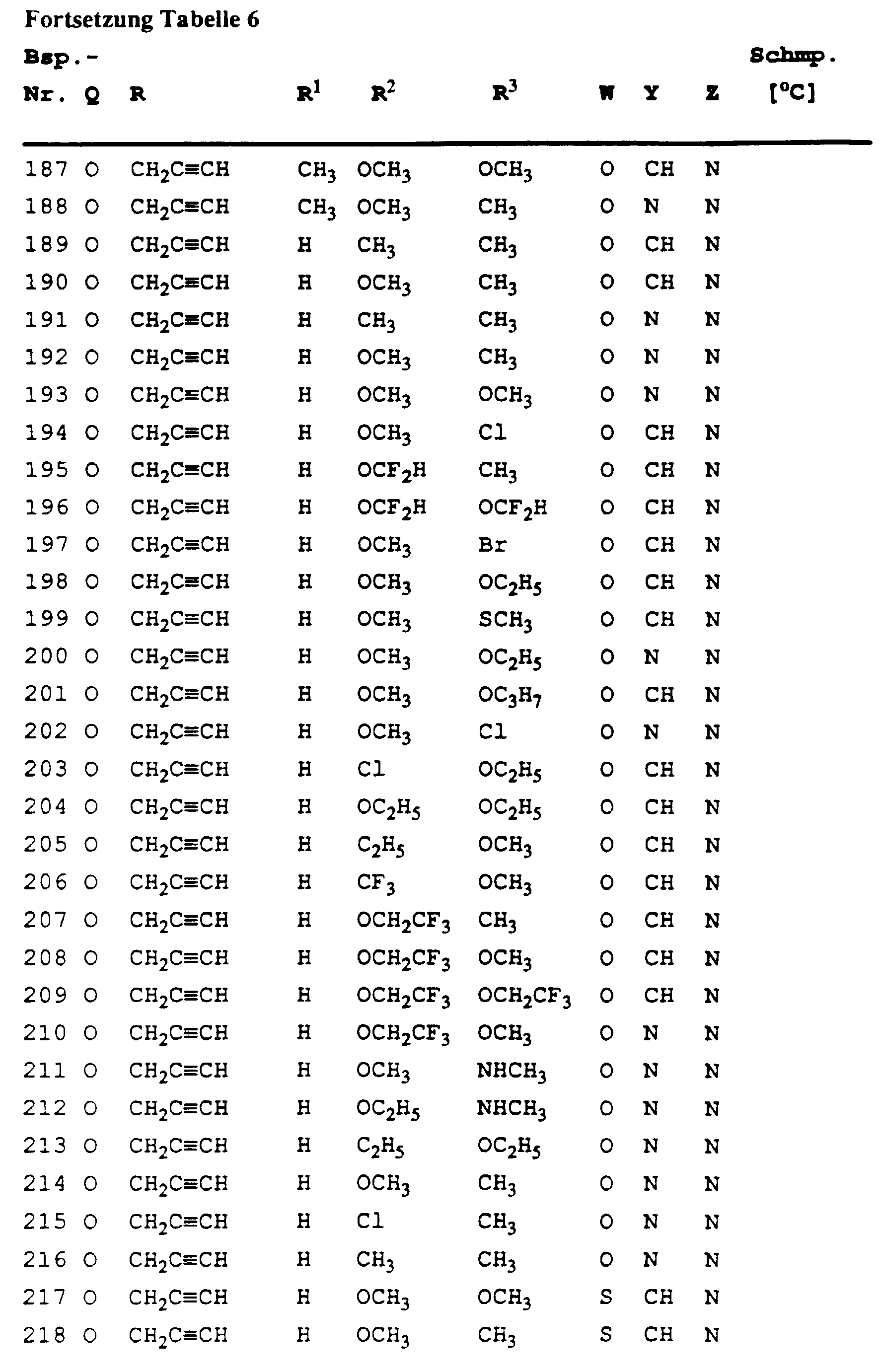

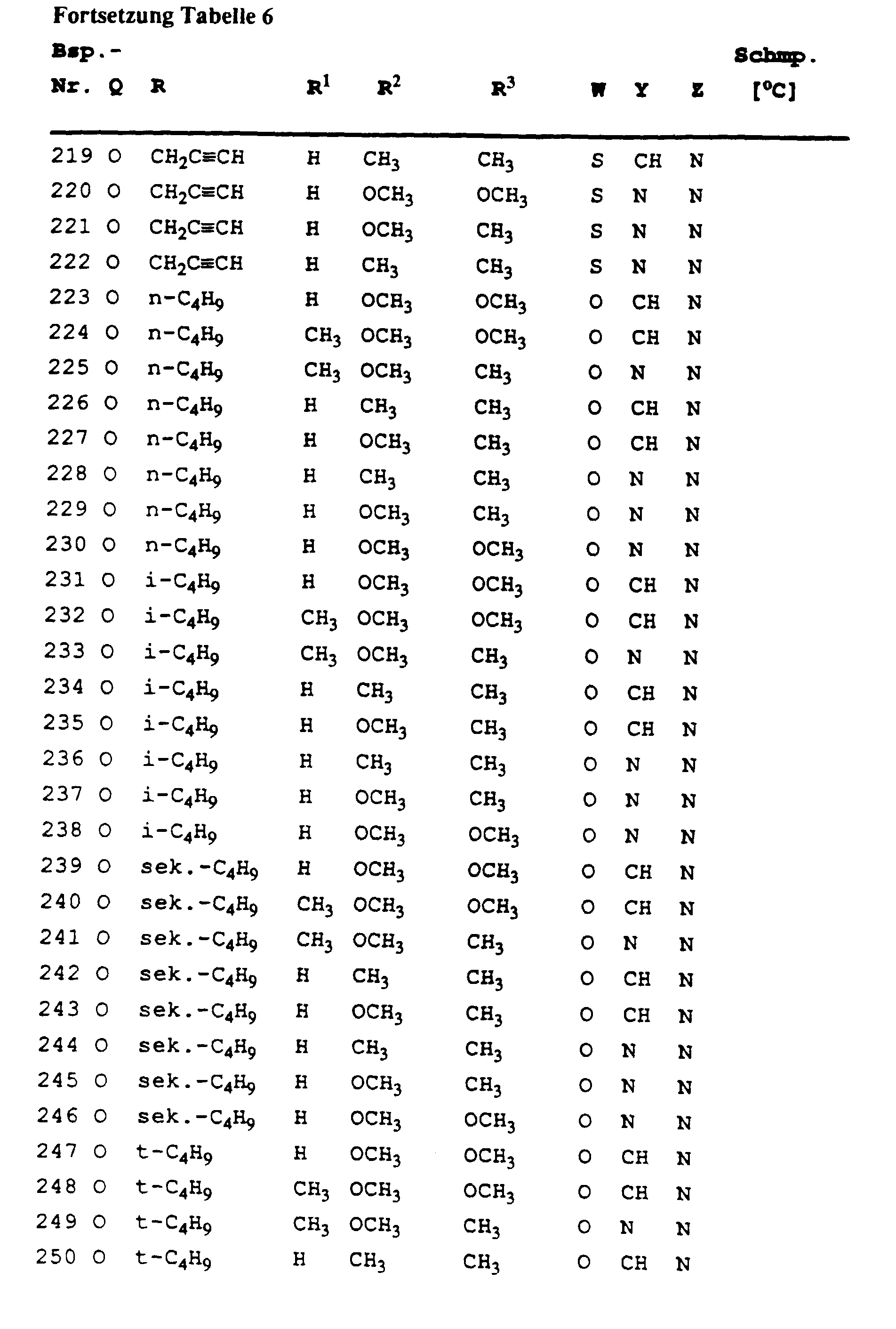

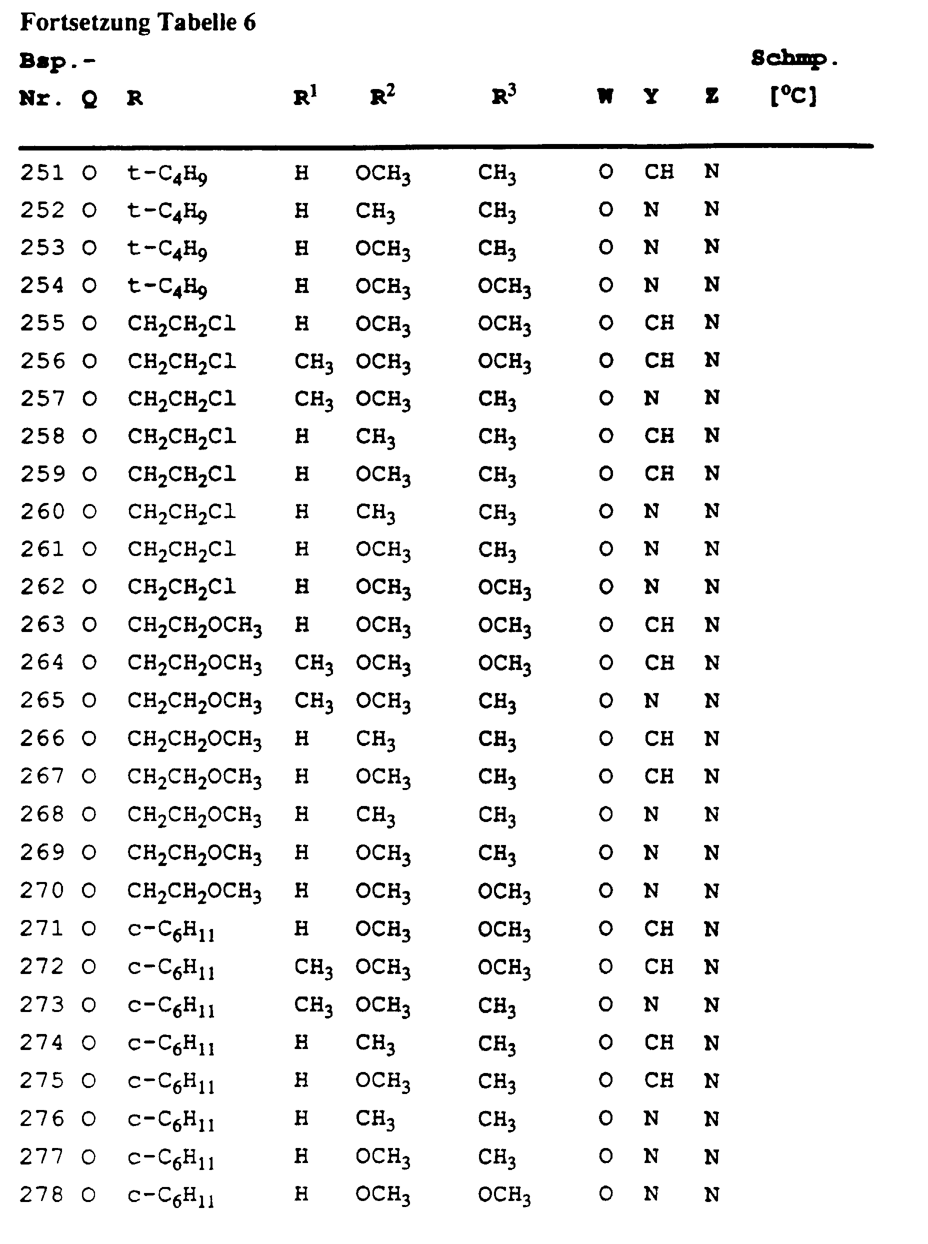

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether ((R)Trtton X 207), 3 Gew.-Teilen Isotridecanolpolyglykolether (8 EO) und 71 Gew.-Teilen paraffinischem Mineralöl (Siedebereich z.B. ca. 255 bis über 277°C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexan als Lösungsmittel und 10 Gew.-Teilen oxethyliertem Nonylphenol als Emulgator.
e) Ein in Wasser dispergierbares Granulat wird erhalten, indem man
   75 Gewichtsteile einer Verbindung der Formel (I),
   10 " ligninsulfonsaures Calcium,
   5 " Natriumlaurylsulfat,
   3 " Polyvinylalkohol und
   7 " Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 " 2,2'-dinaphthylmethan-6,6'-disulfonsaures Natrium,
   2 " oleoylmethyltaurinsaures Natrium,
   1 " Polyvinylalkohol,
   17 " Calciumcarbonat und
   50 " Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.
g) Ein Extruder-Granulat erhält man, indem man 20 Gewichtsteile Wirkstoff, 3 Gewichtsteile ligninsulfonsäures Natrium, 1 Gewichtsteil Carboxymethylcellulose und 76 Gewichtsteile Kaolin vermischt, vermahlt und mit Wasser anfeuchtet. Dieses Gemisch wird extrudiert und anschließend im Luftstrom getrocknet

### C. Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0-5 ausgedrückt ist. Dabei bedeutet:
0 = ohne Wirkung bzw. Schaden
1 = 0- 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

### 1. Unkrautwirkung im Vorauflauf

Samen bzw. Rhizomstücke von mono- und dikoytylen Unkrautpflanzen wurden in Plastiktöpfen in sandiger Lehmerde ausgelegt und mit Erde abgedeckt. Die in Form von benetzbaren Pulvem oder Emulsionskonzentraten formulierten erfindungsgemäßen Verbindungen wurden dann als wäßrige Suspensionen bzw. Emulsionen mit einer Wasseraufwandmenge von umgerechnet 600-800 Vha in unterschiedlichen Dosierungen auf die Oberfläche der Abdeckerde appliziert. Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 34 Wochen im Vergleich zu unbehandelten Kontrollen. Wie die Boniturwerte in Tabelle 7 zeigen, weisen die erfindungsgemäßen Verbindungen eine gute herbizide Vorauflaufwirksamkeit gegen ein breites Spektrum von Ungräsern und Unkräutern auf.

**Tabelle 7:**

| Vorauflaufwirkung | | | | | | | |
|---|---|---|---|---|---|---|---|
| Wirkstoff Tab/Bsp. | Dosis kg ai/ha | herbizide Wirkung | | | | | |
| | | STME | CRSE | SIAL | LOMU | ECCR | AVSA |
| 5/1 | 0,3 | 5 | 5 | 4 | 3 | 3 | 3 |
| 3/1 | 0,3 | 5 | 5 | 5 | 5 | 5 | 4 |
| Abkürzungen: STME = Stellaria media CRSE = Chrysanthemum segetum SIAL = Sinapis alba LOMU = Lolium multiflorum ECCR = Echinochloa crus-galli AVSA = Avena sativa a.i. = Aktivsubstanz | | | | | | | |

Vergleichbar gute wirksamkeiten werden in der Regel auch bei den anderen Verbindungen aus den Tabellen 2 bis 7 gefunden.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus untcr guten Wachstumsbedingungen angezogen. Drei Wochen nach der Aussaat wurden die Versuchspflanzen im Dreiblattstadium behandelt.
Die als Spritzpulver bzw. als Emulsionskonzentrate formulierten erfindungsgemäßen Verbindungen wurden in verschiedenen Dosierungen mit einer Wasseraufwandmenge von umgerechnet 600-800 l/ha auf die grünen Pflanzenteile gesprüht und nach ca. 34 Wochen Standzeit der Versuchspflanzen im Gewächshaus unter optimalen Wachstumsbedingungen die Wirkung der Präparate optisch im Vergleich zu unbehandelten Kontrollen bonitiert.
Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 8).

**Tabelle 8:**

| Nachauflaufwirkung | | | | | | | |
|---|---|---|---|---|---|---|---|
| Wirkstoff Tab/Bsp. | Dosis kg ai/ha | herbizide Wirkung | | | | | |
| | | STME | CRSE | SIAL | LOMU | ECCR | AVSA |
| 3/1 | 0,3 | 5 | 5 | 5 | 5 | 5 | 2 |
| Abkürzungen: STME = Stellaria media CRSE = Chrysanthemum segetum SIAL = Sinapis alba LOMU = Lolium multiflorum ECCR = Echinochloa crus-galli AVSA = Avena sativa a.i. = Aktivsubstanz | | | | | | | |

Vergleichbar gute wirksamkeiten werden in der Regel auch bei den anderen Verbindungen aus den Tabellen 2 bis 7 gefunden. Im Vergleich zu Verbindungen aus EP-A-7687 oder US-A-4,566,898 zeigen die erfindungsgemäßen Verbindungen der Formel I meist höhere Wirksamkeiten bei Problemunkräutern wie Galium aparine oder Echinochloa crus-galli.

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.
Ein Teil der Töpfe wurde sofort wie unter 1. beschrieben behandelt, die übrigen im Gewächshaus aufgestellt, bis die Pflanzen zwei bis drei echte Blätter entwickelt hatten, und dann mit den erfindungsgemäßen Substanzen in unterschiedlichen Dosierungen wie unter 2. beschrieben besprüht.
Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Weizen, Roggen, Sorghum-Hirsen, Mais oder Reis. Die erfindungsgemäßen Verbindungen weisen somit eine hohe Selektivität bei Anwendung zur Bekämpfung von unerwünschtem Pflanzenwuchs in landwirtschaftlichen Kulturen auf. Im Vergleich zu der Verbindung aus US-A-4,566,898 (siehe Verbindung der Formel (3)) oder Beispiel 80 aus EP-A-0291851 zeigen die erfindungsgemäßen Verbindungen der Formel I meist höhere Selektivität,
insbesondere bei der Bekämpfung von Problemunkräutern wie Galium aparine oder Echinochloa crus-galli in Nutzpflanzenkulturen.

### 4. Herbizide Wirkung bei Anwendung in Reis

Knollen und Rhizome bzw. Jungpflanzen oder Samen verschiedener Reisunkräuter wie Cyperus-Arten, Eleocharis, Scirpus und Echinochloa wurden in geschlossenen Plastiktöpfen in spezielle Reiserde ausgelegt bzw. gepflanzt und mit Wasser bis zu einer Höhe von 1 cm über dem Boden angestaut. Ebenso wurde mit Reispflanzen verfahren.

Im Vorauflaufverfahren, d.h. 3-4 Tage nach dem Verpflanzen, wurden die erfindungsgemäßen Verbindungen in Form wäßriger Suspensionen oder Emulsionen ins Anstauwasser gegossen oder als Granulate ins Wasser gestreut. Jeweils drei Wochen später wurde die herbizide Wirkung und eine eventuelle Schadwirkung gegenüber Reis optisch bonitiert. Die Ergebnisse zeigen, daß sich die erfindungsgemäßen Verbindungen zur selektiven Unkrautbekämpfung in Reis eignen.
Gegenüber bisherigen Reisherbiziden zeichnen sich die erfindungsgemäßen Verbindungen dadurch aus, daß sie zahlreiche, insbesondere auch schwer bekämpfbare Unkräuter, die aus Dauerorganen keimen, wirkungsvoll bekämpfen und dabei von Reis toleriert werden.

## Patentansprüche

1. Verbindungen der Formel (I) und deren Salze, 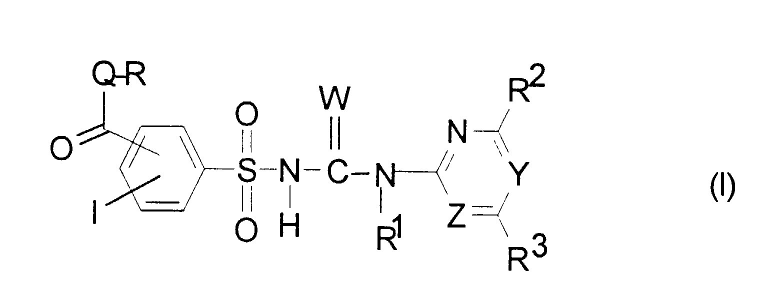 worin
Q Sauerstoff, Schwefel oder -N(R⁴)-,
W Sauerstoff oder Schwefel,
Y,Z unabhängig voneinander CH oder N, wobei Y und Z nicht gleichzeitig CH sind,
R Wasserstoff, (C₁-C₁₂)-Alkyl; (C₂-C₁₀)-Alkenyl; (C₂-C₁₀)-Alkinyl; (C₁-C₆)-Alkyl, das ein- bis vierfach durch Reste aus der Gruppe Halogen, (C₁-C₄)-Alkoxy, (C₁-C₄)-Thioalkyl, CN, (C₂-C₅)-Alkoxycarbonyl und (C₂-C₆)-Alkenyl substituiert ist; oder (C₃-C₈)-Cycloalkyl, das unsubstituiert oder durch Reste aus der Gruppe (C₁-C₄)-Alkyl_{,} (C₁-C₄)-Alkoxy, (C₁-C₄)-Alkylthio und Halogen substituiert ist; (C₅-C₈)-Cycloalkenyl; Phenyl-(C₁-C₄)-alkyl, das im Phenylrest unsubstituiert oder durch einen oder mehrere Reste aus der Gruppe Halogen, (C₁-C₄)-Alkyl, (C₁-C₄)-Alkoxy, (C₁-C₄)-Haloalkyl, (C₁-C₄)-Thioalkyl, (C₂-C₅)-Alkoxycarbonyl, (C₂-C₅)-Alkylcarbonyloxy, Carbonamid, (C₂-C₅)-Alkylcarbonylamino, (C₂-C₅)-Alkylaminocarbonyl, Di-[(C₁-C₄)-alkyl]-carbonyl und Nitro substituiert ist; oder einen Rest der Formeln A-1 bis A-10 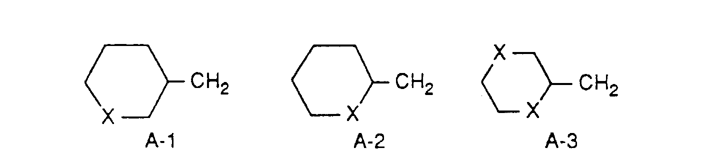 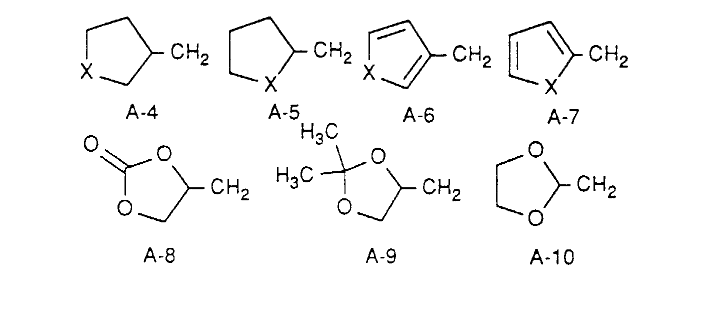
worin
X O, S, S(O) oder SO₂;
R¹ Wasserstoff oder (C₁-C₃)-Alkyl;
R² Wasserstoff, Halogen, (C₁-C₃)-Alkyl oder (C₁-C₃)-Alkoxy, wobei die beiden letztgenannten Reste unsubstituiert oder durch ein- oder mehrfach durch Halogen oder (C₁-C₃)-Alkoxy substituiert sind;
R³ Wasserstoff, Halogen, (C₁-C₃)-Alkyl, (C₁-C₃)-Alkoxy oder (C₁-C₃)-Alkylthio, wobei die vorgenannten alkylhaltigen Reste unsubstituiert oder ein- oder mehrfach durch Halogen oder ein- oder zweifach durch (C₁-C₃)-Alkoxy oder (C₁-C₃)-Alkylthio substituiert sind; oder einen Rest der Formel NR⁵R⁶, (C₃-C₆)-Cydoalkyl, (C₂-C₄)-Alkenyl, (C₂-C₄)-Alkinyl, (C₃-C₄)-Alkenyloxy oder (C₃-C₄)-Alkinyloxy;
R⁴ Wasserstoff, (C₁-C₄)-Alkyl oder (C₁-C₄)-Alkoxy und
R⁵ und R⁶ unabhängig voneinander Wasserstoff, (C₁-C₄)-Alkyl, (C₃-C₄)-Alkenyl, (C₁-C₄)-Haloalkyl oder (C₁-C₄)-Alkoxy bedeuten.

2. Verbindungen oder deren Salze nach Anspruch 1, dadurch gekennzeichnet, daß
Q O oder S,
W O,
Y CH oder N und
Z N bedeuten.

3. Verbindungen oder deren Salze nach Anspruch 1 oder 2, dadurch gekennzeichnet, daß
R Wasserstoff; (C₁-C₆)-Alkyl; (C₂-C₆)-Alkenyl; (C₂-C₆)-Alkinyl; (C₁-C₄)-Alkyl, das ein- bis vierfach durch Reste aus der Gruppe Halogen, (C₁-C₂)-Alkoxy-, (C₁-C₂)-Thioalkyl, (C₂-C₃)-Alkoxycarbonyl und (C₂-C₄)-Alkenyl substituiert ist; (C₅-C₆)-Cycloalkyl, das unsubstituiert oder durch Reste aus der Gruppe (C₁-C₄)-Alkyl, (C₁-C₄)-Alkoxy, (C₁-C₄)-Alkylthio und Halogen substituiert ist; (C₅-C₆)-Cycloalkenyl; Benzyl, das im Phenylrest unsubstituiert oder durch einen bis drei Reste aus der Gruppe Halogen, (C₁-C₂)-Alkyl, (C₁-C₂-Alkoxy, (C₁-C₂)-Haloalkyl, (C₁-C₂)-Thioalkyl und (C₂-C₄)-Alkoxycarbonyl substituiert ist, oder einen Rest der genannten Formeln A-1 bis A-10, worin
X O, S, S(O) oder SO₂,
bedeuten.

4. Verbindungen der Formel (I) oder deren Salze nach einem oder mehreren der Ansprüche 1 bis 3, dadurch gekennzeichnet, daß
R¹ Wasserstoff oder CH₃;
R² Wasserstoff, Halogen, (C₁-C₂)-Alkyl oder (C₁-C₂)-Alkoxy, wobei die beiden letztgenannten Reste unsubstituiert oder ein- oder mehrfach durch Halogen oder (C₁-C₃)-Alkoxy substituiert sind;
R³ Wasserstoff, Halogen, (C₁-C₂)-Alkyl, (C₁-C₂)-Alkoxy, oder (C₁-C₂)-Alkylthio, wobei die vorgenannten alkylhaltigen Reste unsubstituiert oder ein- oder mehrfach durch Halogen oder ein- oder zweifach durch (C₁-C₂)-Alkoxy oder (C₁-C₂)-Alkylthio substituiert sind; oder einen Rest der Formel NR⁵R⁶;
R⁴ Wasserstoff oder (C₁-C₂)-Alkyl und
R⁵ und R⁶ unabhängig voneinander Wasserstoff oder (C₁-C₂)-Alkyl
bedeuten.

5. Verbindungen oder deren Salze nach einem oder mehreren der Ansprüche 1 bis 4, dadurch gekennzeichet, daß
W Sauerstoff,
R¹ Wasserstoff oder CH₃,
Y CH oder N,
Z N,
R² Wasserstoff, CH₃, CH₂CH₃, OCH₃, OCH₂CH₃, OCHF₂ oder Cl und
R³ Wasserstoff, CH₃, CH₂CH₃, OCH₃, OCH₂CH₃, OCHF₂, NH(CH₃), N(CH₃)₂, CF₃, OCH₂CF₃ oder Cl sind.

6. Verfahren zur Herstellung von Verbindungen der Formel (I) oder deren Salzen, dadurch gekennzeichnet, daß man
a) eine Verbindung der Formel (II) 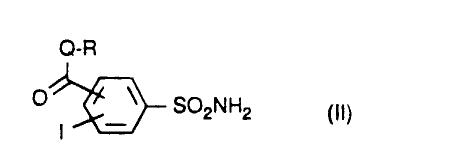 mit einem heterocyclischen Carbamat der Formel (III), 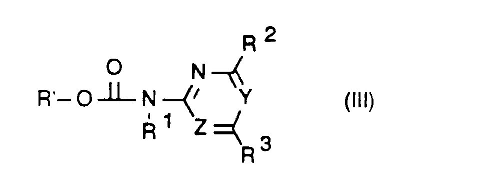 worin R' unsubstituiertes oder substituiertes Aryl oder Alkyl ist, umsetzt oder
b) ein Phenylsulfonylcarbamat der Formel (IV) 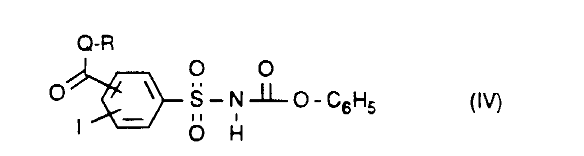 mit einem Aminoheterocyclus der Formel (V) 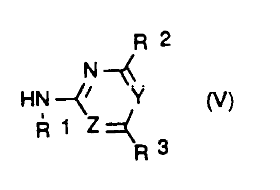 umsetzt oder
c) ein Sulfonylisocyanat der Formel (VI) 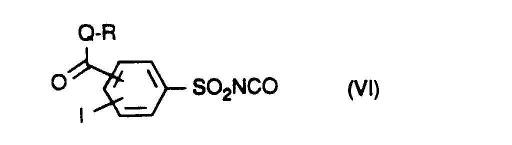 mit einem Aminoheterocyclus der unter b) genannten Formel (V) umsetzt.

7. Herbizide oder pflanzenwachstumsregulatorische Mittel, dadurch gekennzeichnet, daß sie eine Verbindung der Formel (I) oder deren Salze nach einem oder mehreren der Ansprüche 1 bis 5 und übliche Formulierungshilfsmittel enthalten.

8. Verwendung von Verbindungen der Formel (I) oder deren Salze nach einem oder mehreren der Ansprüche 1 bis 5 als Herbizide oder Pflanzenwachstumsregulatoren.

9. Verfahren zur selektiven Bekämpfung von Schadpflanzen, dadurch gekennzeichnet, daß man eine wirksame Menge einer der nach einem oder mehreren der Ansprüche 1 bis 5 definierten Verbindungen oder deren Salze auf die Pflanzen, Pflanzensamen oder deren Anbaufläche appliziert

10. Verfahren zur Pflanzenwachstumsregulierung, dadurch gekennzeichnet, daß man eine wirksame Menge einer der nach einem oder mehreren der Ansprüche 1 bis 5 definierten Verbindungen oder deren Salze auf die Pflanzen, Pflanzensamen oder deren Anbaufläche appliziert

11. Verbindungen der Formel (II), 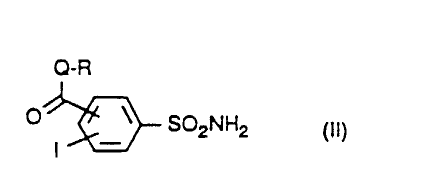 worin Q und R wie in Formel (I) nach einem oder mehreren der Anspüche 1 bis 5 definiert sind, ausgenommen 3-Aminosulfonyl-4-iod-benzoesäure.

12. Verfahren zur Herstellung der Verbindungen der Formel (II) nach Anspruch 11, dadurch gekennzeichnet, daß man Sulfonsäurehalogenide der Formel 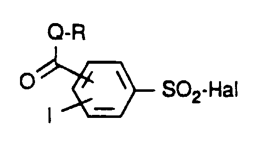 worin Q und R wie in Formel (II) definiert sind und Hal = F, Cl, Br oder I bedeutet, mit Ammoniak oder mit tert.- Butylamin und anschließender Schutzgruppenabspaltung mit Trifluoressigsäure umsetzt.

13. Sulfonylisocyanat der Formel (VI) 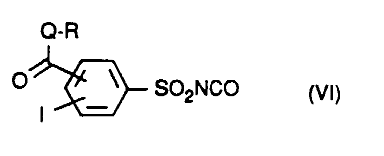 worin Q und R eine wie in Formel (I) nach einem oder mehreren der Ansprüche 1 bis 5 definierte Bedeutung haben.

## Claims

1. A compound of the formula (I) and salts thereof 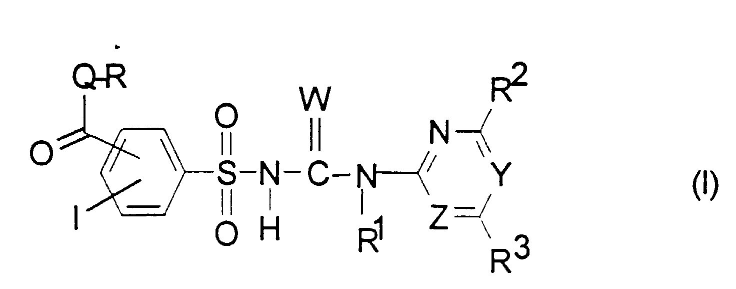 where
Q is oxygen, sulfur or -N(R⁴)-,
W is oxygen or sulfur,
Y and Z independently of one another are CH or N, where Y and Z are not simultaneously CH,
R is hydrogen; (C₁-C₁₂) alkyl; (C₂-C₁₀)-alkenyl; (C₂-C₁₀)alkynyl; (C₁-C₆)alkyl which is monosubstituted to tetrasubstituted by radicals selected from the group comprising halogen, (C₁-C₄)alkoxy, (C₁-C₄)thioalkyl, CN, (C₂-C₅)alkoxycarbonyl and (C₂-C₆)alkenyl; or (C₃-C₈)cycloalkyl which is unsubstituted or substituted by radicals selected from the group comprising (C₁-C₄)alkyl, (C₁-C₄)-alkoxy, (C₁-C₄)alkylthio and halogen; (C₅-C₈)cycloalkenyl; phenyl(C₁-C₄)alkyl which is unsubstituted or substituted in the phenyl radical by one or more radicals selected from the group comprising halogen, (C₁-C₄)alkyl, (C₁-C₄)alkoxy, (C₁-C₄)haloalkyl, (C₁-C₄)thioalkyl, (C₂-C₅)alkoxycarbonyl, (C₂-C₅)alkylcarbonyloxy, carboxamide, (C₂-C₅)alkylcarbonylamino, (C₂-C₅) alkylaminocarbonyl, di[(C₁-C₄)alkyl]carbonyl and nitro; or a radical of the formulae A-1 to A-10 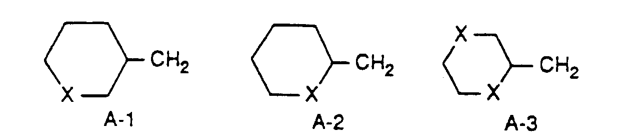 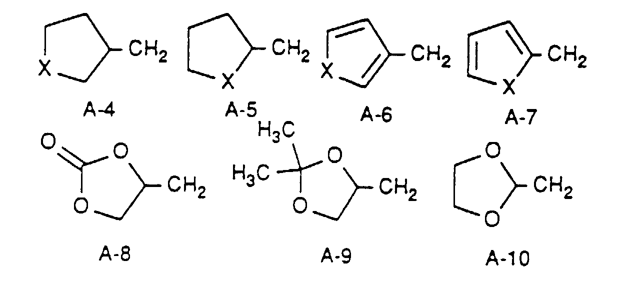
where
X is O, S, S(O) or SO₂;
R¹ is hydrogen or (C₁-C₃)alkyl;
R² is hydrogen, halogen, (C₁-C₃)alkyl or (C₁-C₃)alkoxy, where the two last-mentioned radicals are unsubstituted or monosubstituted or polysubstituted by halogen or (C₁-C₃)alkoxy;
R³ is hydrogen, halogen, (C₁-C₃)alkyl, (C₁-C₃)alkoxy or (C₁-C₃)alkylthio, where the abovementioned alkyl-containing radicals are unsubstituted or monosubstituted or polysubstituted by halogen or monosubstituted or disubstituted by (C₁-C₃)alkoxy or (C₁-C₃)alkylthio; or a radical of the formula NR⁵R⁶, (C₃-C₆)cycloalkyl, (C₂-C₄)alkenyl, (C₂-C₄)alkynyl, (C₃-C₄)alkenyloxy or (C₃-C₆)alkynyloxy;
R⁴ is hydrogen, (C₁-C₄)alkyl or (C₁-C₄)alkoxy and
R⁵ and R⁶ independently of one another are hydrogen, (C₁-C₄)alkyl, (C₃-C₄)alkenyl, (C₁-C₄)haloalkyl or (C₁-C₄)alkoxy.

2. A compound or salts thereof as claimed in claim 1, wherein
Q is O or S,
W is O,
Y is CH or N and
Z is N.

3. A compound or salts thereof as claimed in claim 1 or 2, wherein
R is hydrogen; (C₁-C₆)alkyl; (C₂-C₆)alkenyl; (C₂-C₆)alkynyl; (C₁-C₄)alkyl which is monosubstituted to tetrasubstituted by radicals selected from the group comprising halogen, (C₁-C₂)alkoxy, (C₁-C₂)thioalkyl, (C₂-C₃) alkoxycarbonyl and (C₂-C₄)alkenyl; (C₅-C₆)cycloalkyl which is unsubstituted or substituted by radicals selected from the group comprising (C₁-C₄)alkyl, (C₁-C₄)alkoxy, (C₁-C₄)alkylthio and halogen; (C₅-C₆)cycloalkenyl; benzyl which is unsubstituted or substituted in the phenyl radical by one to three radicals selected from the group comprising halogen, (C₁-C₂)alkyl, (C₁-C₂)alkoxy, (C₁-C₂)haloalkyl, (C₁-C₂)thioalkyl and (C₂-C₄)alkoxycarbonyl, or a radical of the abovementioned formulae A-1 to A-10, where
X is O, S, S(O) or SO₂.

4. A compound of the formula (I) or salts thereof as claimed in one or more of claims 1 to 3, wherein
R¹ is hydrogen or CH₃;
R² is hydrogen, halogen, (C₁-C₂)alkyl or (C₁-C₂)alkoxy, where the two last-mentioned radicals are unsubstituted or monosubstituted or polysubstituted by halogen or (C₁-C₃)alkoxy;
R³ is hydrogen, halogen, (C₁-C₂)alkyl, (C₁-C₂)alkoxy or (C₁-C₂)alkylthio, where the abovementioned alkyl-containing radicals are unsubstituted or monosubstituted or polysubstituted by halogen or monosubstituted or disubstituted by (C₁-C₂)alkoxy or (C₁-C₂)alkylthio; or a radical of the formula NR⁵R⁶;
R⁴ is hydrogen or (C₁-C₂)alkyl and
R⁵ and R⁶ independently of one another are hydrogen or (C₁-C₂)alkyl.

5. A compound or salts thereof as claimed in one or more of claims 1 to 4, wherein
W is oxygen,
R¹ is hydrogen or CH₃,
Y is CH or N,
Z is N,
R² is hydrogen, CH₃, CH₂CH₃, OCH₃, OCH₂CH₃, OCHF₂ or Cl and
R³ is hydrogen, CH₃, CH₂CH₃, OCH₃, OCH₂CH₃, OCHF₂, NH(CH₃), N(CH₃)₂, CF₃, OCH₂CF₃ or Cl.

6. A process for the preparation of a compound of the formula (I) or salts thereof, which comprises
a) reacting a compound of the formula (II) 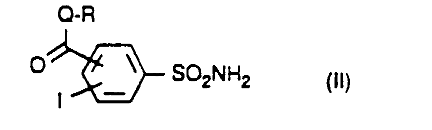 with a heterocyclic carbamate of the formula (III) 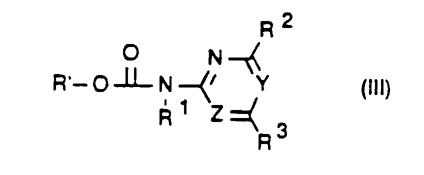 where R' is unsubstituted or substituted aryl or alkyl, or
b) reacting a phenylsulfonyl carbamate of the formula (IV) 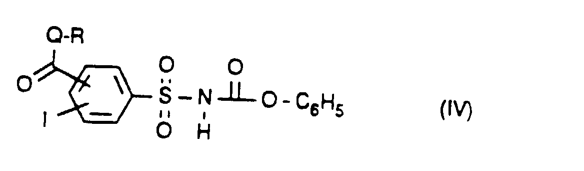 with an aminoheterocycle of the formula (V) 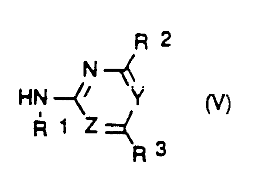 or
c) reacting a sulfonyl isocyanate of the formula (VI) 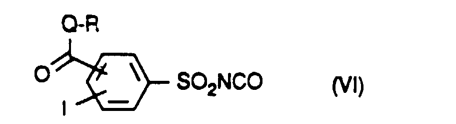 with an aminoheterocycle of the formula (V) mentioned under b).

7. A herbicidal or plant growth-regulating agent comprising a compound of the formula (I) or salts thereof as claimed in one or more of claims 1 to 5 and customary formulation auxiliaries.

8. The use of a compound of the formula (I) or salts thereof as claimed in one or more of claims 1 to 5 as herbicides or plant growth regulators.

9. A method of selectively controlling weeds, which comprises applying an effective amount of a compound or salts thereof as defined in one or more of claims 1 to 5 to the plants, seeds of the plants or the area where they are grown.

10. A method of regulating plant growth, which comprises applying an effective amount of a compound or salts thereof as defined in one or more of claims 1 to 5 to the plants, seeds of the plants or the area where they are grown.

11. A compound of the formula (II) 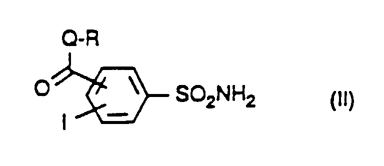 where Q and R are defined as in formula (I) as claimed in one or more of claims 1 to 5, with the exception of 3-aminosulfonyl-4-iodobenzoic acid.

12. A process for the preparation of a compound of the formula (II) as claimed in claim 11, which comprises reacting sulfonyl halides of the formula 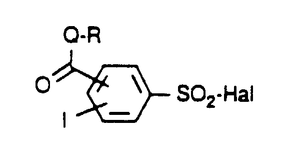 where Q and R are defined as in formula (II) and Hal is F, C1, Br or I, with ammonia or with tert.-butylamine, followed by elimination of the protective groups using trifluoroacetic acid.

13. A sulfonyl isocyanate of the formula (VI) 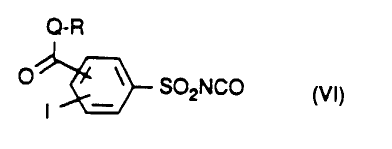 where Q and R are defined as in formula (I) as claimed in one or more of claims 1 to 5.

## Revendications

1. Composés de formule (I) et leurs sels, 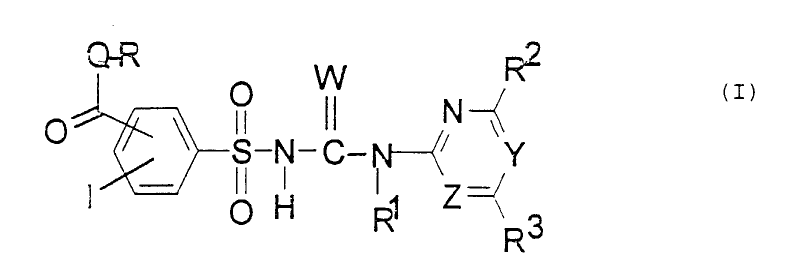 dans laquelle
Q représente un atome d'oxygène, un atome de soufre, ou -N(R⁴)- ;
W représente un atome d'oxygène ou de soufre ;
Y, Z, indépendamment l'un de l'autre, représentent CH ou N, Y et Z n'étant pas simultanément CH, de préférence Y = CH ou N et Z = N ;
R représente un atome d'hydrogène, des groupes alkyle en C₁-C₁₂, alcényle en C₂-C₁₀, alcynyle en C₂-C₁₀ ; alkyle en C₁-C₆ qui est substitué 1 à 4 fois par des restes pris parmi les atomes d'halogène, les groupes alkoxy en C₁-C₄, thioalkyle en C₁-C₄, -CN, alkoxy-carbonyle en C₂-C₅ et alcényle en C₂-C₆; ou cycloalkyle en C₃-C₈ qui est non substitué ou substitué par des restes pris parmi les groupes alkyle en C₁-C₄, alkoxy en C₁-C₄, (alkyl en C₁-C₄)-thio et un atome d'halogène ; cycloalcényle en C₅-C₈ ; phénylalkyle en C₁-C₄, qui est non substitué ou substitué sur le reste phényle par un ou plusieurs restes pris parmi les atomes d'halogène, les groupes alkyle en C₁-C₄, alkoxy en C₁-C₄, haloalkyle en C₁-C₄, thioalkyle en C₁-C₄, alkoxycarbonyle en C₂-C₅, alkyl-carbonyloxy en C₂-C₅, carboxamide, alkylaminocarbonyle en C₂-C₅, di-(alkyl en C₁-C₄)carbonyle et nitro ; un reste de formules A-1 à A-10 : 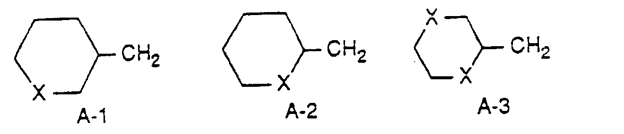 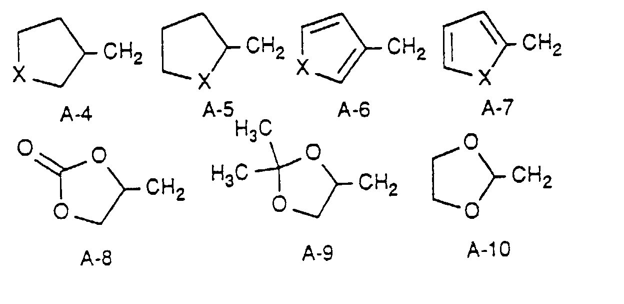
dans lesquelles
X représente O, S(O) ou SO₂ ;
R¹ représente un atome d'hydrogène ou un groupe alkyle en C₁-C₃ ;
R² représente un atome d'hydrogène, un atome d'halogène, des groupes alkyle en C₁-C₃ ou alkoxy en C₁-C₃, les deux derniers restes étant non substitués ou substitués une ou plusieurs fois par des atomes d'halogène ou un groupe alkoxy en C₁-C₃ ;
R³ représente un atome d'hydrogène, un atome d'halogène, des groupes alkyle en C₁-C₃, alkoxy en C₁-C₃ ou (alkyl en C₁-C₃)thio, les derniers restes alkylés étant non substitués ou substitués une ou plusieurs fois par des atomes d'halogène, ou 1 ou 2 fois par des groupes alkoxy en C₁-C₃ ou (alkyl en C₁-C₃)thio, ou un reste de formule NR⁵R⁶, cycloalkyle en C₃-C₆, alcényle en C₂-C₄, alcynyle en C₂-C₄, alcényloxy en C₃-C₄ ou alcynyloxy en C₃-C₆ ;
R⁴ représente un atome d'hydrogène, un groupe alkyle en C₁-C₄ ou alkoxy en C₁-C₄, et
R⁵ et R⁶, indépendamment l'un de l'autre, représentent un atome d'hydrogène, des groupes alkyle en C₁-C₄, alcényle en C₃-C₄, haloalkyle en C₁-C₄, ou alkoxy en C₁-C₄.

2. Composés ou leurs sels selon la revendication 1, caractérisés en ce que
Q représente O, ou S,
W représente O,
Y représente CH ou N et
Z représente N.

3. Composés ou leurs sels selon la revendication 1 ou 2, caractérisés en ce que
R représente un atome d'hydrogène ; des groupes alkyle en C₁-C₆ ; alcényle en C₂-C₆ ; alcynyle en C₂-C₆ ; alkyle en C₁-C₄ qui est substitué 1 à 4 fois par des restes pris parmi les atomes d'halogène, les groupes alkoxy en C₁-C₂, thioalkyle en C₁-C₂, alkoxy-carbonyle en C₂-C₃ et alcényle en C₂-C₄ ; cycloalkyle en C₅-C₆, qui est non substitué ou substitué par des restes pris parmi les groupes alkyle en C₁-C₄, alkoxy en C₁-C₄, (alkyl en C₁-C₄)thio et un atome d'halogène ; cycloalcényle en C₅-C₆, benzyle qui est non substitué ou substitué sur le reste phényle par 1 à 3 restes pris parmi les atomes d'halogène, les groupes alkyle en C₁-C₂, alkoxy en C₁-C₂, haloalkyle en C₁-C₂, thioalkyle en C₁-C₂ et alkoxy-carbonyle en C₂-C₄, ou un reste des formules A-1 à A-10 précitées,
dans lesquelles
X représente O, S, S(O) ou SO₂.

4. Composés de formule (I) ou leurs sels selon une ou plusieurs des revendications 1 à 3, caractérisés en ce que :
R¹ représente un atome d'hydrogène ou CH₃ ;
R² représente un atome d'hydrogène, un atome d'halogène, des groupes alkyle en C₁-C₂ ou alkoxy en C₁-C₂, les deux derniers restes cités étant non substitué ou substitué une ou plusieurs fois par des atomes d'halogène ou un groupe alkoxy en C₁-C₃ ;
R³ représente un atome d'hydrogène, un atome d'halogène, des groupes alkyle en C₁-C₂, alkoxy en C₁-C₂ ou (alkyl en C₁-C₂)thio, les restes alkylés précités étant non substitué ou substitué une ou plusieurs fois par des atomes d'halogène, ou 1 ou 2 fois par des groupes alkoxy en C₁-C₂ ou (alkyl en C₁-C₂)-thio ; ou un reste de formule NR⁵R⁶ ;
R⁴ représente un atome d'hydrogène ou un groupe alkyle en C₁-C₂ et
R⁵ et R⁶, indépendamment l'un de l'autre, représentent un atome d'hydrogène ou un groupe alkyle en C₁-C₂.

5. Composés ou leurs sels selon une ou plusieurs des revendications 1 à 4, caractérisés en ce que :
W représente un atome d'oxygène, et
R¹ représente un atome d'hydrogène ou CH₃,
Y représente CH ou N,
Z représente N,
R² représente un atome d'hydrogène, CH₃, CH₂CH₃, OCH₃, OCH₂CH₃, OCHF₂ ou Cl et
R³ représente un atome d'hydrogène, CH₃, CH₂CH₃, OCH₃, OCH₂CH₃, OCHF₂, NH (CH₃), N(CH₃)₂, CF₃, OCH₂CF₃ ou Cl.

6. Procédé pour la préparation de composés de formule (I) ou de leurs sels, caractérisé en ce que :
a) on fait réagir un composé de formule (II) 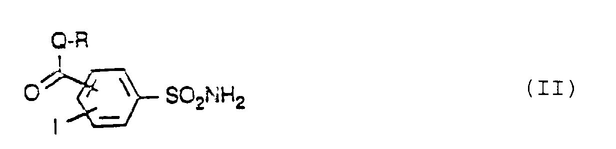 avec un carbamate hétérocyclique de formule (III) 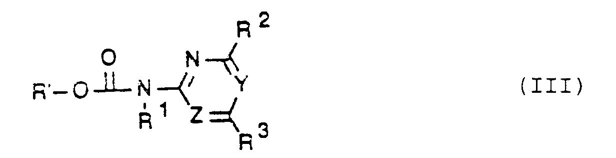 dans laquelle R' représente un aryle ou un alkyle non substitué ou substitué,
b) on fait réagir un phénylsulfonylcarbamate de formule (IV) 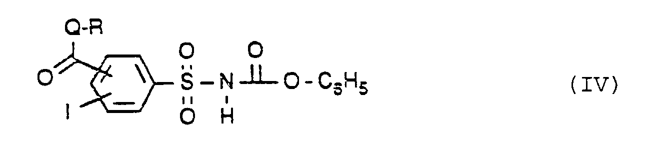 avec un hétérocycle aminé de formule (V) 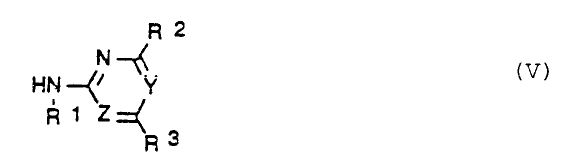 ou
c) on fait réagir un sulfonylisocyanate de formule (VI) 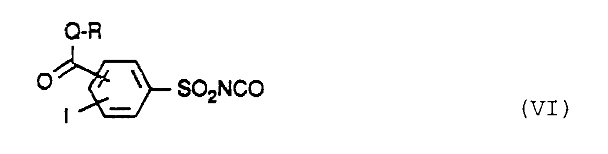 avec un hétérocycle aminé de formule (V), citée au point b).

7. Agents herbicides ou régulateurs de croissance caractérisés en ce qu'il contiennent un composé de formule (I) ou ses sels selon une ou plusieurs des revendications 1 à 5, et des adjuvants de formulation usuels.

8. Utilisation de composés de formule(I) ou de leurs sels selon une ou plusieurs des revendications 1 à 5, en tant qu'herbicides ou régulateurs de croissance végétale.

9. Procédé pour la lutte sélective contre des plantes adventices caractérisé en ce que l'on applique une quantité efficace d'un des composés ou de ses sels définis selon une ou plusieurs des revendications 1 à 5, sur les plantes, les grains ou la surface cultivable.

10. Procédé pour la régulation de la croissance végétale, caractérisé en ce que l'on applique une quantité efficace d'un ou plusieurs composés ou de leurs sels définis selon les revendications 1 à 5, sur les plantes, les grains ou la surface cultivable.

11. Composés de formule (II) 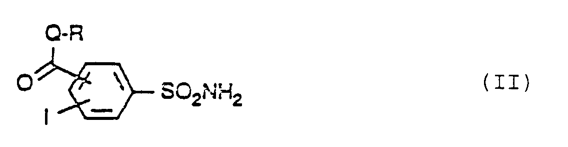 dans laquelle Q et R sont définis comme à la formule (I) selon une ou plusieurs des revendications 1 à 5, à l'exception de l'acide 3-aminosulfonyl-4-iodobenzoïque.

12. Procédé pour la préparation des composés de formule (II) selon la revendication 11, caractérisé en ce que l'on fait réagir des halogénures d'acides sulfoniques de formule 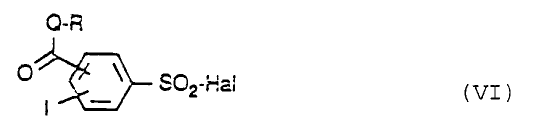 dans laquelle Q et R sont définis comme à la formule (II) et Hal = F, Cl, Br ou I, avec l'ammoniac ou avec la tert-butylamine et par dissociation ultérieure des groupes protecteurs avec l'acide trifluoracétique.

13. Sulfonylisocyanate de formule (VI) 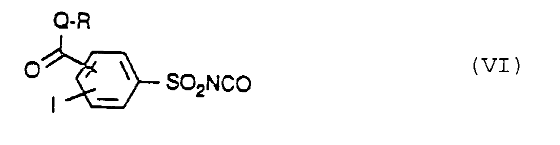 dans laquelle Q et R sont définis comme à la formule (I) selon une ou plusieurs des revendications 1 à 5.
